# EUROPEAN PATENT SPECIFICATION

(11) **EP 3 248 013 B1**
(45) Date of publication and mention of the grant of the patent: **15.07.2020**
(21) Application number: 16740906.9
(22) Date of filing: 25.01.2016
(51) Int. Cl.: G01N 33/53

(54) **CANCER MARKERS AND METHODS OF USE THEREOF**
KREBSMARKER UND VERFAHREN ZUR VERWENDUNG DAVON
MARQUEURS DE CANCER ET LEURS PROCÉDÉS D'UTILISATION

(30) Priority: 24.01.2015 US 201562107378 P; 11.12.2015 US 201562266514 P
(43) Date of publication of application: 29.11.2017
(73) Proprietor: Academia Sinica, Nankang, Taipei 115 (TW)
(72) Inventor: WONG, Chi-Huey, Rancho Santa Fe, CA 92067 (US); WU, Chung-Yi, 221 New Taipei City (TW); CHEUNG, Sarah, K.C., 115 Taipei (TW); CHUANG, Po-Kai, 115 Taipei (TW); HSU, Tsui-Ling, 115 Taipei (TW)
(74) Representative: Lavoix
(86) International application number: PCT/US2016/014771
(87) International publication number: WO 2016/118961

(56) References cited:
- WO-A1-2013/126993
- US-A1- 2012 294 859
- US-A1- 2012 328 646
- US-A1- 2012 328 646
- US-A1- 2015 344 551
- US-A1- 2016 274 121
- Y.-L. HUANG ET AL: "Carbohydrate-based vaccines with a glycolipid adjuvant for breast cancer", PROCEEDINGS OF THE NATIONAL ACADEMY OF SCIENCES, vol. 110, no. 7, 25 January 2013 (2013-01-25), pages 2517-2522, XP055394157, US ISSN: 0027-8424, DOI: 10.1073/pnas.1222649110
- SARAH K. C. CHEUNG ET AL: "Stage-specific embryonic antigen-3 (SSEA-3) and [beta]3GalT5 are cancer specific and significant markers for breast cancer stem cells", PROCEEDINGS OF THE NATIONAL ACADEMY OF SCIENCES, vol. 113, no. 4, 17 December 2016 (2016-12-17), pages 960-965, XP055474481, US ISSN: 0027-8424, DOI: 10.1073/pnas.1522602113
- ISSHIKI ET AL.: 'Cloning, Expression, and Characterization of a Novel UDP-galactose:b-N- Acetylglucosamine b1,3-Galactosyltransferase (b3Gal-T5) Responsible for Synthesis of Type 1 Chain in Colorectal and Pancreatic Epithelia and Tumor Cells Derived Therefrom*' JOURNAL OF BIOLOGICAL CHEMISTRY vol. 274, no. 18, 01 April 1999, pages 12499 - 12507, XP002928039 DOI: 10.1074/JBC.274.18.12499
- CHEUNG ET AL.: 'Stage-specific embryonic antlgen-3 (SSEA-3) and beta3GalT5 are cancer specific and significant markers for breast cancer stem cells' PNAS vol. 113, no. 4, 26 January 2016, pages 960 - 965, XP055474481 DOI: 10.1073/PNAS.1522602113
- THEODOROPOULOS P A ET AL: "Circulating tumor cells with a putative stem cell phenotype in peripheral blood of patients with breast cancer", CANCER LETTERS, NEW YORK, NY, US, vol. 288, no. 1, 1 February 2010 (2010-02-01), pages 99-106, XP026851809, ISSN: 0304-3835 [retrieved on 2009-07-19]

### SEQUENCE LISTING

<110> Academia Sinica
<120> CANCER MARKERS AND METHODS OF USE THEREOF
<130> G2112-01605
<140> 15/005,930
   <141> 2016-01-25
<150> 62/107,378
   <151> 2015-01-24
<150> 14/599,174
   <151> 2015-01-16
<150> 62/266,514
   <151> 2015-12-11
<160> 5
<170> PatentIn version 3.5
<210> 1
   <211> 26
   <212> DNA
   <213> Artificial Sequence
<220>
   <223> Description of artificial sequence: synthetic polynucleotide
<400> 1
   gcagatctat ggctttcccg aagatg 26
<210> 2
   <211> 25
   <212> DNA
   <213> Artificial Sequence
<220>
   <223> Description of artificial sequence: synthetic polynucleotide
<400> 2
   gtctcgagtc agacaggcgg acaat 25
<210> 3
   <211> 58
   <212> DNA
   <213> Artificial Sequence
<220>
   <223> Description of artificial sequence: synthetic polynucleotide
<400> 3
   ccgggcaagt ggtttgtcag taaatctcga gatttactga caaaccactt gctttttg 58
<210> 4
   <211> 22
   <212> DNA
   <213> Artificial Sequence
<220>
   <223> Description of artificial sequence: synthetic polynucleotide
<400> 4
   agcggaaacg aaagaggtgg ac 22
<210> 5
   <211> 22
   <212> DNA
   <213> Artificial Sequence
<220>
   <223> Description of artificial sequence: synthetic polynucleotide
<400> 5
   cctgaggaca aaagcgatgg ac 22

## Description

### RELATED APPLICATIONS

This application claims the benefit of priority to USSN 62/107378 filed January 24, 2015 and USSN 62/266514, filed December 11th, 2015.

### FIELD

The present disclosure relates to methods and compositions useful for modulating the globoseries glycosphingolipid synthesis as well as markers useful for selecting cancer stem cells. Particularly, the present disclosure is directed to glycoenzyme inhibitor compound and compositions and methods of use thereof that can modulate the synthesis of globoseries glycosphingolipid SSEA-3/SSEA-4/GloboH in the biosynthetic pathway; particularly, the glycoenzyme inhibitors target the alpha-4GalT; beta-4GalNAcT-I; or beta-3GalT-V enzymes in the globoseries synthetic pathway. Additionally, the present disclosure is also directed to vaccines, antibodies, and/or immunogenic conjugate compositions targeting the SSEA-3/SSEA-4/Globo H associated epitopes (natural and modified) which can elicit antibodies and/or binding fragment production useful for modulating the globoseries glycosphingolipid synthesis. Moreover, the present disclosure is also directed to the method of using the compositions described herein for the treatment or detection of hyperproliferative diseases and/or conditions. Furthermore the present disclosure is also directed to markers useful for selecting cancer stem cells in diagnostic and/or therapeutic applications.

### BACKGROUND OF THE INVENTION

The carbohydrate antigens Globo H, stage-specific embryonic antigen-3 (SSEA-3), and stage-specific embryonic antigen-4 (SSEA-4) are closely related to one another in either structure or in function. Globo H, SSEA-3 and SSEA-4 are globoseries glycosphingolipids,¹⁻³ with SSEA-3 being the non-fucosylated pentasaccharide precursor structure of Globo H, SSEA-4 is sialylated SSEA-3 with sialic acid α2-3 links to the non-reducing end of galactose of SSEA-3.

Stage-specific embryonic antigen-3 (SSEA-3) was first identified and defined by the reactivity of an IgM monoclonal antibody generated in a rat immunized with 4- to 8-cell stage mouse embryos. This monoclonal antibody reacted with all mouse preimplantation embryos from oocytes up to the early blastocyst stage where its expression became more restricted, in the primitive endoderm after implantation. The SSEA-3 antigenic determinant was determined to be a carbohydrate present on glycolipids and glycoproteins; it was also found on human teratocarcinoma cells and human erythrocytes.⁴ In a panel of structures isolated from the 2102Ep human teratocarcinoma cell line, the SSEA-3 antibody had the highest affinity for Galβ(1-3)GalNAcβ(1-3)Galα(1-4)Galβ(1-4)Glcβ(1)Cer.⁵ This structure is also known as Gb5,⁶ galactosyl-globoside, or globopentaosylceramide.¹

Synthesis of SSEA-3 occurs when β1,3-galactosyltransferase V (β3GalT-V) transfers galactose to the GalNAc of globoside to form Gb5 or galactosyl-globoside. In more recent studies, attempts were made to determine if SSEA-3 could be used as a marker to identify stem cells in umbilical cord blood. It was determined that SSEA-3 was not expressed in hematopoietic or mesenchymal stem cells and therefore was not a good marker of multipotent cells. Schrump et al. immortalized lymph node lymphocytes from primary lung cancer patients, generated hybridomas, and selected for antibody secreting clones. Monoclonal antibodies were then generated from two of these clones--J309 and D579, which recognized the SSEA-3 antigenic determinant. The antibodies recognized SSEA-3 on several tumor cell lines including lung and breast cancer cell lines, and a teratocarcinoma cell line; in an immune adherence assay, rodent monoclonal SSEA-3 antibody, also referred to as MC631, reacted against the same cell lines as the J309 and D579 antibodies.⁸ SSEA-3 has also been found on testicular germ cell tumors,⁹ as well as in breast cancer and in BCSCs (breast cancer stem cells).

Chang et al. looked at SSEA-3 expression on normal tissues using a tissue microarray because its location outside of cancer and development was largely unknown. The group found SSEA-3 to be expressed on normal epithelium of colon, esophagus, small intestine, kidney, prostate, rectum, skin, testis, thymus, and uterine cervix. Expression was located only on the apical surfaces of epithelial cells or in the cytoplasm, which are considered immune system restricted or inaccessible sites.¹ In an experiment using a KLH conjugated Globo H monovalent vaccine in mice, an antibody response was made to only the Globo H antigen. When α-GalCer was added as an adjuvant, the amount of overall antibody production increased and the mice made polyclonal antibodies to both the Globo H, the SSEA-3 and the SSEA-4 antigen structures, which vaccination was unable to generate in the absence of the adjuvant.¹ This result showed that SSEA-3, Globo H and SSEA-4 could make promising targets for cancer vaccines and could be targeted simultaneously.

However, most tumor associated carbohydarte antigens have poor immunogenicity and many approaches have been developed to increase the immune response of carbohydrate-based vaccines, including conjugation with a carrier protein administration with an immunologic adjuvant using unnatural glycosidic linkage, clustered antigens, unimolecular polyvalent vaccine or hetero-glycan multivalent vaccine. Using these strategies, a few carbohydrate-based vaccines that could elicit significant immune responses to target glycan structures were designed for cancer therapy and entered clinical trials. Among them, the clinical trials of Theratope and GMK with adjuvant QS-21 failed to produce statistically significant difference between time-to-disease and overall survival rate. Probably these two vaccines could not elicit robust T cell-dependent immune response in patients. Specifically, Theratope and GMK induced a higher level of IgM in patients but could not induce a strong immune IgG response, which is a major problem in carbohydrate-based vaccine development.

Previous studies showed that modification of carbohydrate antigen structures (MCAS) could effectively elicit a higher level of immune response. For example, in the modification study of the capsular polysaccharide of group B meningococci, the *N*-acetyl groups of α-(2,8)-linked polysialic acid (PSA) was replaced with the *N*-propinoyl group and such a modification elicited a high antibody response to recognize not only the *N*-propinoyl PSA, but also the nature *N*-acetyl PSA.²⁰ Similar approaches were applied to STn²¹ and GM3²² antigens to produce high antibody titers against modified and nature forms. The results indicated that *N-*phenylacetyl, *N*-fluoroacetyl or *N*-difluoroacetyl modifications on glycan antigens could improve the immunogenicity. Moreover, the Schultz group reported that incorporation of a *p-*nitrophenylalanine into the tumor necrosis factor-α (TNF-α) could break immune tolerance and induce more antibody response to TNF-α.²³ Using glycans as antigens, although some progress has been achieved, most cases are the N-modification of disaccharide (STn), trisaccharide (GM3) and polysialic acid (PSA) and some are based on fluorinated MUC1 glycopeptide antigens.

The discovery of cancer stem cells (CSCs), which are responsible for self-renewal and tumor-growth in heterogeneous cancer tissues, has stimulated interests in developing new cancer therapies and early diagnosis. The markers currently used for isolation of CSCs, however, are often not selective enough to enrich CSCs for the study of this special cell population.

### SUMMARY OF THE INVENTION

The present invention is defined by the claims. The present disclosure relates to therapeutic methods which are based on the use of an agent that specifically targets tumor-associated carbohydrate antigen(s) and/or pathways regulating those targets, for the treatment of cancers comprising cancer stem cells and related diseases. Also provided are diagnostic and prognostic methods using one or more tumor-associated carbohydrate antigens as markers for cancer stem cells.

In one aspect, a binding agent is provided which specifically binds to one or more tumor-associated carbohydrate antigens for use in the treatment of cancers comprising cancer stem cells expressing said one or more tumor-associated carbohydrate antigens. Also provided is a pharmaceutical composition, comprising a respective binding agent for use in the treatment of cancers comprising cancer stem cells expressing said one or more tumor-associated carbohydrate antigens. Bindign agents include agents targeting SSEA3, SSEA4, and GloboH antigens, any combinations thereof and/or agents targeting related pathway targets.

In a second aspect, a method is provided for identifying a cancer comprising cancer stem cells that is susceptible to treatment with one or more binding agents each of which specifically binds to one or more tumor-associated carbohydrate antigens, wherein said treatment effects the cancer stem cells, the method comprising determining whether a cancer sample obtained from a patient comprises cancer stem cells that express one or more tumor-associated carbohydrate antigens the one or more binding agents is specific for, wherein the presence of said tumor-associated carbohydrate antigen(s) on cancer stem cells indicates that the cancer is susceptible to treatment with the binding agent(s) that specifically binds said tumor-associated carbohydrate antigen and wherein said treatment effects the cancer stem cells. In some embodiments, the tumor-asociated carbohydrate antigens are selected from the list consisting of SSEA3, SSEA4, and GloboH antigens.

In a third aspect, a method is provided for identifying a population of cancer stem cells, the method comprising
a) providing a starting population of cancer cells,
b) determining the expression level of one or more tumor-associated carbohydrate antigens,
c) selecting a population of cells whose expression level of said one or more tumor-associated carbohydrate antigens as determined in step b) is increased when compared to control cells, wherein said selected population of cells are cancer stem cells and
d) optionally isolating and/or enriching for said population of cells selected in step c),
wherein said control cells are cells from the same starting cancer cell population which do not express, or express lower levels of said one or more tumor-associated carbohydrate antigens.
In some aspects, the tumor-associated carbohydrate antigens are selected from the list consisting of SSEA3, SSEA4, and GloboH antigens. In some aspects, the determining step in b) further includes determining the expression level of one or more additional tumor associated antigens, and the selection and isolating steps in c) and d), respectively, also includes consideration of the expression level of said one or more additional tumor associated antigens. In some aspects, the one or more tumor-associated antigens include, but are not limited by CD24, CD44, PROCR, ESA, CD176, CD175, CD175s, CD174, CD173 and CA19-9 antigens. In some aspects, the determining step in b) and/or the isolation step in d) is carried out using FACS. In some aspects, the level of expression of said one or more tumor-associated carbohydrate antigens is high or highly increased, when compared to control cells. In some aspects, the level of expression of said one or more tumor-associated carbohydrate antigens is low or lowly increased, when compared to control cells.

In a fourth aspect, a method for diagnosing, staging and/or prognosing cancer and/or monitoring the susceptibility to treatment is provided, comprising the step of analyzing the expression of one or more tumor-associated carbohydrate antigens on cells in a sample isolated from a patient, wherein the presence of cells expressing the one or more tumor-associated carbohydrate antigens indicates the presence of cancer stem cells in said sample.

In another aspect, a kit for use in a method disclosed herein is provided, comprising a binding agent which specifically binds to one or more tumor-associated carbohydrate antigens and instructions for use in a method disclosed herein.

In another aspect, a method is provided for screening a candidate therapeutic agent, e.g. a chemotherapeutic agent or another anti-cancer drug, for effectiveness against a cancer comprising cells that express one or more tumor-associated carbohydrate antigens as described herein, the method comprising:
a. providing a cancer sample comprising cells that express one or more tumor-associated carbohydrate antigens,
b. contacting said agent with the cells, and
c. determining the effectiveness of said agent against said tumor-associated carbohydrate antigen positive cancer cells.

The present disclosure also relates to methods and compositions which can modulate the globoseries glycosphingolipid synthesis. Particularly, the present disclosure is directed to glycoenzyme inhibitor compound and compositions and methods of use thereof that can modulate the synthesis of globoseries glycosphingolipid SSEA-3/SSEA-4/GloboH in the biosynthetic pathway; particularly, the glycoenzyme inhibitors target the alpha-4GalT; beta-4GalNAcT-I; or beta-3GalT-V enzymes in the globoseries synthetic pathway. Additionally, the present disclosure is also directed to vaccines, antibodies, and/or immunogenic conjugate compositions targeting the SSEA-3/SSEA-4/Globo H associated epitopes (natural and modified) which can elicit antibodies and/or binding fragment production useful for modulating the globoseries glycosphingolipid synthesis. Moreover, the present disclosure is also directed to the method of using the compositions described herein for the treatment or detection of hyperproliferative diseases and/or conditions. In addition, the disclosure is also directed to cancer stem cell markers useful for selecting cancer stem cells (e.g. breast cancer) in diagnostic and/or therapeutic applications.

The present disclosure is also based on a surprising discovery is that the inhibition or silencing the galactosyl transferase (beta3GalT5) for the biosynthesis of SSEA3 abolishes the grow of cancer stem cell. This finding imply the stage-specific embryonic antigen SSEA-3 can serve as a target for development of therapeutics and vaccines. Moreover, the 3 enzymes alpha4GalT, beta4GalNAcT-I, and beta3GalT-V those involved in the biosynthesis of SSEA-3 can be targets for inhibitors development.

The present disclosure is also based on the discovery that the modification of the stage-specific embryonic antigens (SSEA3 and SSEA4) with certain groups disclosed herein elicited robust IgG antibody response to specifically recognize SSEA3 and SSEA4, respectively. The antibodies induced by an immmunogenic composition comprising such unnatural glycan moiety are able to mediate the complement-dependent cell cytotoxicity against tumor cells.

Accordingly, the present disclosure features the design of antibodies against SSEA-3 for treating cancers. The present disclosure also features novel compounds consisting of the modified carbohydrate antigens (SSEA3, SSEA4), glycan conjugates comprising such, and immmunogenic compositions and vaccines thereof.

Present disclosure provides inhibitor compound which modulates the globoseries synthetic pathway and optionally at least one pharmaceutically acceptable carrier for the treatment of a proliferative disease, especially a proliferative disease in which the globoseries pathway is concomitantly dysregulated; a pharmaceutical composition comprising such a composition; the use of such a composition for the preparation of a medicament for the treatment of a proliferative disease; a commercial package or product comprising such a preparation; and to a method of treatment of a warm-blooded animal, especially a human.

The present disclosure is based on a surprising discovery that the inhibition or silencing the glycoenzymes such as galactosyl transferase (β3GalT5), alpha-4GalT, and beta-4GalNAcT-I for the biosynthesis of SSEA3 abolishes the growth of cancer cells and cancer stem cell. This finding imply the stage-specific embryonic antigen SSEA-3 and/or SSEA4 and/or Globo-H can serve as a target for development of therapeutics and vaccines.

The present disclosure is also based on the discovery that the modification of the stage-specific embryonic antigens (SSEA3 and SSEA4) with certain groups disclosed herein elicited robust IgG antibody response to specifically recognize SSEA3 and SSEA4, respectively. The antibodies induced by an immmunogenic composition comprising such unnatural glycan moiety are able to mediate the complement-dependent cell cytotoxicity against tumor cells.

Accordingly, the present disclosure features the design of antibodies against SSEA-3 and/or SSEA4 for treating cancers. The present disclosure also features novel compounds consisting of the modified carbohydrate antigens (SSEA3 and SSEA4), glycan conjugates comprising such, and immmunogenic compositions and vaccines thereof.

In one aspect, the present disclosure provides a compound of formula **(I)**: 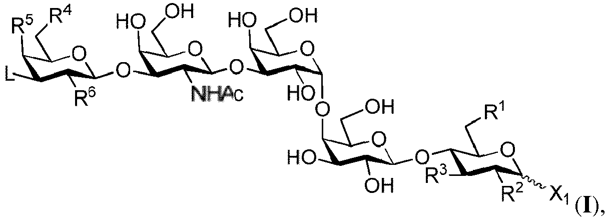 or a salt thereof, wherein X₁, R¹, R², R³, R⁴, R⁵, R⁶ and L are as described herein. In certain aspects, a compound of Formula **(I)** is useful for making an immunogenic composition for treating cancers.

In another aspect, the present disclosure provides a compound of Formula **(II):** 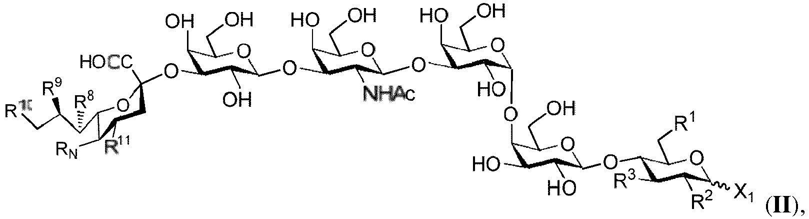 or a salt thereof, wherein X₁, R¹, R², R³, R⁸, R⁹, R¹⁰, R¹¹ and R_{N} are as described herein. In certain aspects, a compound of Formula **(II)** is useful for making an immunogenic composition for treating cancers.

In another aspect, the present disclosure provides an immunogenic composition, comprising (a) a glycan conjugate including a carrier and one or more glycans, and optionally (b) an adjuvant,
wherein: each of the one or more glycans is conjugated with the carrier through a linker, having the formula **(III)** or **(IV):** 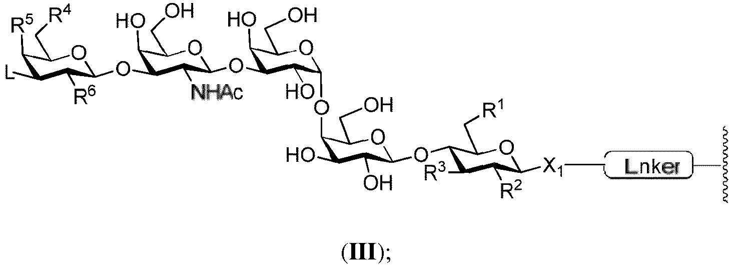 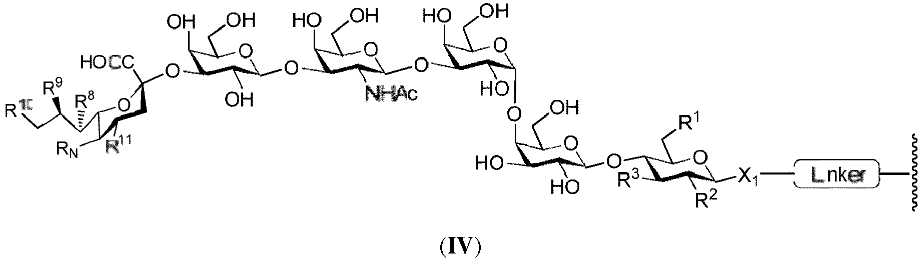 wherein X₁, R¹, R², R³, R⁴, R⁵, R⁶, R⁸, R⁹, R¹⁰, R¹¹, L and R_{N} are as described herein.
In certain aspects, it is contemplated that any construct of vaccine containing a combination of any one or more of the three glycans (SSEA3, SSEA4 and Globo-H) and analogs thereof in any ratio can be linked to a carrier. 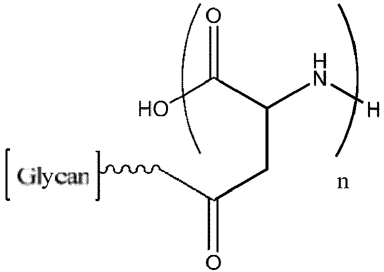
wherein n can be an integer from 1 to 10;
wherein Glycan can be selected from the group consisting of Formulas I, II, III, and IV;
wherein if n is 2 or more, each Glycan can be the same as another Glycan on the aspartyl peptide or a difference Glycan on the aspartyl peptide.
In some aspects, Glycan can be selected from the group consisting of SSEA-3, SSEA-4, and Globo-5. In some aspects, the exemplary multivalent construct can be: 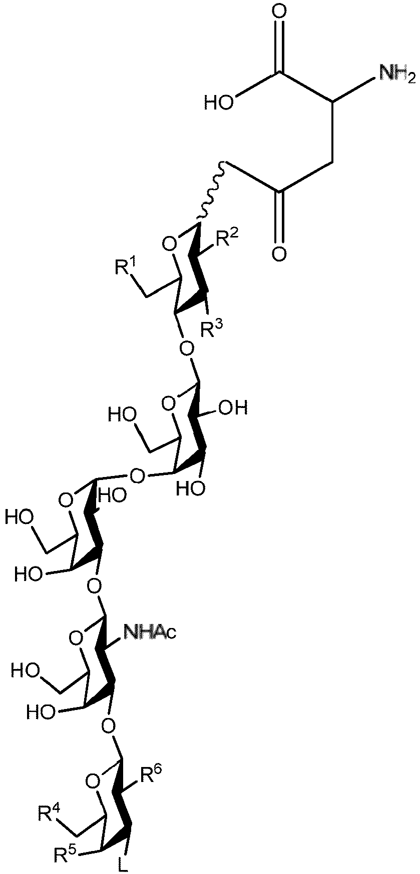 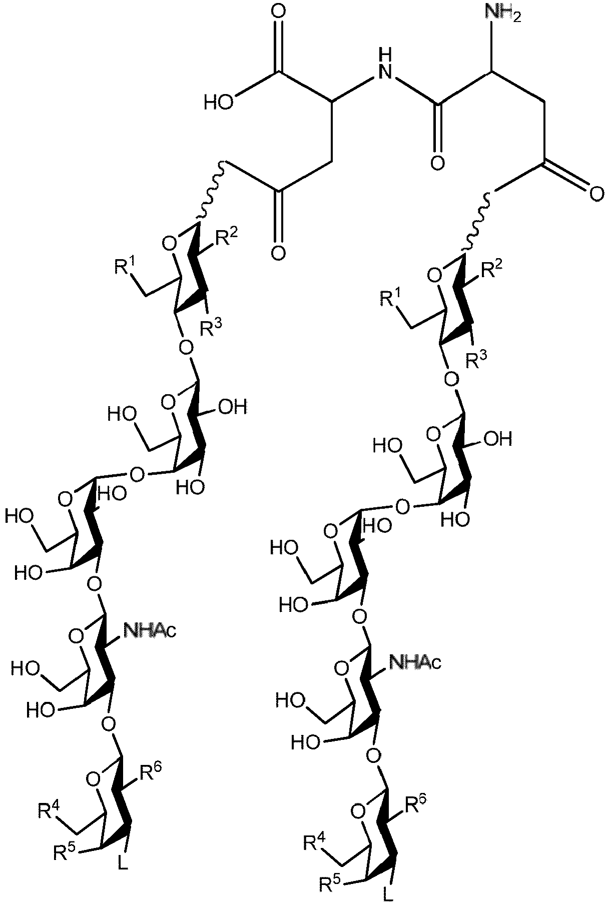 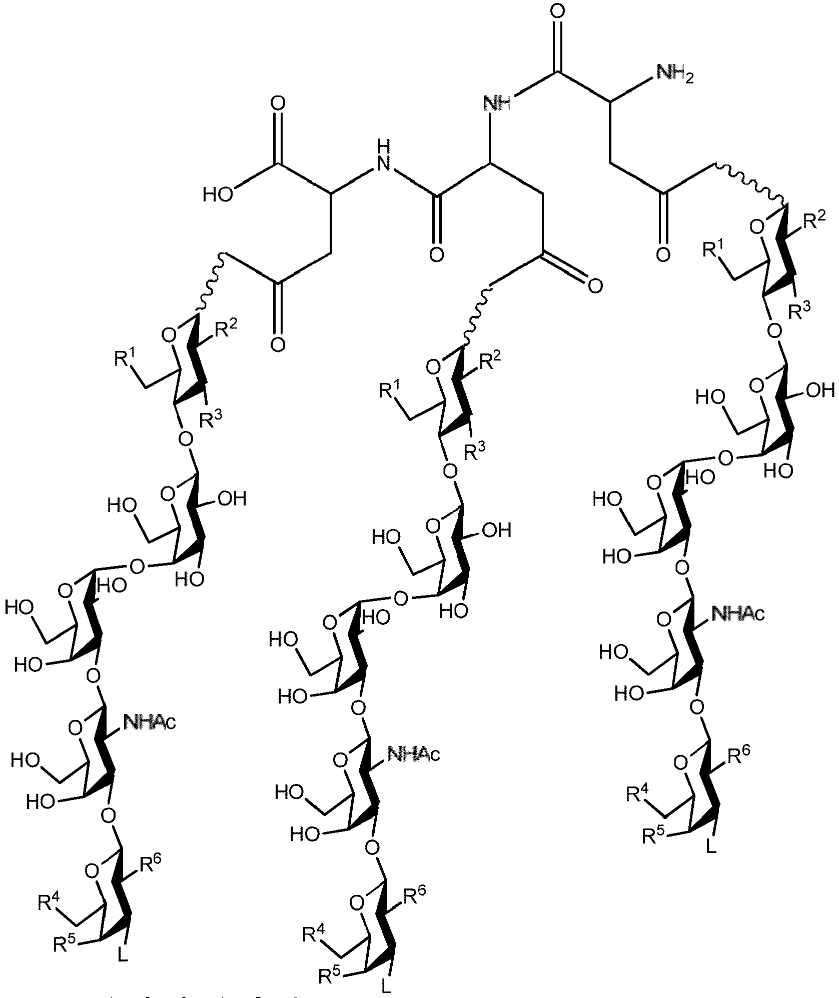 Wherein R¹, R², R³, R⁴, R⁵, R⁶, and L on each Glycan moiety can be the same or different.

In certain aspects, the immmunogenic composition of the disclosure comprises an adjuvant. Adjuvants suitable for the invention are as described herein.

In certain aspects, the immunogenic composition is capable of eliciting an immune response against a cancer cell in a subject. In certain aspects, the cancer cell is selected from the group consisting of a brain cancer cell, a lung cancer cell, a breast cancer cell, an oral cancer cell, an esophagus cancer cell, a stomach cancer cell, a liver cancer cell, a bile duct cancer cell, a pancreas cancer cell, a colon cancer cell, a kidney cancer cell, a bone cancer cell, a skin cancer cell, a cervix cancer cell, an ovary cancer cell, and a prostate cancer cell.

In certain aspects, the immune response includes generation of antibodies that specifically bind to one or more of the antigens selected from the group consisting of Globo H, SSEA-3 and SSEA-4. In certain aspects, the antibodies are developed to neutralize one or more of Globo H, SSEA-3 and SSEA-4 expressed on the surface of cancer cells or cancer stem cells. In certain aspects, the antibodies predominantly include IgG antibodies. In certain aspects, the immmunogenic compositions provided herein mainly induce IgG1, IgG2b, IgG2c and IgG3.

Further, the present disclosure features monoclonal antibodies and binding fragments raised against the immunogenic composition described herein.

In one aspect, the antibody is a human antibody.

In one aspect, the antibody is a humanized antibody.

In one aspect, the antibody is specifically targeted against one or more of SSEA4, SSEA3, or Globo-H.

In one aspect, the antibody is specifically targeted against SSEA3.

In one aspect, the antibody is specifically targeted against SSEA4.

In one aspect, the antibody is a homogeneous antibody having the biantennary glycan terminated by two sialic acid in alpha-2,6-linkage.

In one aspect, the present disclosure provides a pharmaceutical composition comprising an effective amount of the antibody or antigen-binding fragment specifically targeted against one or more of SSEA4, SSEA3, or Globo-H and a pharmaceutically acceptable carrier

In one aspect, the pharmaceutical composition comprises a combination of antibodies and/or binding fragment thereof each independently targeting one or more of the SSEA4, SSEA3, or Globo-H glycans.

In one aspect, the pharmaceutical composition is useful for the treatment of cancer, infectious diseases, and/or anti-inflammatory diseases,

In one aspect, the pharmaceutical composition comprises antibodies or binding fragments thereof having universal biantennary n-glycan terminated with sialic acid in alpha-2,6-linkage.

In another aspect, the present disclosure provides a cancer vaccine comprising an immmunogenic composition described herein and a pharmaceutically acceptable excipient.

In another aspect, the present disclosure provides methods for treating and/or reducing the risk for cancer in a subject comprising administering to a subject in need thereof a therapeutically effective amount of an immunogenic composition or a cancer vaccine as described herein.

The treatment results in reduction of tumor size, elimination of malignant cells, prevention of metastasis, prevention of relapse, reduction or killing of disseminated cancer, prolongation of survival and/or prolongation of time to tumor cancer progression.

In some aspects, the treatment further comprises administering an additional therapy to said subject prior to, during or subsequent to said administering of the immunogenic composition or the cancer vaccine described herein. In some aspects, the additional therapy is treatment with a chemotherapeutic agent. In some aspects, the additional therapy is radiation therapy.

Another aspect of the present disclosure features a method of vaccinating a mammal against cancers, comprising administering to the mammal a pharmacologically effective amount of an immunogenic composition or a cancer vaccine as described herein.

In some aspects, the mammal is a human. In some embodiments, the immunogenic composition or the cancer vaccine described herein is administered subcutaneously.

Examples of the cancer include, but are not limited to, brain cancer, lung cancer, breast cancer, oral cancer, esophagus cancer, stomach cancer, liver cancer, bile duct cancer, pancreas cancer, colon cancer, kidney cancer, cervix cancer, ovary cancer and prostate cancer. In some aspects, the cancer is brain cancer, lung cancer, breast cancer, ovarian cancer, prostate cancer, colon cancer, or pancreas cancer.

In another aspect, the present disclosure provides methods of synthesizing the compounds of the invention as described herein.

In yet another aspect, the present disclosure features the process for making an immunogenic composition or a cancer vaccine as described herein.

The details of certain embodiments of the invention are set forth herein. Other features, objects, and advantages of the invention will be apparent from the Detailed Description, the Figures, the Examples, and the Claims.

### BRIEF DESCRIPTION OF THE DRAWINGS

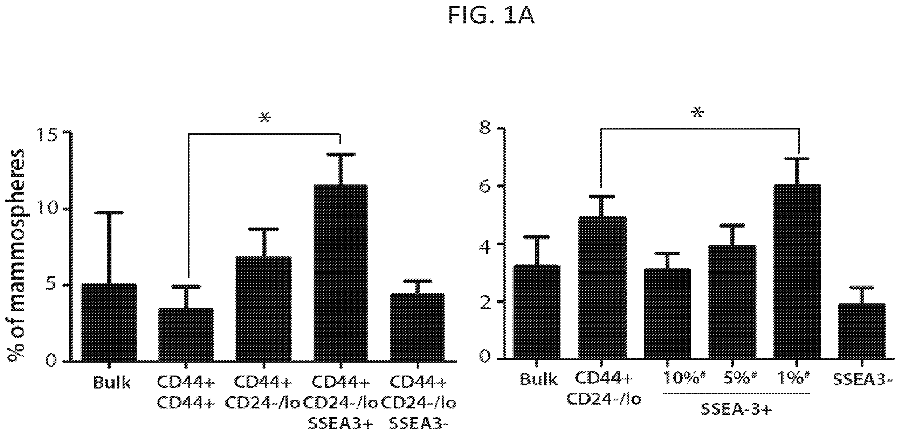

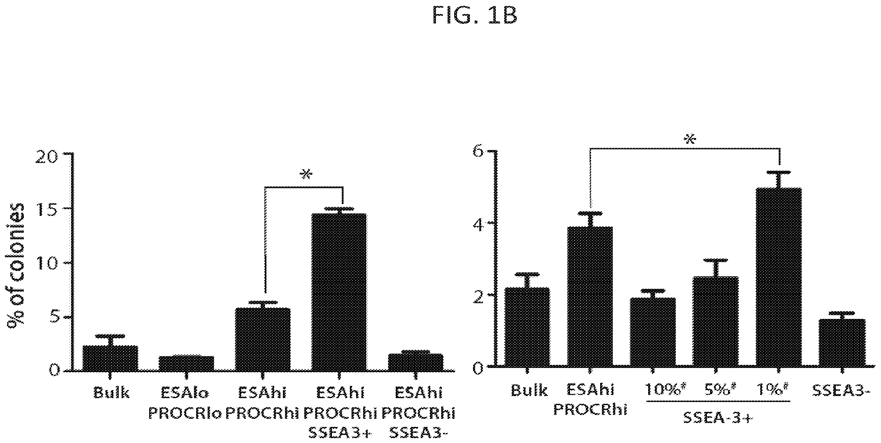

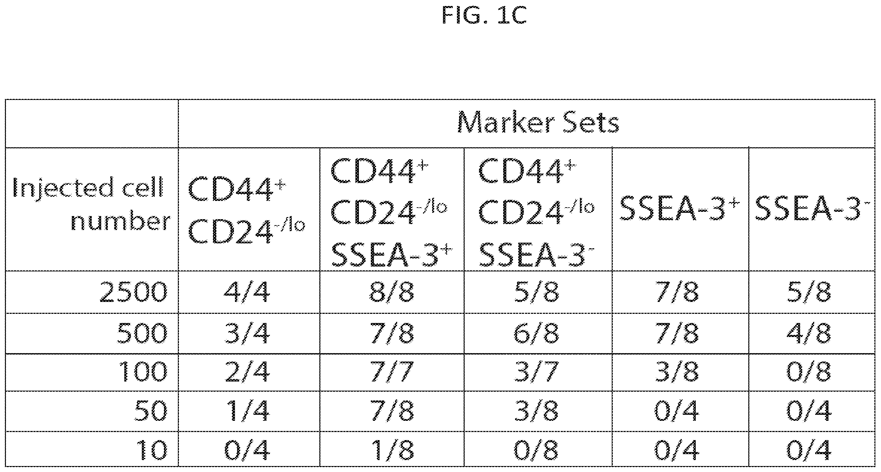

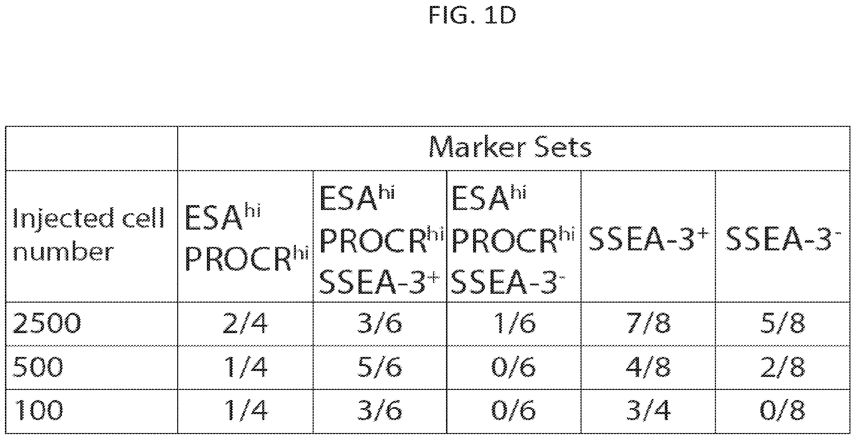

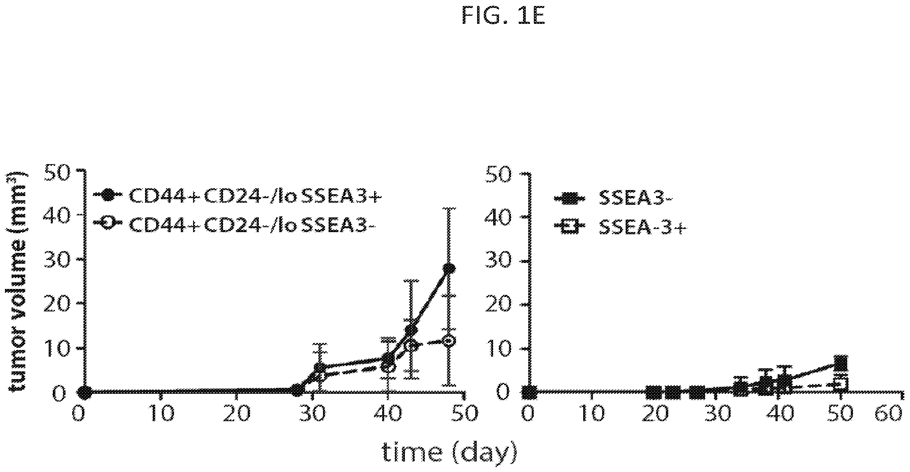

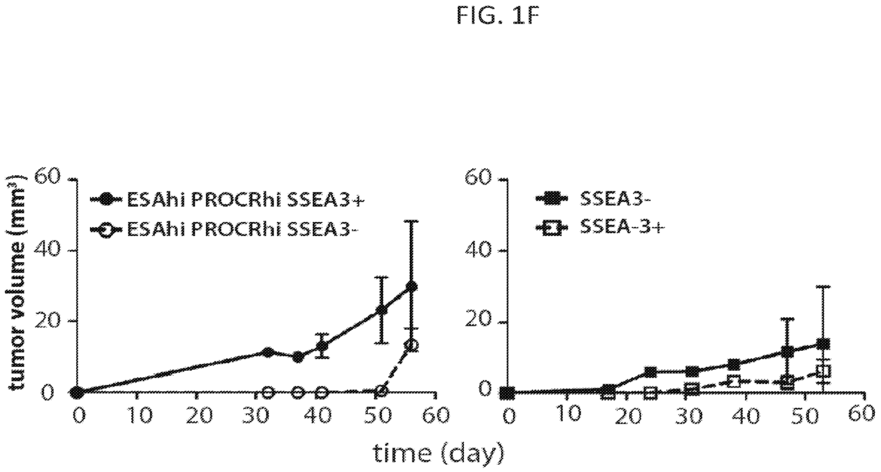

**Figure 1** **the tumorigenicity of cells carrying conventional markers and SSEA-3 was higher than other subpopulations. A, C** Percentage of cell colony or mammosphere formation of the subpopulation isolated by selected marker(s) for suspension culture or soft agar assay in MCF-7 or MDA-MB-231 respectively. Graphs are the triple samples from one representative experiment. **B, D** The number of tumor formed in mammary gland of NS injected with selected marker-expressing cell subpopulations from breast cancer cell lines MDA-MB-231 and MCF-7. The corresponding limiting dilution assay was done *in vivo.* **E, F** The tumor volume from different subpopulations of MDA-MB-231 (2500 cells/injection), and MCF-7 (500 cells/injection) was monitored and compared (n=4 tumors per group). ^{#}the percentage of SSEA-3⁺ cells sorted in the total cell population. Data represent the mean and standard deviation (S.D.). Asterisks indicate statistical significance, *p*< 0.05.

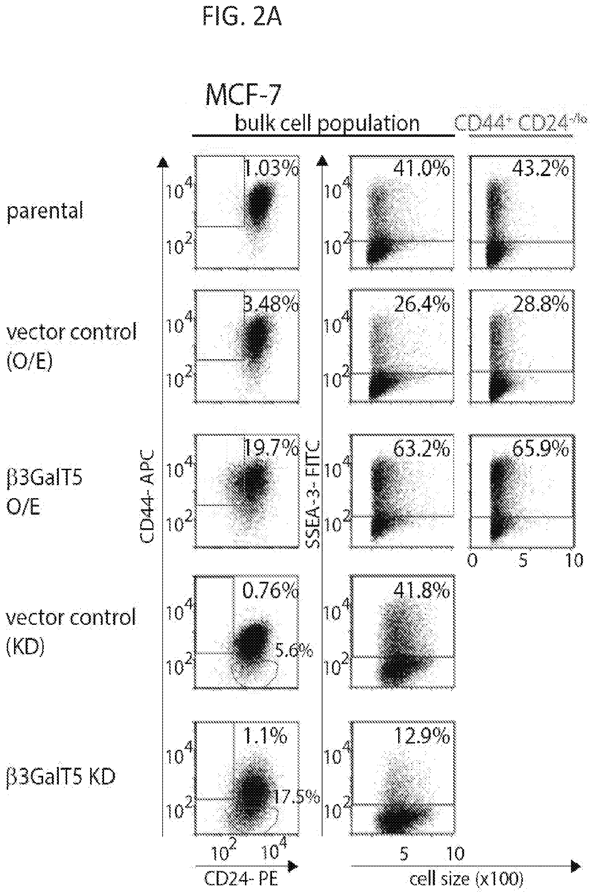

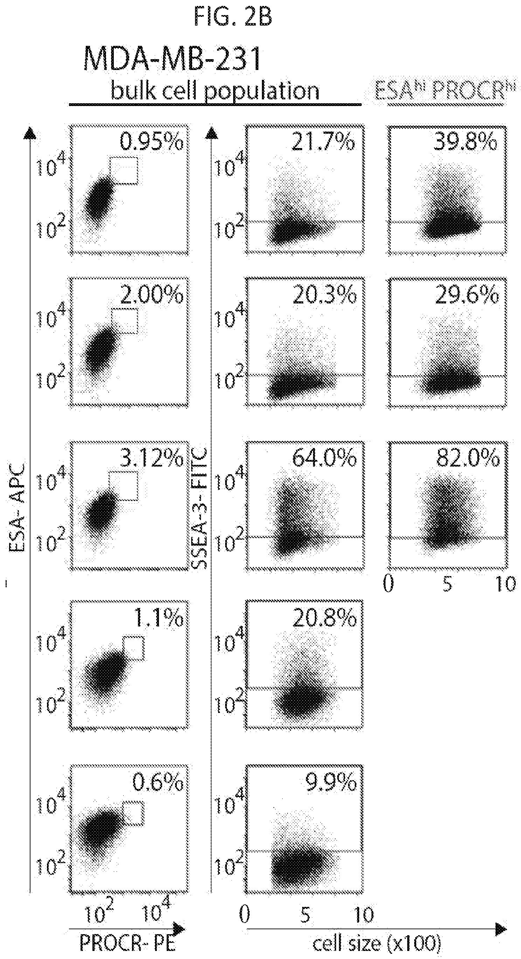

**Figure 2** Knockdown or overexpression of β3GalT5 in MCF-7 and MDA-MB-231 cell culture reduced or increased the level of SSEA-3 on cell surface and sternness properties by FACS analysis. The expression of cancer stem cell markers and SSEA-3 in parental cells. The level of SSEA-3 in subpopulations CD44⁺CD24^{-/lo} and ESA^{hi} PROCR^{hi} gated in overexpressed β3GalT5 group was also determined. A The expression of CD24, CD44 and SSEA-3 in MCF-7 with overexpression, knocked down of β3GalT5 or their corresponding vector control. B The expression of ESA, PROCR and SSEA-3 in MDA-MB-231 with overexpression, knockdown down of beta-3GalT5 or their corresponding vector control. All of experiments are the representative sample from triplicate.

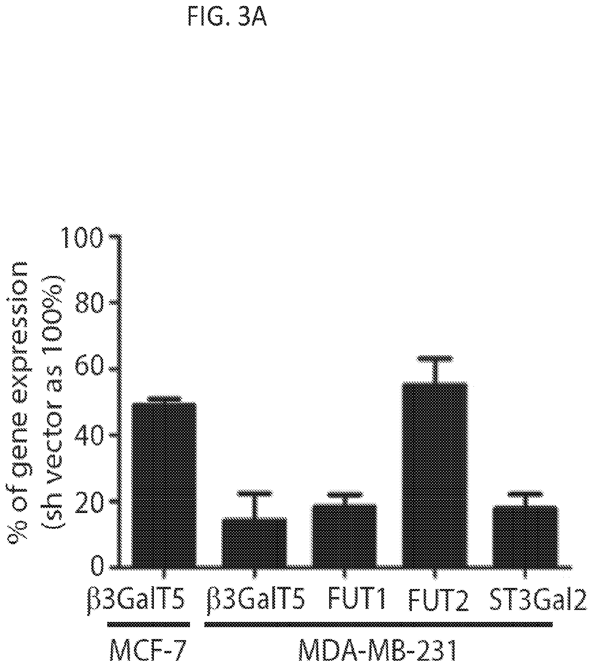

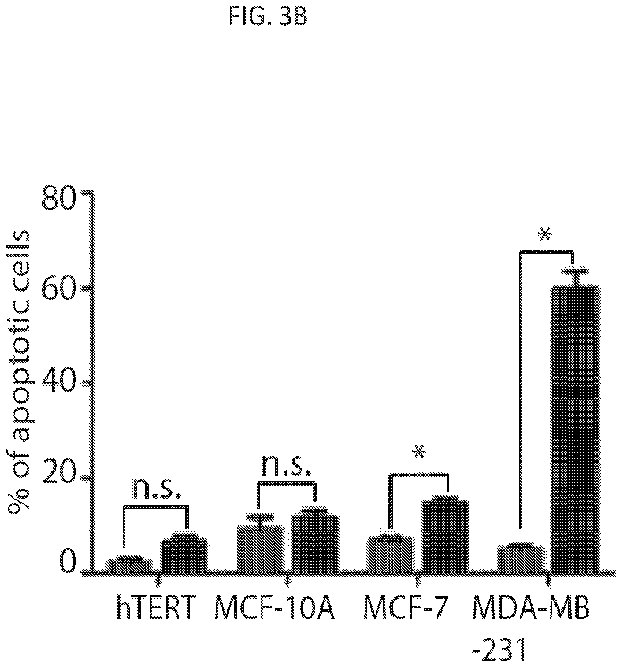

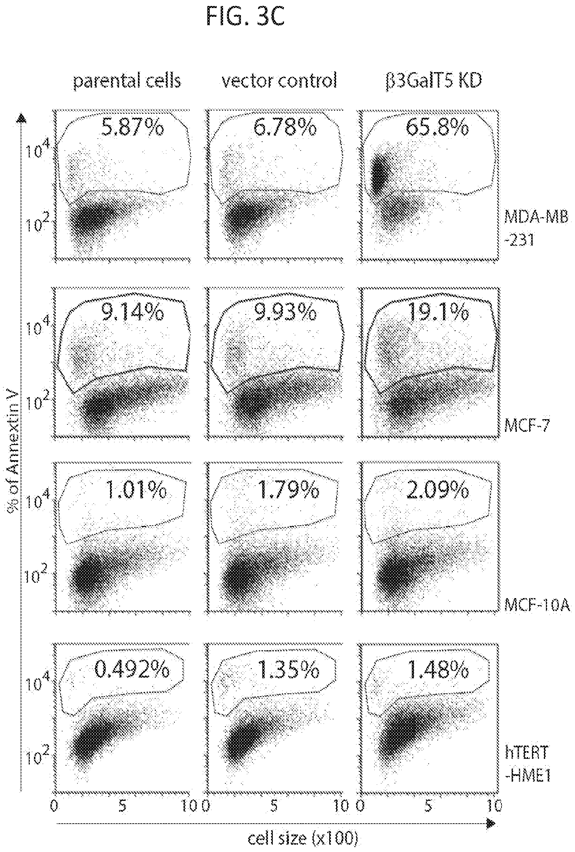

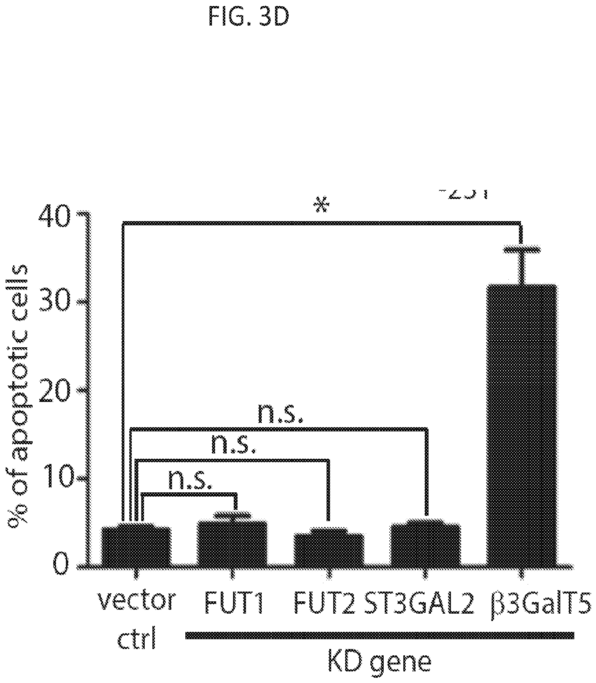

**Figure 3****- the induction of apoptosis in β3GalT5 knockdown cell lines.** A The relative percentage of gene expression (β3GalT5 in MCF-7, β3GalT5, FUT1, FUT2 and ST3Gal2 in MDA-MB-231) after knocking down the respective genes. The percentage of gene expression in vector control cells were normalized to 100. B. The mean percentage of apoptosis in breast normal (hTERT-HME1, MCF-10A) and cancer (MCF-7, MDA-MB-231) cell lines from three experiments. **C** Flow cytometric analysis of the apoptosis percentage in breast cancer cell lines MDA-MB-231, MCF-7, and breast non-cancer lines hTERT-HME1 and MCF-10A was examined after knockdown of beta3GalT5 for 4 days. The apoptotic cells were compared with unstained cells and gated. **D** The percentage of apoptosis in MDA-MB-231 cells with knockdown of gene FUT1, FUT2, ST3Gal2 or beta3GalT5 and vector control. The mean of apoptotic cells is from three experiments. Asterisks indicate statistical significance, *p*< 0.05; n.s. not significant.

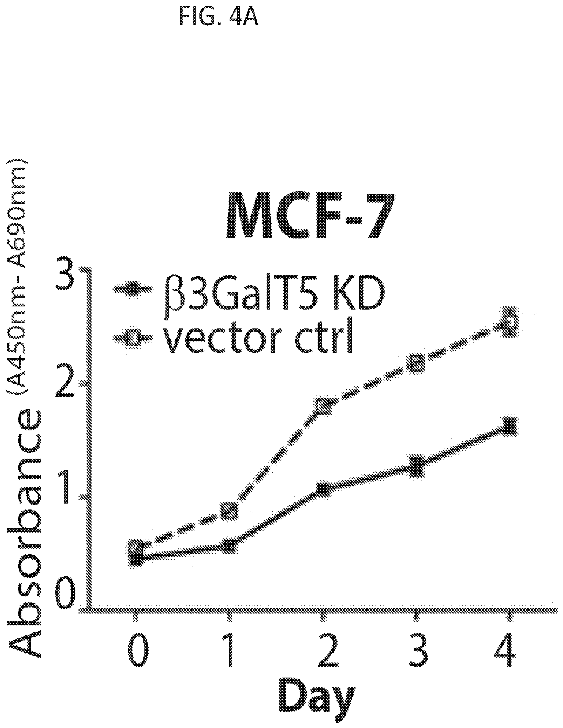

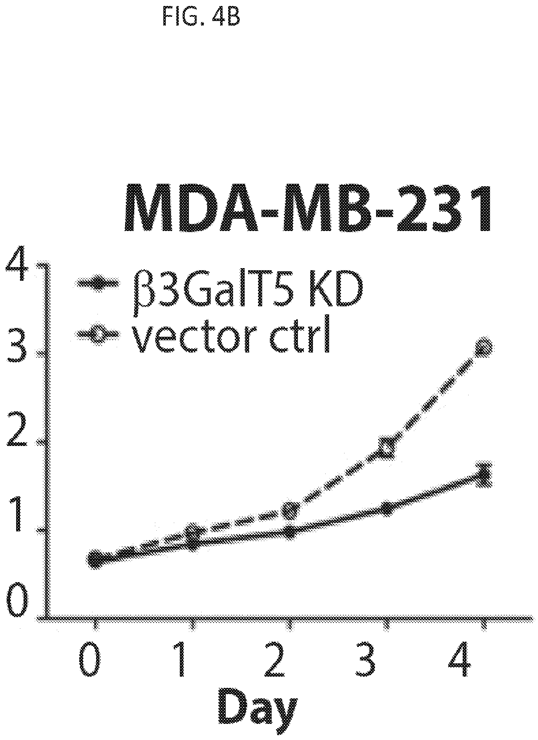

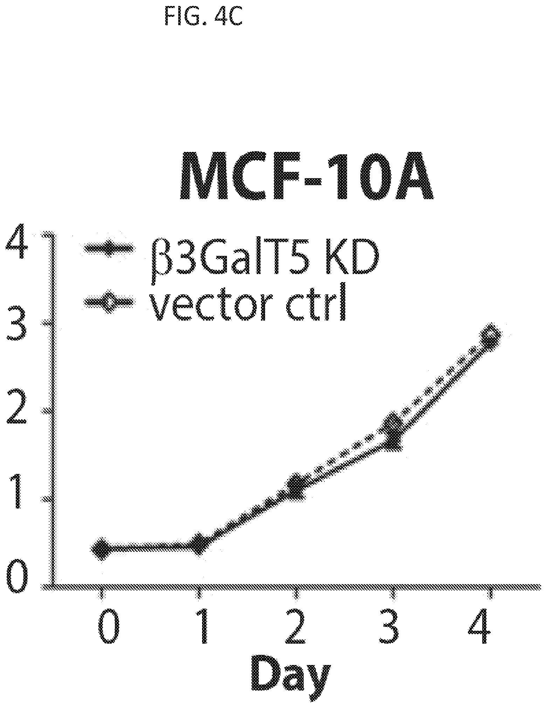

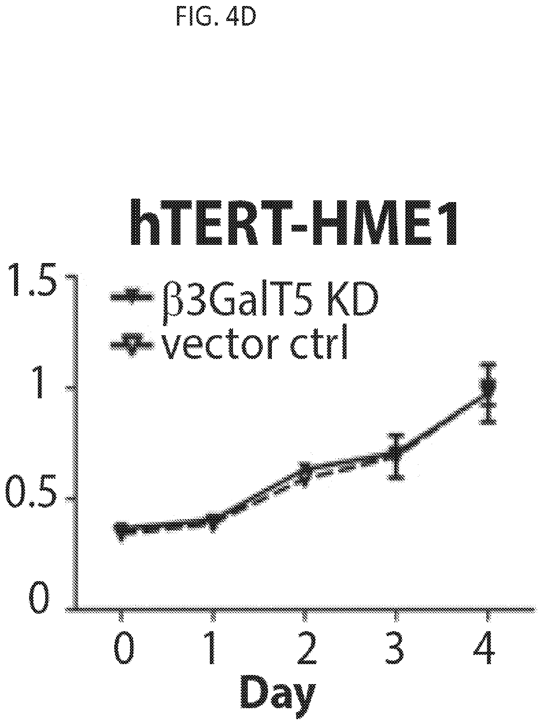

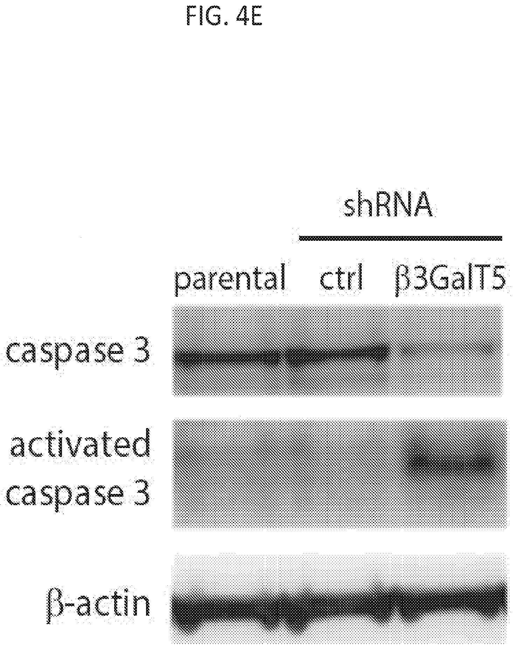

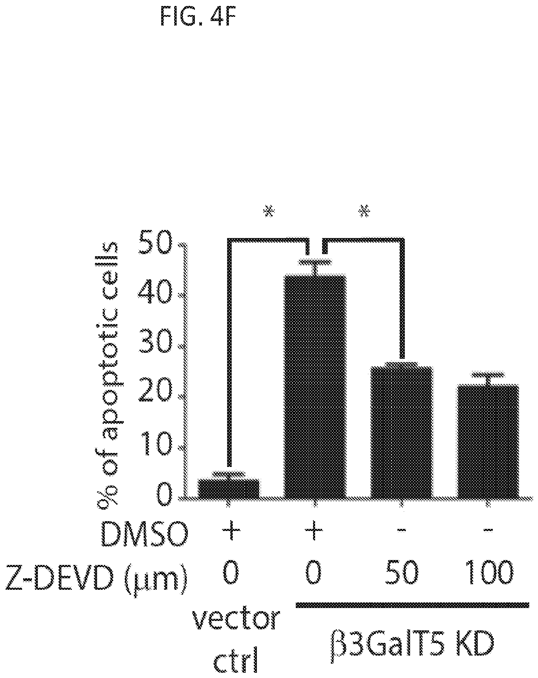

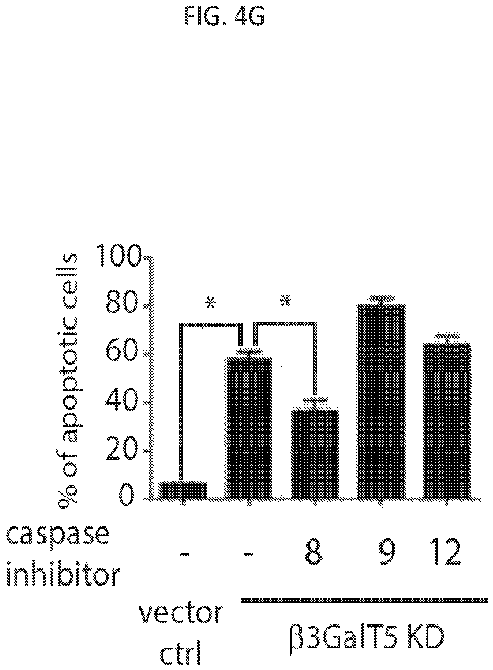

**Figure 4****-** Knockdown of β3GalT5 caused reduced proliferation rate and increased apoptosis in cancer cell culture but no effect on normal breast cell culture. A-D The rate of proliferation in cancer cell culture MCF-7 and MDA-231, as well as breast normal cell culture MCF-10A and hTERT-HME1. Proliferation rate, in terms of absorbance ^{(A450nm- A690nm)}, is the triplicate from a representative sample. E MDA-MB-231 cells infected with shRNA *beta3GalT5* or shRNA vector were lysed and whole-cell extract, cytoplasmic and nuclear fractions were prepared. Top, western-blot analysis of anti-caspase-3 antibody; middle, that of cleaved caspase-3 antibody; bottom, that of β-actin (served as a loading control) F, G The percentage of apoptotic MDA-MB-231 cells with β3GalT5 knockdown. MDA-MB-231 cells were treated with caspase-3 inhibitors Z-DEVD in different concentrations or the inhibitors for caspase-8, 9 or 12. Data represent here is the mean and standard deviation (S.D.) from triplicated sample. Asterisks indicate statistical significance, *p*< 0.05; n.s. not significant.

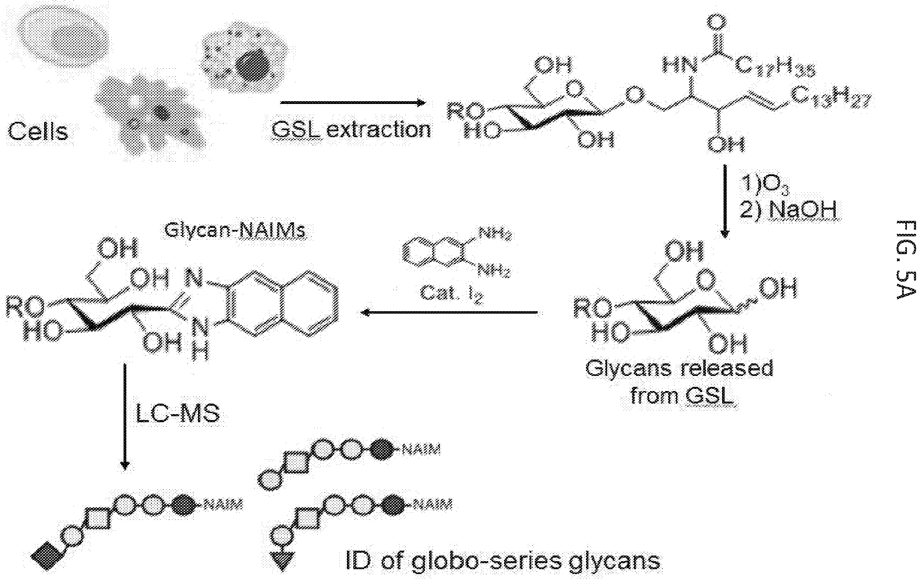

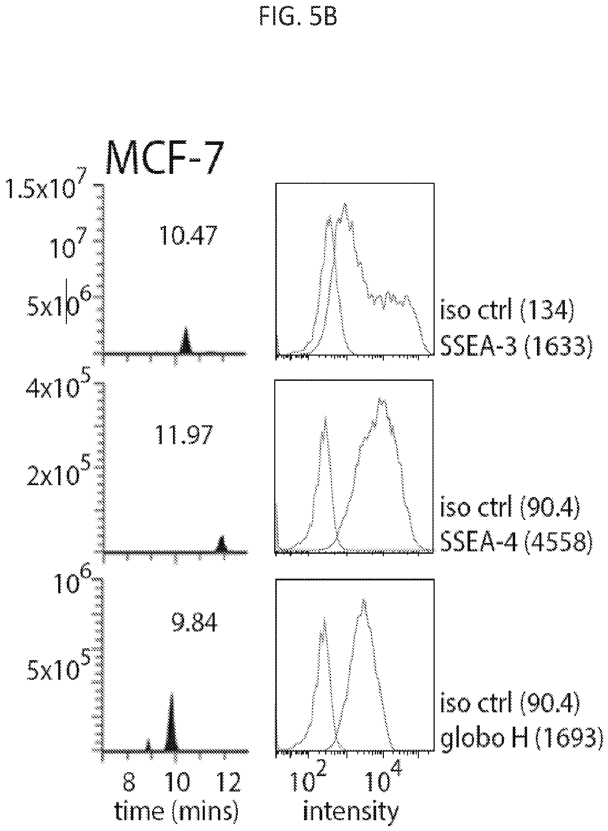

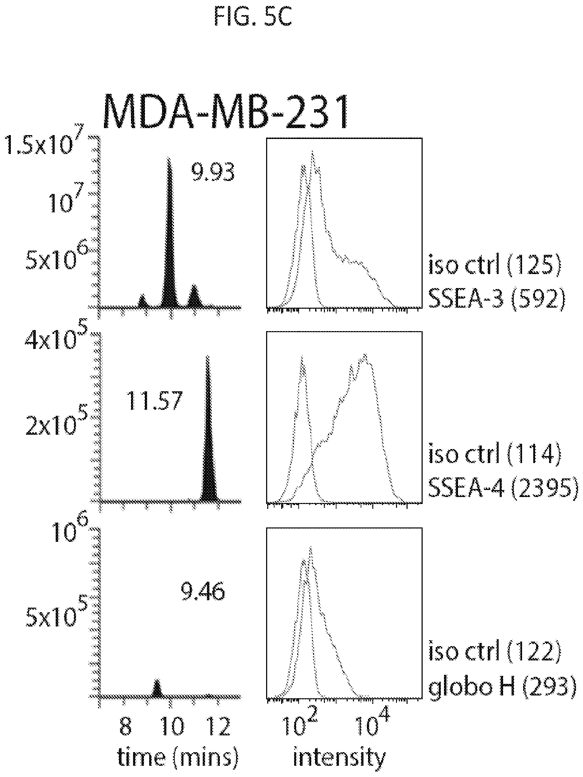

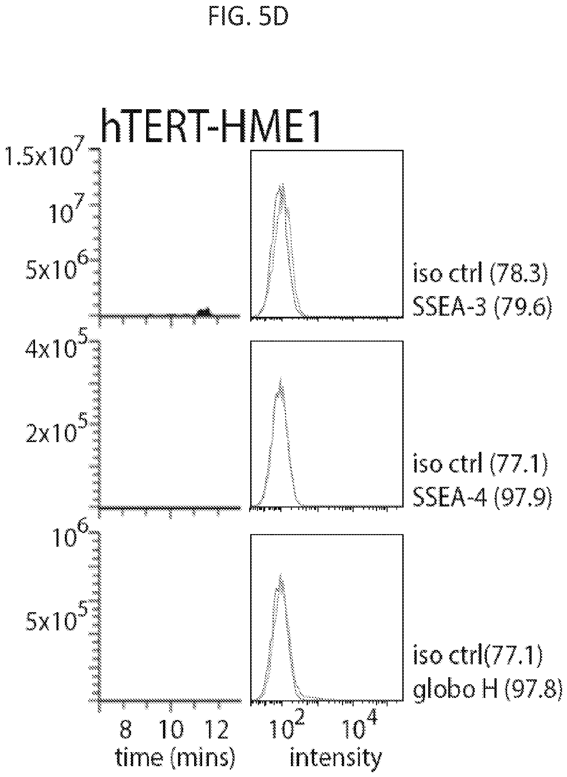

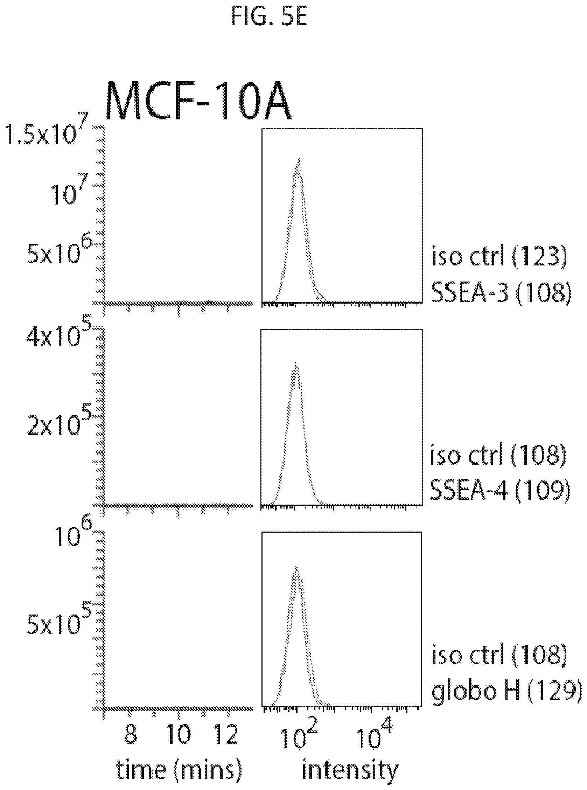

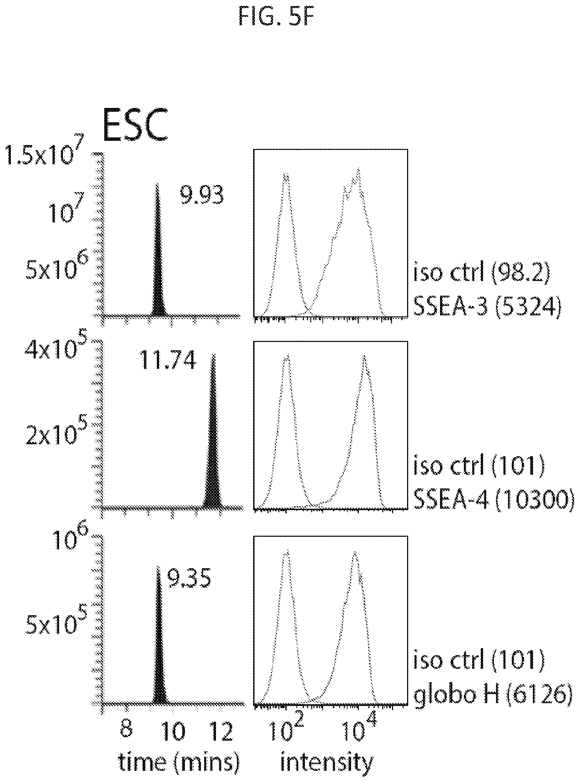

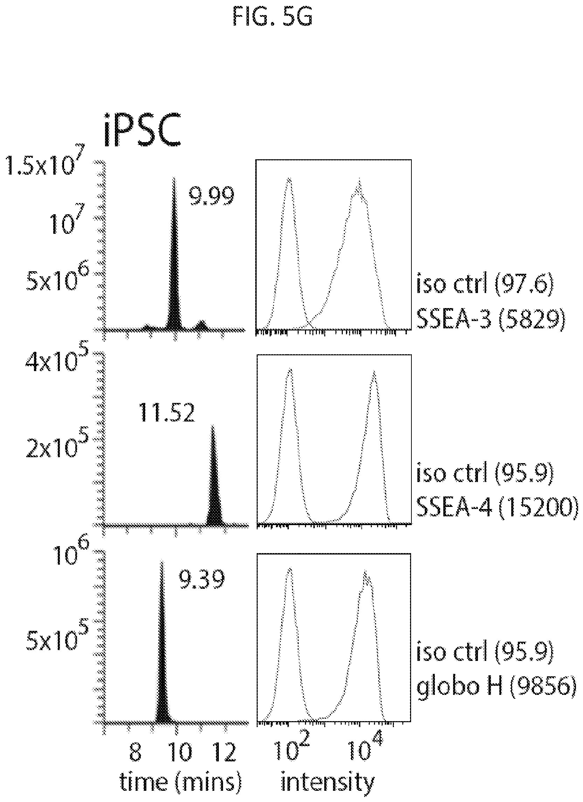

**Figure 5****-** the comparison of the abundance of globo-series epitopes in cell lines by flow cytometry and mass spectrometry. A The scheme of extraction of glycan from glycolipid on cells for fluorescent labeling and LC-MS analysis. B-G The relative abundance of globo-series epitopes SSEA-3, SSEA-4 and globo-H in breast cancer cell lines MCF-7 and MDA-MB-231, normal cell lines hTERT-HME1 and MCF-10A, embryonic stem cells (ESC) as well as induced pluripotent stem cells (iPSC) was detected by FACS and mass spectrometry. For figures of flow cytometry, histograms of cells stained with anti-glycan antibodies (in red, blue or green) and their corresponding antibody isotype controls (in gray) were shown. Geometric mean of fluorescence was shown in the bracket. For MS, the retention times of m/z (SSEA-3=1008.3667, SSEA-4=1299.4621 and globo-H=1 154.4246) were shown in graphs.

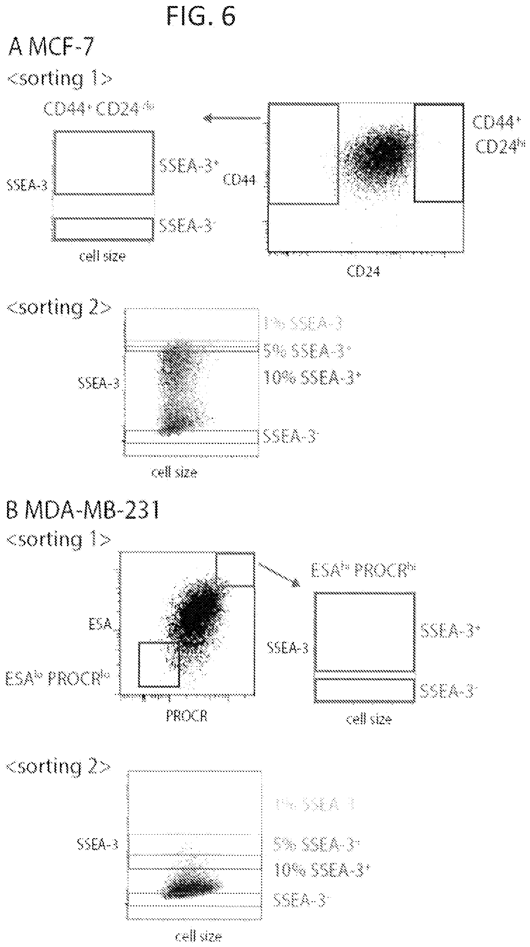

**Figure 6****. (Fig. S1)- the subpopulations in cell lines obtained by sorting for *in vitro* and *in vivo* assays.** Subpopulations including A CD44⁺ CD24^{hi}, CD44⁺ CD24^{-/lo}, CD44⁺ CD24^{-/lo} SSEA-3⁺, CD44⁺CD24^{/lo} SSEA-3⁻, various percentages of SSEA-3⁺ (top 1, 5, 10%), and SSEA-3⁻ in MCF-7, as well as **B** ESA^{lo}PROCR^{lo}, ESA^{hi}PROCR^{hi}, ESA^{hi}PROCR^{hi}SSEA-3⁺, ESA^{hi}PROCR^{hi}SSEA-3⁻, various percentages of SSEA-3⁺ (top 1, 5, 10%), and SSEA-3⁻ in MDA-MB-231 were enriched by cell staining (as the methods and materials) and flow cytometry for the further analysis.

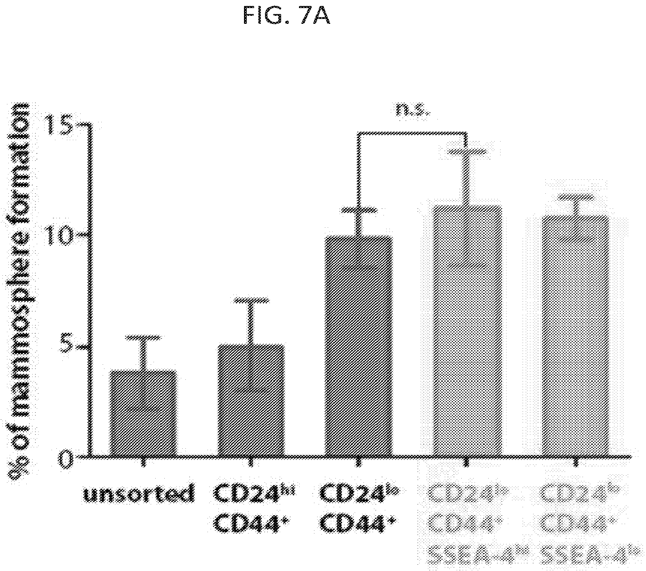

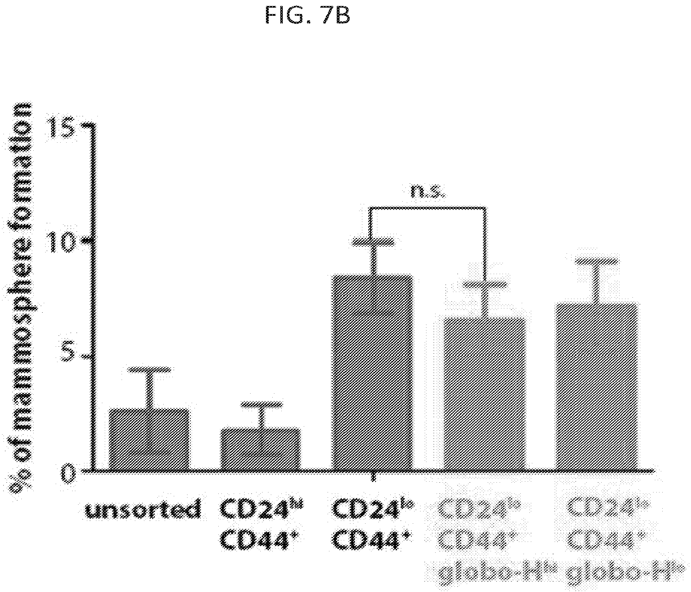

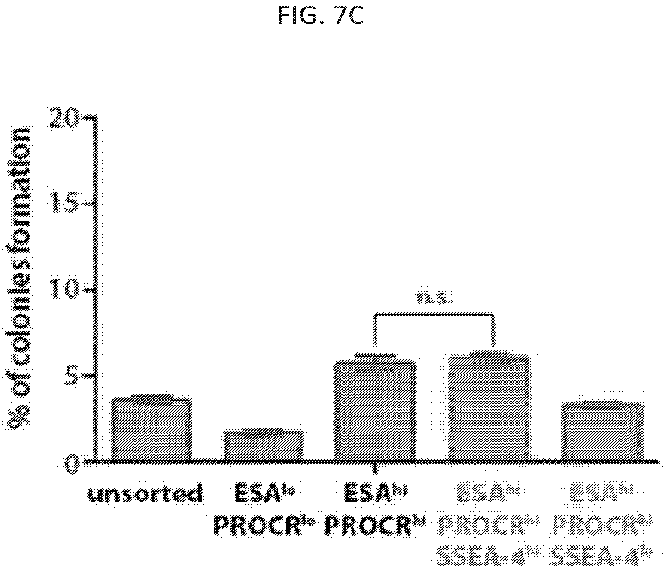

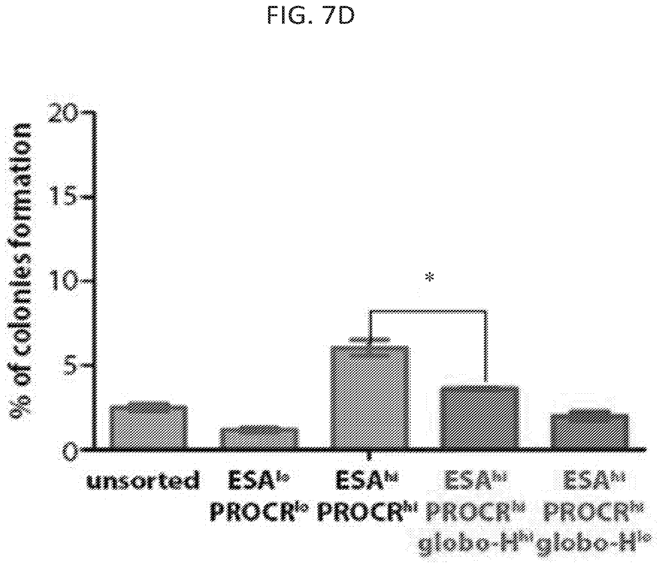

**Figure 7** **(Fig. S2)- BCSCs were not enriched with globo-series epitopes SSEA-4 and globo-H by *in vitro* assays. A-D** Percentage of cell colony formation of unsorted cells or selected marker (known marker set CD24/CD44, or ESA/PROCR, along with SSEA-4 or globo-H) expressing cell subpopulation from breast cancer cell lines MCF-7 and MDA-MB-231. Graphs are the triple samples from one representative experiment. Data represent the mean and standard deviation (S.D.). Asterisks indicate statistical significance, *p<* 0.05; n.s. not significant.

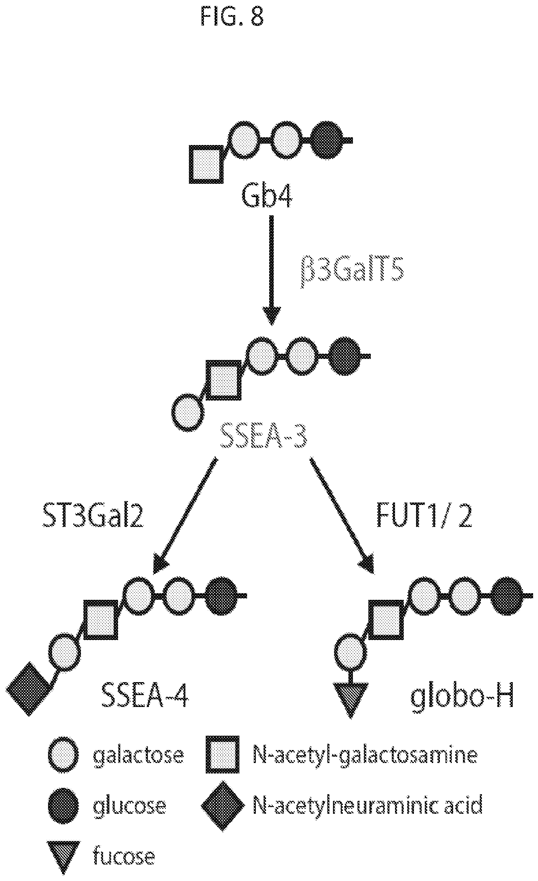

**Figure 8** **(Fig. S3)- the globo-series pathway in human.** The biosynthetic pathway of globo-series epitopes SSEA-3, SSEA-4 and globo-H from Gb4 with corresponding glycotransferases.

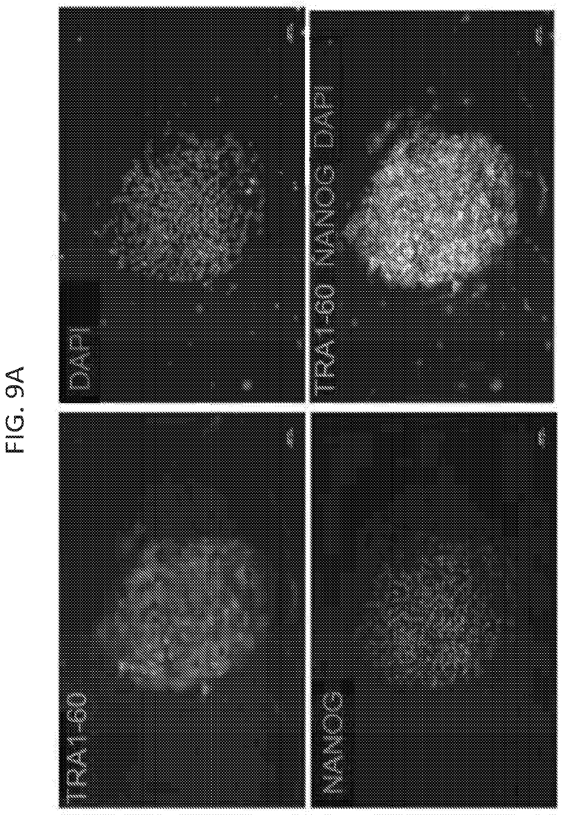

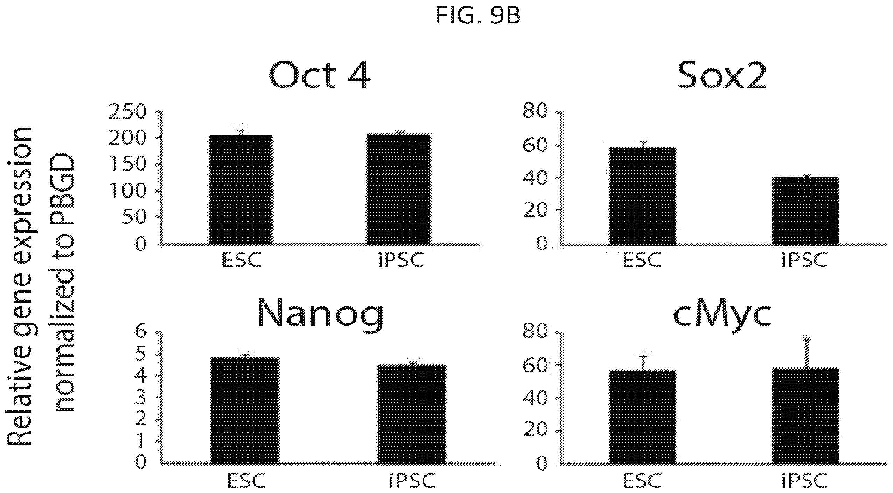

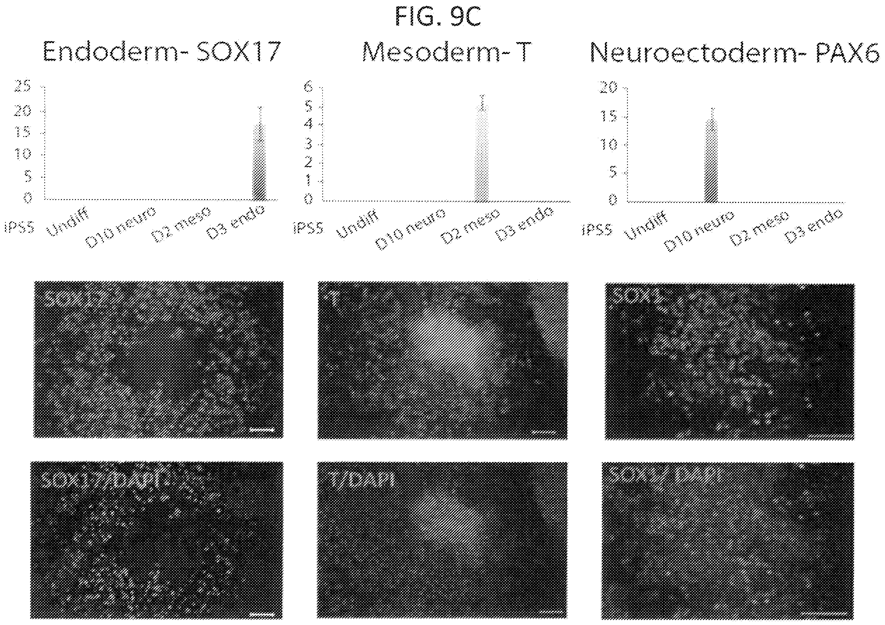

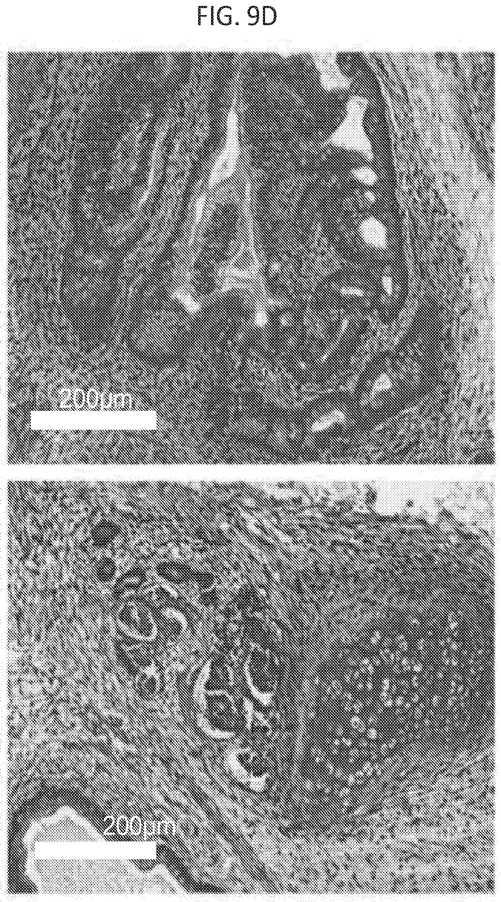

**Figure 9** **(Fig. S4)- the characterization of iPSC5. A** Immunofluorescent staining of stem cell proteins TRA1-60 and Nanog. Nuclei were stained with DAPI. **B** qPCR of stem genes OCT4, SOX2, NANOG and c-Myc. **C** *in vitro* differentiation capability of iPSC5 to three germ layer lineages. **D** H&E staining of iPSC5 to three germ layer lineages in teratoma.

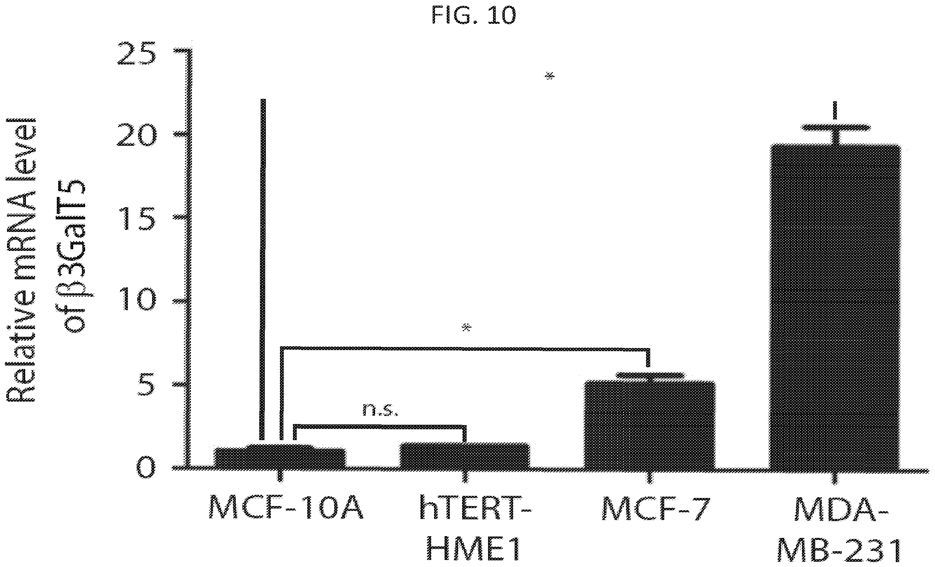

**Figure 10** **(Fig S5)- the mRNA level of beta3GalT5 in breast cancer cell culture is higher than that of normal cell culture.** GAPDH-normalized qPCR level of beta3GalT5 gene in normal cell culture MCF-10A and hTERT-HME1, as well as in breast cancer cell culture MCF-7 and MDA-MB-231. Triplicated samples from one representative experiment are shown. Data represent the mean and standard deviation (S.D.). Asterisks indicate statistical significance, *p*< 0.05; n.s. not significant.

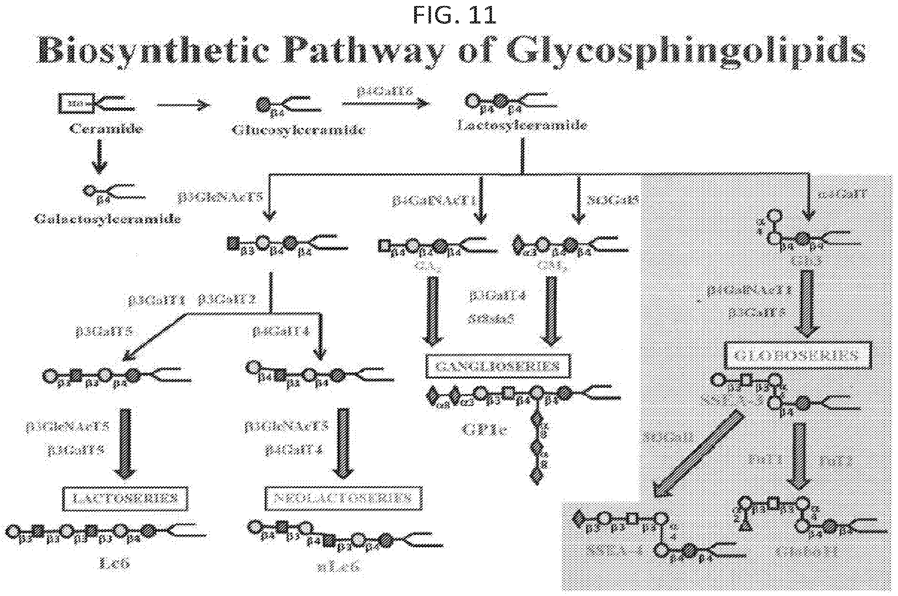

**Figure 11****.** Biosynthetic Pathway of Globo Series of Glycosphingolipids.

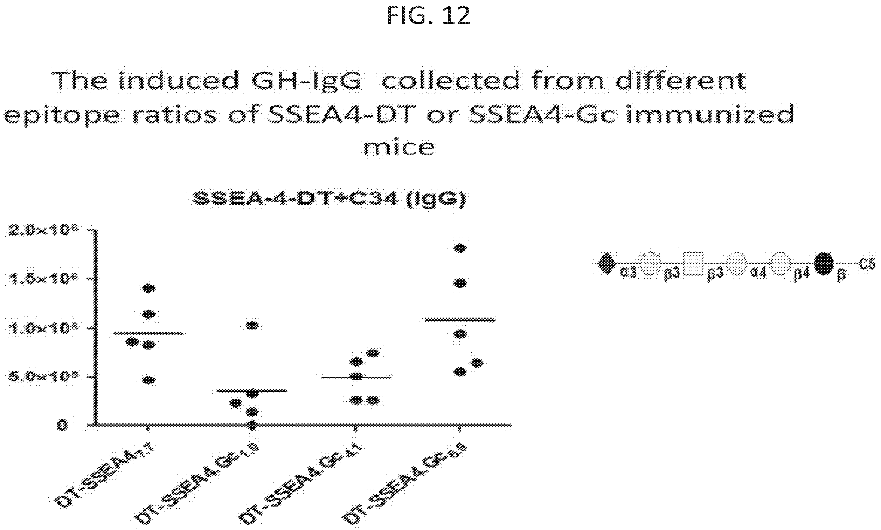

**Figure 12****.** The induced GH-IgG collected from different epitope ratios of SSE4-DT or SSEA4-Gc-DT immunization

### DETAILED DESCRIPTIONS

The present invention is based on the finding that tumor-associated carbohydrate antigens are suitable cancer stem cell markers.

The present disclosure is based on the surprising discovery that tumor-associated carbohydrate antigens are expressed on cancer stem cells. Thus, these tumor-associated carbohydrate antigens are suitable markers for cancer stem cells and furthermore, provide suitable therapeutic targets for a therapy that attacks cancer stem cells.

Both normal stem cells and tumorigenic cells have extensive proliferative potential and the ability to give rise to new (normal or abnormal) tissues. Tumorigenic cells can be thought of as cancer stem cells (CSC) or cancer initiating cells (CIC - the terms CSC and CIC are used as synonyms herein) that undergo an aberrant and poorly regulated process of organogenesis analogous to what normal stem cells do. Both tumors and normal tissues are composed of heterogeneous combinations of cells, with different phenotypic characteristics and different proliferative potentials.

Cancer stem cells are believed to be a certain fraction of tumor cells with stem cell-like properties, which initiate and maintain neoplastic clones. These cells have the ability to self-renew, but also give rise to progenitors that yield phenotypically diverse cancer cells but with lower tumorigenic potential. This subpopulation of stem cell-like cells are the ones that are efficient at tumor formation and metastatic tumor spread as compared to tumor cells that are not cancer stem cells.

Cancer stem cells (CSCs) have now been identified in a wide variety of cancers including leukemias, glioblastomas, medulloblastomas, and almost all types of epithelial tumors (carcinomas). Cancer stem cells can be characterized based on the investigation of distinct surface marker patterns within primary tumors. CD44 was reported as a robust marker of cancer stem cells. A single CD44+ cell from a colorectal tumor could form a sphere in vitro and was able to generate a xenograft tumor resembling the properties of the primary tumor. CD133 is also a marker of cancer stem cells.

The presence of cancer stem cells has profound implications for cancer therapy. Existing therapies have been developed largely against the bulk population of tumor cells, because the therapies are identified by their ability to shrink the tumor mass. However, cancer stem cells are often resistant to chemotherapy and can account for chemotherapy failure. To design novel therapeutic agents that (also) target cancer-initiating cells (also referred herein as cancer stem cells), it will be desirable to seek molecular targets of cancer stem cells that are preferably absent on benign tumors and/or normal non-tumor cells, and at the same time are specifically directed against cancer stem cells. Such agents are anticipated to result in more durable responses and cures of tumors, and especially of metastatic tumors. Therefore, new cancer stem cell markers are wanted to provide novel therapeutic targets to improve therapy. Most of the known stem-cell markers are proteins. Many of them have also been found to be normal stem cell markers and are thus expressed on non-tumor stem cells. This makes them not or at least less suitable as therapeutic target. At present, there is no clear cut distinction between normal and cancer stem cell markers.

A "binding agent" may be any compound or complex of compounds which is capable of binding a target substance such as a tumor-associated carbohydrate antigen and/or combination of carbohydrate and non-carbohydrate specific antigens, individually or in a combination (such as a panel). Preferably, the binding agent is capable of specifically binding the target substance. Suitable binding agents may be obtained by screening a binding agent library in order to identify/obtain binding agents that bind to the target substance. Examples for respective binding agent includes glycoantibodies. Such as antibodies against SSEA3, SSEA4, and/or globoH. The binding agents may have any structure, as long as they are able to specifically recognize and bind the target substance, here a tumor-associated carbohydrate antigen. Binding agents may be selected from the group consisting of antibodies, antigen-binding fragments or derivatives thereof or binding agents having a protein scaffold providing a binding function such as for example anticalins or lectins. Binding agents may also be peptides or fusion proteins providing a binding function. An overview of binding agents which have a similar binding function as antibodies is given in Hey, et al. (Hey et al. (2005) "Artificial, non-antibody binding proteins for pharmaceutical and industrial application", Trends in Biotechnology 23(10), 514-522). An antibody derivative also includes antibodies or antibody fragments having the same binding function but e.g. an altered amino acid sequence.

As used herein, exemplary binding agents targeting the SSEA3 marker is reported in USSN 14/599,174.

According to the invention, "staging" of a cancer preferably refers to the classification of the progression and extent of a cancer. A preferred cancer staging system is the TNM classification of malignant tumors, wherein T describes the size of the tumor and whether it has invaded nearby tissue, N describes regional lymph nodes that are involved, and M describes distant metastasis. Each of these parameters is given a particular value depending on the situation in the patient, wherein generally a higher number indicates a more severe situation (T(0-4), N(0-3), M(0/1)). Additionally, for a more detailed classification further parameters can be determined and/or prefixes can be used. Furthermore, the TNM classification may be summarized in a cancer staging system according to the UICC, referring to cancer of from stage 0 to stage IV.

According to the invention, a "sample" in particular refers to but is not limited to a tissue sample, a body fluid and/or a cellular sample and may be obtained by conventional manners such as by tissue biopsy, including punch biopsy or by taking blood, bronchial aspirate, sputum, urine, feces or other body fluids or tissue sections, slides, etc. containing or suspected of containing cancer cells. According to the invention, the term "sample" also includes fractions or components of respective samples.

The terms "cell proliferation" and "to proliferate" as used herein in particular refer to the amplification of the cell by cell division. The term "cancer stem cells" in particular relates but is not limited to cells capable of generating aggregates of undifferentiated cells, so called tumor spheres, under suitable conditions in vitro. The cells that form spheres are capable of self-renewal; when they are dissociated and grown under the same conditions, they will form spheres again. In vivo, cancer stem cells are characterized by their potential to form metastases and the expression of stem cell markers such as, e.g., CD44. They may also provide drug resistance. The terms "cancer stem cells" and "cancer initiating cells" are used as synonyms herein.

The term "tumor-associated carbohydrate antigen" in particular refers to a carbohydrate antigen that is expressed on cancer and/or tumor cells, in particular on malignant cancer and/or malignant tumor cells.

The term "tumor-specific carbohydrate antigen" in particular refers to a carbohydrate antigen that is predominantly or even exclusively expressed on cancer and/or tumor cells and thus not or only to a low extent on non-cancer respectively non-tumor cells. Preferably, the term "tumor-specific carbohydrate antigen" refers to a carbohydrate antigen that is predominantly or preferably exclusively expressed on malignant cancer and/or malignant tumor cells and thus not or only to a low extent on non-cancer respectively non-tumor cells, on benign cancer and/or benign tumor cells and/or on healthy tissue of the same patient. Preferentially, the tumor-specific carbohydrate antigen is not expressed on most normal cells, even more preferred it is expressed only on few normal cells or cell types, even more preferred the expression on these normal cells has a special localization, e.g. strictly apical or in between the tight junctions, so that a binding molecule administered systemically, and especially i.v., can not or barely reach the antigen on these normal cells, even more preferred it is not expressed on normal epithelial cells, most preferred it is not expressed on normal cells. In certain aspects, the tumor-specific carbohydrate antigen may be attached to a carrier molecule when expressed. Such carrier molecule may in particular be a protein, peptide or carbohydrate.

"CD44" is an adherence molecule (H-CAM, Pgp-1) of varying molecular weight. It is a cell surface hyaluronan receptor, interacts with matrix metalloproteinases, and plays a key role in cell migration. CD44 has been described as a cancer stem cell marker in breast, ovarian, pancreatic, prostate, colon, gastric, and other cancer types (see Li et al, 2007, and Takaishi et al, 2009). It is also a marker of normal pluripotent stem cells.

The present inventors have shown that several tumor-associated antigens such as CD24, CD44, SSEA3, PROCR, ESA are expressed on cancer stem cells and thus, are novel cancer stem cell markers. The identification of tumor-associated carbohydrate antigens as cancer stem cell markers provides novel therapeutic applications for agents specifically binding to one or more of said tumor-associated carbohydrate antigens. Agents specifically binding to one or more respective tumor-associated carbohydrate antigen can now be therapeutically used for targeting cancer stem cells that express said tumor- associated carbohydrate antigen. This provides the opportunity of therapeutic treatments that target and preferably kill cancer stem cells. The respective therapeutic agents can for example be used to target and thus destroy cancer stem cells which are resistant to regular chemotherapy. Thereby, improved cancer therapies are provided with the present disclosure. According to one aspect, the tumor-associated carbohydrate antigen is expressed predominantly or even exclusively on breast cancer stem cells. According to another aspect, the tumor-associated carbohydrate antigen is expressed on the cancer stem cells as well as on cancer cells that are not cancer stem cells. If the tumor-associated carbohydrate antigen is expressed on both cell populations, this has the advantage that treatment with the binding agent specifically binding the tumor-associated carbohydrate antigen targets both cell populations.

According to one aspect, the binding agent specifically binding to the one or more tumor-associated carbohydrate antigens is therapeutically active. One example of a respective aspect is the use of a therapeutically active antibody or antigen-binding fragment or derivative thereof as binding agent. A therapeutically active antibody or antigen-binding fragment or derivative thereof preferably is capable of inducing complement-dependent cytotoxicity (CDC) and/or antibody-dependent cellular cytotoxicity (ADCC), which preferably results in lysis of the target cell, in particular the cancer stem cell expressing the tumor-associated carbohydrate antigen. According to a further aspect, the binding agent specifically binding the tumor-associated carbohydrate antigen functions as a targeting molecule and is coupled to at least one therapeutic agent. Coupling can be achieved by covalent or non-covalent means. When the binding agent specifically binding the tumor-associated carbohydrate antigen functions as a targeting molecule, it can be itself therapeutically active or it may not be therapeutically active. In case it is not therapeutically active, it basically functions as a molecular carrier which brings the actual therapeutic agent (e.g. a radiopharmaceutical, chemotherapeutic agent or a toxin) to the desired target side of action, namely the cancer stem cells expressing the tumor-associated carbohydrate antigen. The therapeutic agent coupled to the binding agent specifically binding the tumor-associated carbohydrate antigen can be for example a chemotherapeutic agent or other anti-cancer drug. Said coupled therapeutic agent preferably destroys or kills the targeted cancer stem cells or inhibits proliferation thereof. This can either be achieved directly by the coupled therapeutic agent or indirectly via induction of suitable biological mechanisms of the targeted cancer stem cells and/or of the subject to be treated.

In certain aspects, the tumor-associated carbohydrate antigen as described herein can be further combined with any one or more of the markers selected from the group consisting of CD24, CD44, PROCR, ESA, CD176, CD175, CD175s, CD174, CD173 and CA19-9.

According to a further aspect of the present disclosure, a method is provided for identifying a cancer comprising cancer stem cells that is susceptible to treatment with a binding agent that specifically binds a tumor-associated carbohydrate antigen wherein said treatment effects the cancer stem cells, comprising determining whether a cancer sample obtained from a patient comprises cancer stem cells that express the tumor-associated carbohydrate antigen the binding agent is specific for, wherein the presence of said tumor-associated carbohydrate antigen on cancer stem cells indicates that the cancer is susceptible to treatment with the binding agent that specifically binds a tumor- associated carbohydrate antigen and wherein said treatment also effects the cancer stem cells.

This method according to the present disclosure allows to test whether the cancer stem cells of a cancer are susceptible to treatment with a binding agent that specifically binds a tumor-associated carbohydrate antigen. The results of the method provide valuable diagnostic information to the physician. E.g. in case the cancer would comprise cancer stem cells which do not express said tumor-associated carbohydrate antigen, treatment with a binding agent specifically binding said tumor-associated carbohydrate antigen would not affect the cancer stem cells and accordingly, would be useless against the cancer stem cells. However, in case it is shown by said method that the cancer stem cells express said tumor-associated carbohydrate antigen the binding agent is specific for, chances are good that treatment with said binding agent will also target and accordingly affect the cancer stem cells. Thus, the method according to the present disclosure provides valuable aid to the physician for choosing the best therapy for the patient and to estimate whether a certain treatment will affect the cancer stem cells of a cancer.

According to a related diagnostic aspect, a method is provided for diagnosing, staging and/or prognosing cancer and/or monitoring the susceptibility to treatment, comprising the step of analyzing the expression of a tumor-associated carbohydrate antigen on cells in a sample isolated from a patient, wherein the presence of cells expressing the tumor-associated carbohydrate antigen indicates the presence of cancer stem cells in said sample.

According to a further aspect of the present disclosure, a method is provided for identifying a population of cancer stem cells, the method comprising: a) providing a starting population of cancer cells, b) determining the expression level of one or more tumor-associated carbohydrate antigens, c) selecting a population of cells whose expression level of said one or more tumor-associated carbohydrate antigens as determined in step b) is increased when compared to control cells, wherein said selected population of cells are cancer stem cells and d) optionally isolating and/or enriching for said population of cells selected in step c), wherein said control cells are cells from the same starting cancer cell population which do not express, or express lower levels of said one or more tumor-associated carbohydrate antigens. In some aspects, the tumor-associated carbohydrate antigens are selected from the list consisting of SSEA3, SSEA4, and GloboH antigens. In some aspects, the determining step in b) further includes determining the expression level of one or more additional tumor associated antigens, and the selection and isolating steps in c) and d), respectively, also includes consideration of the expression level of said one or more additional tumor associated antigens. In some aspects, the one or more tumor-associated antigens include, but are not limited by CD24, CD44, PROCR, ESA, CD176, CD175, CD175s, CD174, CD173 and CA19-9 antigens. In some aspects, the determining step in b) and/or the isolation step in d) is carried out using FACS. In some aspects, the level of expression of said one or more tumor-associated carbohydrate antigens is high or highly increased, when compared to control cells. In some aspects, the level of expression of said one or more tumor-associated carbohydrate antigens is low or lowly increased, when compared to control cells.

The presence of cancer stem cells in a patient sample can be indicative of the stage of a cancer. In addition, detection of cancer stem cells can be used to monitor response to therapy and to aid in prognosis. The information obtained by the methods disclosed herein is useful in prognosis and diagnosis, including analysing the susceptibility to acceleration of the disease, the analysis by active monitoring of the disease wherein it is analysed whether the cancer progresses and e.g. needs treatment, the status of a disease state, the response to changes in the environment, such as the passage of time, the treatment with a chosen therapeutic agent, in particular a binding agent as described above, or other modalities. By analysing whether cells contained in the sample express a tumor-associated carbohydrate antigen and accordingly, the sample comprises cancer stem cells, the cells can also be classified as to their ability to respond to therapeutic agents and treatments. Furthermore, the information derived is useful in determining and/or predicting the metastatic behavior of a cancer.

According to one aspect of the diagnostic methods disclosed
herein, the binding agents disclosed herein which specifically bind to one or more tumor-associated carbohydrate antigens expressed on cancer stem cells are used for *in vivo* diagnostic, in particular in vivo imaging. A respective method is also useful for diagnostic purposes. E.g. it can be determined, whether cancer cells expressing the tumor-associated carbohydrate antigen expressed on cancer stem cells can be identified and/or located in the patient. If this is the case, there is a risk that there are cancer stem cells. As is described above and below, it is preferred that a second stem cell marker is additionally detected to confirm and/or determine the nature of the cancer stem cells. Furthermore, the response to therapy can be monitored as it can be determined e.g. whether the tumor size descreases or whether metases develop. Furthermore, a respective method is advantageous to identify the suitable dosage for a patient. According to one embodiment, the binding agent is labelled, e.g. being a radiopharmaceutical comprising a radionuclide. However, the binding agent may also be coupled to other agents/compounds such as e.g. a PET tracer that allow *in vivo* imaging. Suitable compounds are known in the prior art and thus, do not need further description here. Details regarding the binding agents, the tumor-associated carbohydrate antigens, further cancer stem cell markers and cancer types are described above and below and also apply to the *in vivo* imaging aspect. It is referred to the respective disclosure.

According to one aspect, samples, containing or suspected of containing
cancer cells, are contacted and preferably stained with at least one agent specifically binding the tumor-associated, preferably tumor-specific carbohydrate antigen and thus the carbohydrate cancer stem cell marker and optionally at least one further agent specifically binding at least one second cancer stem cell marker, such as CD44. This allows to detect the presence of cancer stem cells in the sample. According to one aspect, binding of the binding agent against the tumor-associated carbohydrate antigen and preferably the binding of the binding agent against the second cancer stem cell marker is detected by appropriate detection methods as known in the art and as described herein. Suitable detection methods are for example ELISA, FACS, fluorescence microscopy and the like.

Samples to be analysed by the methods disclosed herein may be obtained from a variety of sources, particularly from a biopsy sample. Cells of such samples can be separated by centrifugation, elutriation, density gradient separation, apheresis, affinity selection,
panning, FACS, centrifugation with Hypaque, etc. prior to analysis. Once a sample is obtained, it can be used directly, frozen, or maintained in appropriate culture medium for short periods of time, or fixed in a suited fixation solution, or fixed and embedded in a medium suited for histologigal or immunohistological examination. Various media can be employed to maintain cells. The samples may be obtained by any convenient procedure, such as biopsy, or from surgical specimen. Usually a sample will comprise at least about 10² cells, more usually at least about 10³ cells, and preferable 10⁴, 10⁵ or more cells. In one aspect, the sample comprises 10 cells. In one aspect, the sample comprises any number of cells between 10 and 100 cells. In one aspect, the sample comprises any number of cells between 10 and 1000 cells. In one aspect, the sample comprises any number of cells between 1 and 10 cells. Typically the samples will be from human patients, although animal models may find use, e.g. equine, bovine, porcine, canine, feline, rodent, e.g. mice, rats, hamster, primate, etc.

The samples may be frozen, embedded, fixed, present in a tissue microarray, and the like. The agents used for binding, detecting and in particular staining the tumor-associated carbohydrate antigen and, optionally, a further cancer stem cell marker can be e.g. binding agents specifically binding the cancer stem cell markers such as e.g. antibodies. Suitable examples are described above. These agents may be detectably labelled, or may be indirectly labelled in the staining procedure. According to one aspect, the label can also be used for separating the tumor- associated carbohydrate antigen positive cells. Suitable labels as well as staining procedures are known in the prior art and accordingly, do not need further description here even though some examples are described herein. A standard procedure for analyis may include a histological fixation of the sample (e.g. by formalin) and subsequent staining as is e.g. described in the examples. The obtained data allows to determine the number and distribution of cancer stem cells in the sample.

Methods suitable for detecting and/or quantifying cells expressing the tumor-associated carbohydrate antigen include, for example, immunologic assays such ELISA, RIA, Western blot and immunohistochemistry, flow cytometry, immunohistochemistry or the like.

In screening assays for candidate therapeutic agents usually a culture comprising cancer stem cells expressing the tumor-associated carbohydrate antigen of interest is contacted with the binding agent of interest, and the effect of the agent assessed by monitoring output parameters, such as expression of markers, cell viability, and the like. The screening may also involve determining modulation of growth, proliferation, viability, and/or differentiation status of the cell in the presence of the candidate therapeutic agent as compared to the growth, proliferation, viability, and/or differentiation status of the cell in the absence of the candidate therapeutic agent.

Isolated cell populations that are highly enriched for cancer stem cells expressing one or more tumor-associated carbohydrate antigens can be isolated and enriched/purified using these markers. In some aspects, said cancer stem cell populations are isolated from the circulation, such as from the blood. In some aspects, said cancer stem cell populations are isolated from the a tumor sample, or from pleural effusions or other fluids obtained from a cancer patient.

### Chemical definitions

Definitions of specific functional groups and chemical terms are described in more detail below. The chemical elements are identified in accordance with the Periodic Table of the Elements, CAS version, Handbook of Chemistry and Physics, 75th Ed., inside cover, and specific functional groups are generally defined as described therein. Additionally, general principles of organic chemistry, as well as specific functional moieties and reactivity, are described in Thomas Sorrell, Organic Chemistry, University Science Books, Sausalito, 1999; Smith and March, March's Advanced Organic Chemistry, 5th Edition, John Wiley & Sons, Inc., New York, 2001; Larock, Comprehensive Organic Transformations, VCH Publishers, Inc., New York, 1989; and Carruthers, Some Modern Methods of Organic Synthesis, 3rd Edition, Cambridge University Press, Cambridge, 1987. Moreover, exemplary glycan and antibody methodologies are described in Wong et al, US20100136042, US20090317837, and US20140051127.

Compounds described herein can comprise one or more asymmetric centers, and thus can exist in various isomeric forms, *e.g*., enantiomers and/or diastereomers. For example, the compounds described herein can be in the form of an individual enantiomer, diastereomer or geometric isomer, or can be in the form of a mixture of stereoisomers, including racemic mixtures and mixtures enriched in one or more stereoisomer. Isomers can be isolated from mixtures by methods known to those skilled in the art, including chiral high pressure liquid chromatography (HPLC) and the formation and crystallization of chiral salts; or preferred isomers can be prepared by asymmetric syntheses. See, for example, Jacques et al., Enantiomers, Racemates and Resolutions (Wiley Interscience, New York, 1981); Wilen et al., Tetrahedron 33:2725 (1977); Eliel, Stereochemistry of Carbon Compounds (McGraw-Hill, NY, 1962); and Wilen, Tables of Resolving Agents and Optical Resolutions p. 268 (E.L. Eliel, Ed., Univ. of Notre Dame Press, Notre Dame, IN 1972). The disclosure additionally encompasses compounds described herein as individual isomers substantially free of other isomers, and alternatively, as mixtures of various isomers.

When a range of values is listed, it is intended to encompass each value and sub-range within the range. For example "C₁₋₆" is intended to encompass C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆.

"Alkyl" refers to a radical of a straight-chain or branched saturated hydrocarbon group having from 1 to 20 carbon atoms ("C₁₋₂₀ alkyl"). In some aspects, an alkyl group has 1 to 10 carbon atoms ("C₁₋₁₀ alkyl"). In some aspects, an alkyl group has 1 to 9 carbon atoms ("C₁₋₉ alkyl"). In some aspects, an alkyl group has 1 to 8 carbon atoms ("C₁₋₈ alkyl"). In some aspects, an alkyl group has 1 to 7 carbon atoms ("C₁₋₇ alkyl"). In some aspects, an alkyl group has 1 to 6 carbon atoms ("C₁₋₆ alkyl"). In some aspects, an alkyl group has 1 to 5 carbon atoms ("C₁₋₅ alkyl"). In some aspects, an alkyl group has 1 to 4 carbon atoms ("C₁₋₄ alkyl"). In some aspects, an alkyl group has 1 to 3 carbon atoms ("C₁₋₃ alkyl"). In some aspects, an alkyl group has 1 to 2 carbon atoms ("C₁₋₂ alkyl"). In some aspects, an alkyl group has 1 carbon atom ("C₁ alkyl"). In some aspects, an alkyl group has 2 to 6 carbon atoms ("C₂₋₆ alkyl"). Examples of C₁₋₆ alkyl groups include methyl (C₁), ethyl (C₂), *n*-propyl (C₃), *iso*-propyl (C₃), *n*-butyl (C₄), *tert*-butyl (C₄), *sec*-butyl (C₄), *iso*-butyl (C₄), *n*-pentyl (C₅), 3-pentanyl (C₅), amyl (C₅), neopentyl (C₅), 3-methyl-2-butanyl (C₅), tertiary amyl (C₅), and *n*-hexyl (C₆). Additional examples of alkyl groups include *n*-heptyl (C₇), *n*-octyl (C₈) and the like. Unless otherwise specified, each instance of an alkyl group is independently optionally substituted, *i.e*., unsubstituted (an "unsubstituted alkyl") or substituted (a "substituted alkyl") with one or more substituents. In certain aspects, the alkyl group is unsubstituted C₁₋₁₀ alkyl (*e.g.,* - CH₃). In certain aspects, the alkyl group is substituted C₁₋₁₀ alkyl.

"Alkenyl" refers to a radical of a straight-chain or branched hydrocarbon group having from 2 to 20 carbon atoms, one or more carbon-carbon double bonds, and no triple bonds ("C₂₋₂₀ alkenyl"). In some aspects, an alkenyl group has 2 to 10 carbon atoms ("C₂₋₁₀ alkenyl"). In some aspects, an alkenyl group has 2 to 9 carbon atoms ("C₂₋₉ alkenyl"). In some aspects, an alkenyl group has 2 to 8 carbon atoms ("C₂₋₈ alkenyl"). In some aspects, an alkenyl group has 2 to 7 carbon atoms ("C₂₋₇ alkenyl"). In some aspects, an alkenyl group has 2 to 6 carbon atoms ("C₂₋₆ alkenyl"). In some aspects, an alkenyl group has 2 to 5 carbon atoms ("C₂₋₅ alkenyl"). In some aspects, an alkenyl group has 2 to 4 carbon atoms ("C₂₋₄ alkenyl"). In some aspects, an alkenyl group has 2 to 3 carbon atoms ("C₂₋₃ alkenyl"). In some aspects, an alkenyl group has 2 carbon atoms ("C₂ alkenyl"). The one or more carbon- carbon double bonds can be internal (such as in 2-butenyl) or terminal (such as in 1-butenyl). Examples of C₂₋₄ alkenyl groups include ethenyl (C₂), 1-propenyl (C₃), 2-propenyl (C₃), 1-butenyl (C₄), 2-butenyl (C₄), butadienyl (C₄), and the like. Examples of C₂₋₆ alkenyl groups include the aforementioned C₂₋₄ alkenyl groups as well as pentenyl (C₅), pentadienyl (C₅), hexenyl (C₆), and the like. Additional examples of alkenyl include heptenyl (C₇), octenyl (C₈), octatrienyl (C₈), and the like. Unless otherwise specified, each instance of an alkenyl group is independently optionally substituted, *i.e*., unsubstituted (an "unsubstituted alkenyl") or substituted (a "substituted alkenyl") with one or more substituents. In certain aspects, the alkenyl group is unsubstituted C₂₋₁₀ alkenyl. In certain aspects, the alkenyl group is substituted C₂₋₁₀ alkenyl.

"Alkynyl" refers to a radical of a straight-chain or branched hydrocarbon group having from 2 to 20 carbon atoms, one or more carbon-carbon triple bonds, and optionally one or more double bonds ("C₂₋₂₀ alkynyl"). In some aspects, an alkynyl group has 2 to 10 carbon atoms ("C₂₋₁₀ alkynyl"). In some aspects, an alkynyl group has 2 to 9 carbon atoms ("C₂₋₉ alkynyl"). In some aspects, an alkynyl group has 2 to 8 carbon atoms ("C₂₋₈ alkynyl"). In some aspects, an alkynyl group has 2 to 7 carbon atoms ("C₂₋₇ alkynyl"). In some aspects, an alkynyl group has 2 to 6 carbon atoms ("C₂₋₆ alkynyl"). In some aspects, an alkynyl group has 2 to 5 carbon atoms ("C₂₋₅ alkynyl"). In some aspects, an alkynyl group has 2 to 4 carbon atoms ("C₂₋₄ alkynyl"). In some aspects, an alkynyl group has 2 to 3 carbon atoms ("C₂₋₃ alkynyl"). In some aspects, an alkynyl group has 2 carbon atoms ("C₂ alkynyl"). The one or more carbon- carbon triple bonds can be internal (such as in 2-butynyl) or terminal (such as in 1-butynyl). Examples of C₂₋₄ alkynyl groups include, without limitation, ethynyl (C₂), 1-propynyl (C₃), 2-propynyl (C₃), 1-butynyl (C₄), 2-butynyl (C₄), and the like. Examples of C₂₋₆ alkenyl groups include the aforementioned C₂₋₄ alkynyl groups as well as pentynyl (C₅), hexynyl (C₆), and the like. Additional examples of alkynyl include heptynyl (C₇), octynyl (C₈), and the like. Unless otherwise specified, each instance of an alkynyl group is independently optionally substituted, *i.e*., unsubstituted (an "unsubstituted alkynyl") or substituted (a "substituted alkynyl") with one or more substituents. In certain aspects, the alkynyl group is unsubstituted C₂₋₁₀ alkynyl. In certain aspects, the alkynyl group is substituted C₂₋₁₀ alkynyl.

"Heterocyclyl" or "heterocyclic" refers to a radical of a 3- to 10-membered nonaromatic ring system having ring carbon atoms and 1 to 4 ring heteroatoms, wherein each heteroatom is independently selected from nitrogen, oxygen, sulfur, boron, phosphorus, and silicon ("3-10 membered heterocyclyl"). In certain aspects, the heteroatom is independently selected from nitrogen, sulfur, and oxygen. In heterocyclyl groups that contain one or more nitrogen atoms, the point of attachment can be a carbon or nitrogen atom, as valency permits. A heterocyclyl group can either be monocyclic ("monocyclic heterocyclyl") or a fused, bridged or spiro ring system such as a bicyclic system ("bicyclic heterocyclyl"), and can be saturated or partially unsaturated. Heterocyclyl bicyclic ring systems can include one or more heteroatoms in one or both rings. "Heterocyclyl" also includes ring systems wherein the heterocyclic ring, as defined above, is fused with one or more carbocyclyl groups wherein the point of attachment is either on the carbocyclyl or heterocyclic ring, or ring systems wherein the heterocyclic ring, as defined above, is fused with one or more aryl or heteroaryl groups, wherein the point of attachment is on the heterocyclic ring, and in such instances, the number of ring members continue to designate the number of ring members in the heterocyclic ring system. Unless otherwise specified, each instance of heterocyclyl is independently optionally substituted, *i.e*., unsubstituted (an "unsubstituted heterocyclyl") or substituted (a "substituted heterocyclyl") with one or more substituents. In certain embodiments, the heterocyclyl group is unsubstituted 3-10 membered heterocyclyl. In certain embodiments, the heterocyclyl group is substituted 3-10 membered heterocyclyl.

"Aryl" refers to a radical of a monocyclic or polycyclic (*e.g.*, bicyclic or tricyclic) 4n+2 aromatic ring system (*e.g*., having 6, 10, or 14 π electrons shared in a cyclic array) having 6-14 ring carbon atoms and zero heteroatoms in the aromatic ring system ("C₆₋₁₄ aryl"). In some aspects, an aryl group has six ring carbon atoms ("C₆ aryl"; *e.g.,* phenyl). In some aspects, an aryl group has ten ring carbon atoms ("C₁₀ aryl"; *e.g*., naphthyl such as 1-naphthyl and 2-naphthyl). In some aspects, an aryl group has fourteen ring carbon atoms ("C₁₄ aryl"; *e.g.*, anthracyl). "Aryl" also includes ring systems wherein the aryl ring, as defined above, is fused with one or more carbocyclyl or heterocyclyl groups wherein the radical or point of attachment is on the aryl ring, and in such instances, the number of carbon atoms continue to designate the number of carbon atoms in the aryl ring system. Unless otherwise specified, each instance of an aryl group is independently optionally substituted, *i.e*., unsubstituted (an "unsubstituted aryl") or substituted (a "substituted aryl") with one or more substituents. In certain aspects, the aryl group is unsubstituted C₆₋₁₄ aryl. In certain aspects, the aryl group is substituted C₆₋₁₄ aryl.

Alkyl, alkenyl, alkynyl, carbocyclyl, heterocyclyl, aryl, and heteroaryl groups, as defined herein, which are divalent bridging groups are further referred to using the suffix - ene, *e.g*., alkylene, alkenylene, alkynylene, carbocyclylene, heterocyclylene, arylene, and heteroarylene.

The term "alkoxy" or "alkyloxy" refers to an -O-alkyl radical, wherein alkyl is optionally substituted alkyl as defined herein. Examples of alkoxy include, but are not limited to, methoxy, ethoxy, n-propoxy, isopropoxy, n-butoxy, iso-butoxy, sec-butoxy, and tert-butoxy.

The term "aryloxy" refers to an -O-aryl, wherein aryl is optionally substituted aryl as defined herein.

As used herein, the term "optionally substituted" refers to a substituted or unsubstituted moiety.

Alkyl, alkenyl, alkynyl, carbocyclyl, heterocyclyl, aryl, and heteroaryl groups, as defined herein, are optionally substituted (*e.g*., "substituted" or "unsubstituted" alkyl, "substituted" or "unsubstituted" alkenyl, "substituted" or "unsubstituted" alkynyl, "substituted" or "unsubstituted" carbocyclyl, "substituted" or "unsubstituted" heterocyclyl, "substituted" or "unsubstituted" aryl or "substituted" or "unsubstituted" heteroaryl group). In general, the term "substituted", whether preceded by the term "optionally" or not, means that at least one hydrogen present on a group (*e.g*., a carbon or nitrogen atom) is replaced with a permissible substituent, *e.g*., a substituent which upon substitution results in a stable compound, *e.g*., a compound which does not spontaneously undergo transformation such as by rearrangement, cyclization, elimination, or other reaction. Unless otherwise indicated, a "substituted" group has a substituent at one or more substitutable positions of the group, and when more than one position in any given structure is substituted, the substituent is either the same or different at each position. The term "substituted" is contemplated to include substitution with all permissible substituents of organic compounds, any of the substituents described herein that results in the formation of a stable compound. The present disclosure contemplates any and all such combinations in order to arrive at a stable compound. For purposes of this disclosure, heteroatoms such as nitrogen may have hydrogen substituents and/or any suitable substituent as described herein which satisfy the valencies of the heteroatoms and results in the formation of a stable moiety.

"Halo" or "halogen" refers to fluorine (fluoro, -F), chlorine (chloro, -Cl), bromine (bromo, -Br), or iodine (iodo, -I).

"Acyl" as used herein refers to a moiety selected from the group consisting of - C(=O)R^{aa},-CHO, -CO₂R^{aa}, -C(=O)N(R^{bb})_{2,} -C(=NR^{bb})R^{aa}, -C(=NR^{bb})OR^{aa}, - C(=NR^{bb})N(R^{bb})₂, -C(=O)NR^{bb}SO₂R^{aa}, -C(=S)N(R^{bb})₂, -C(=O)SR^{aa}, and -C(=S)SR^{aa}, wherein R^{aa} and R^{bb} are as defined herein.

Nitrogen atoms can be substituted or unsubstituted as valency permits, and include primary, secondary, tertiary, and quarternary nitrogen atoms. Exemplary nitrogen atom substituents include, but are not limited to, hydrogen, -OH, -OR^{aa}, -N(R^{cc})₂, -CN, - C(=O)R^{aa}, -C(=O)N(R^{cc})₂, -CO₂R^{aa}, -SO₂R^{aa}, -C(=NR^{bb})R^{aa}, -C(=NR^{cc})OR^{aa}, - C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, - C(=S)SR^{cc}, -P(=O)₂R^{aa}, -P(=O)(R^{aa})₂, -P(=O)₂N(R^{cc})₂, -P(=O)(NR^{cc})₂, C₁₋₁₀ alkyl, C₁₋₁₀ perhaloalkyl, C₂₋₁₀ alkenyl, C₂₋₁₀ alkynyl, C₃₋₁₀ carbocyclyl, 3-14 membered heterocyclyl, C₆₋₁₄ aryl, and 5-14 membered heteroaryl, or two R^{cc} groups attached to a nitrogen atom are joined to form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 R^{dd} groups, and wherein R^{aa}, R^{bb}, R^{cc}, and R^{dd} are as defined above.

In certain aspects, the substituent present on an oxygen atom is an oxygen protecting group (also referred to as a hydroxyl protecting group). Oxygen protecting groups include, but are not limited to, -R^{aa}, -N(R^{bb})₂, -C(=O)SR^{aa}, -C(=O)R^{aa}, -CO₂R^{aa}, - C(=O)N(R^{bb})₂, -C(=NR^{bb})R^{aa}, -C(=NR^{bb})OR^{aa}, -C(=NR^{bb})N(R^{bb})₂, -S(=O)R^{aa}, -SO₂R^{aa}, - Si(R^{aa})₃, -P(R^{cc})₂, -P(R^{cc})₃, -P(=O)₂R^{aa}, -P(=O)(R^{aa})₂, -P(=O)(OR^{cc})₂, -P(=O)₂N(R^{bb})₂, and - P(=O)(NR^{bb})₂, wherein R^{aa}, R^{bb}, and R^{cc} are as defined herein. Oxygen protecting groups are well known in the art and include those described in Protecting Groups in Organic Synthesis, T. W. Greene and P. G. M. Wuts, 3rd edition, John Wiley & Sons, 1999.

Exemplary oxygen protecting groups include, but are not limited to, methyl, methoxylmethyl (MOM), methylthiomethyl (MTM), *t*-butylthiomethyl, (phenyldimethylsilyl)methoxymethyl (SMOM), benzyloxymethyl (BOM), *p-*methoxybenzyloxymethyl (PMBM), (4-methoxyphenoxy)methyl (*p*-AOM), guaiacolmethyl (GUM), *t*-butoxymethyl, 4-pentenyloxymethyl (POM), siloxymethyl, 2-methoxyethoxymethyl (MEM), 2,2,2-trichloroethoxymethyl, bis(2-chloroethoxy)methyl, 2-(trimethylsilyl)ethoxymethyl (SEMOR), tetrahydropyranyl (THP), 3-bromotetrahydropyranyl, tetrahydrothiopyranyl, 1-methoxycyclohexyl, 4-methoxytetrahydropyranyl (MTHP), 4-methoxytetrahydrothiopyranyl, 4-methoxytetrahydrothiopyranyl S,S-dioxide, 1-[(2-chloro-4-methyl)phenyl]-4-methoxypiperidin-4-yl (CTMP), 1,4-dioxan-2-yl, tetrahydrofuranyl, tetrahydrothiofuranyl, 2,3,3a,4,5,6,7,7a-octahydro-7,8,8-trimethyl-4,7-methanobenzofuran-2-yl, 1-ethoxyethyl, 1-(2-chloroethoxy)ethyl, 1-methyl-1-methoxyethyl, 1-methyl-1-benzyloxyethyl, 1-methyl-1-benzyloxy-2-fluoroethyl, 2,2,2-trichloroethyl, 2-trimethylsilylethyl, 2-(phenylselenyl)ethyl, *t*-butyl, allyl, *p*-chlorophenyl, *p*-methoxyphenyl, 2,4-dinitrophenyl, benzyl (Bn), *p*-methoxybenzyl, 3,4-dimethoxybenzyl, *o*-nitrobenzyl, *p*-nitrobenzyl, *p-*halobenzyl, 2,6-dichlorobenzyl, *p*-cyanobenzyl, *p*-phenylbenzyl, 2-picolyl, 4-picolyl, 3-methyl-2-picolyl *N*-oxido, diphenylmethyl, *p,p*'-dinitrobenzhydryl, 5-dibenzosuberyl, triphenylmethyl, α-naphthyldiphenylmethyl, *p*-methoxyphenyldiphenylmethyl, di(*p-*methoxyphenyl)phenylmethyl, tri(*p*-methoxyphenyl)methyl, 4-(4'-bromophenacyloxyphenyl)diphenylmethyl, 4,4',4"-tris(4,5-dichlorophthalimidophenyl)methyl, 4,4',4"-tris(levulinoyloxyphenyl)methyl, 4,4',4"-tris(benzoyloxyphenyl)methyl, 3-(imidazol-1-yl)bis(4',4"-dimethoxyphenyl)methyl, 1,1-bis(4-methoxyphenyl)-1'-pyrenylmethyl, 9-anthryl, 9-(9-phenyl)xanthenyl, 9-(9-phenyl-10-oxo)anthryl, 1,3-benzodithiolan-2-yl, benzisothiazolyl S,S-dioxido, trimethylsilyl (TMS), triethylsilyl (TES), triisopropylsilyl (TIPS), dimethylisopropylsilyl (IPDMS), diethylisopropylsilyl (DEIPS), dimethylthexylsilyl, *t*-butyldimethylsilyl (TBDMS), *t-*butyldiphenylsilyl (TBDPS), tribenzylsilyl, tri-*p*-xylylsilyl, triphenylsilyl, diphenylmethylsilyl (DPMS), *t*-butylmethoxyphenylsilyl (TBMPS), formate, benzoylformate, acetate, chloroacetate, dichloroacetate, trichloroacetate, trifluoroacetate, methoxyacetate, triphenylmethoxyacetate, phenoxyacetate, *p*-chlorophenoxyacetate, 3-phenylpropionate, 4-oxopentanoate (levulinate), 4,4-(ethylenedithio)pentanoate (levulinoyldithioacetal), pivaloate, adamantoate, crotonate, 4-methoxycrotonate, benzoate, *p*-phenylbenzoate, 2,4,6-trimethylbenzoate (mesitoate), methyl carbonate, 9-fluorenylmethyl carbonate (Fmoc), ethyl carbonate, 2,2,2-trichloroethyl carbonate (Troc), 2-(trimethylsilyl)ethyl carbonate (TMSEC), 2-(phenylsulfonyl) ethyl carbonate (Psec), 2-(triphenylphosphonio) ethyl carbonate (Peoc), isobutyl carbonate, vinyl carbonate, allyl carbonate, *t*-butyl carbonate (BOC), *p*-nitrophenyl carbonate, benzyl carbonate, *p*-methoxybenzyl carbonate, 3,4-dimethoxybenzyl carbonate, *o*-nitrobenzyl carbonate, *p*-nitrobenzyl carbonate, *S*-benzyl thiocarbonate, 4-ethoxy-1-napththyl carbonate, methyl dithiocarbonate, 2-iodobenzoate, 4-azidobutyrate, 4-nitro-4-methylpentanoate, *o*-(dibromomethyl)benzoate, 2-formylbenzenesulfonate, 2-(methylthiomethoxy)ethyl, 4-(methylthiomethoxy)butyrate, 2-(methylthiomethoxymethyl)benzoate, 2,6-dichloro-4-methylphenoxyacetate, 2,6-dichloro-4-(1,1,3,3-tetramethylbutyl)phenoxyacetate, 2,4-bis(1,1-dimethylpropyl)phenoxyacetate, chlorodiphenylacetate, isobutyrate, monosuccinoate, (*E*)-2-methyl-2-butenoate, *o-*(methoxyacyl)benzoate, α-naphthoate, nitrate, alkyl *N,N,N',N'-*tetramethylphosphorodiamidate, alkyl *N*-phenylcarbamate, borate, dimethylphosphinothioyl, alkyl 2,4-dinitrophenylsulfenate, sulfate, methanesulfonate (mesylate), benzylsulfonate, and tosylate (Ts).

### Other definitions

It must be noted that as used herein and in the appended claims, the singular forms "a", "an", and "the" include plural reference unless the context clearly dictates otherwise. As well, the terms "a" (or "an"), "one or more" and "at least one" can be used interchangeably herein. It is also to be noted that the terms "comprising", "including", and "having" can be used interchangeably.

The practice of the present invention will employ, unless otherwise indicated, conventional techniques of molecular biology, microbiology, recombinant DNA, and immunology, which are within the skill of the art. Such techniques are explained fully in the literature. See, for example, Molecular Cloning A Laboratory Manual, 2nd Ed., ed. by Sambrook, Fritsch and Maniatis (Cold Spring Harbor Laboratory Press, 1989); DNA Cloning, Volumes I and II (D. N. Glover ed., 1985); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the treatise, Methods In Enzymology (Academic Press, Inc., N.Y.); Gene Transfer Vectors For Mammalian Cells (J. H. Miller and M. P. Calos eds., 1987, Cold Spring Harbor Laboratory); Methods In Enzymology, Vols. 154 and 155 (Wu et al. eds.), Immunochemical Methods In Cell And Molecular Biology (Mayer and Walker, eds., Academic Press, London, 1987); Antibodies: A Laboratory Manual, by Harlow and Lane s (Cold Spring Harbor Laboratory Press, 1988); and Handbook Of Experimental Immunology, Volumes I-IV (D. M. Weir and C. C. Blackwell, eds., 1986).

As used herein, the term "glycan" refers to a polysaccharide, or oligosaccharide. Glycan is also used herein to refer to the carbohydrate portion of a glycoconjugate, such as a glycoprotein, glycolipid, glycopeptide, glycoproteome, peptidoglycan, lipopolysaccharide or a proteoglycan. Glycans usually consist solely of O-glycosidic linkages between monosaccharides. For example, cellulose is a glycan (or more specifically a glucan) composed of β-1,4-linked D-glucose, and chitin is a glycan composed of β-1,4-linked N-acetyl-D-glucosamine. Glycans can be homo or heteropolymers of monosaccharide residues, and can be linear or branched. Glycans can be found attached to proteins as in glycoproteins and proteoglycans. They are generally found on the exterior surface of cells. O- and N-linked glycans are very common in eukaryotes but may also be found, although less commonly, in prokaryotes. N-Linked glycans are found attached to the R-group nitrogen (N) of asparagine in the sequon. The sequon is a Asn-X-Ser or Asn-X-Thr sequence, where X is any amino acid except praline.

As used herein, the term "antigen" is defined as any substance capable of eliciting an immune response.

As used herein, the term "immunogenicity" refers to the ability of an immunogen, antigen, or vaccine to stimulate an immune response.

As used herein, the term "CD1d" refers to a member of the CD1 (cluster of differentiation 1) family of glycoproteins expressed on the surface of various human antigen-presenting cells. CDld presented lipid antigens activate natural killer T cells. CDld has a deep antigen-binding groove into which glycolipid antigens bind. CDld molecules expressed on dendritic cells can bind and present glycolipids, including α-GalCer analogs such as C34.

As used herein, the term "epitope" is defined as the parts of an antigen molecule which contact the antigen binding site of an antibody or a T cell receptor.

As used herein, the term "vaccine" refers to a preparation that contains an antigen, consisting of whole disease-causing organisms (killed or weakened) or components of such organisms, such as proteins, peptides, or polysaccharides, that is used to confer immunity against the disease that the organisms cause. Vaccine preparations can be natural, synthetic or derived by recombinant DNA technology.

As used herein, the term "antigen specific" refers to a property of a cell population such that supply of a particular antigen, or a fragment of the antigen, results in specific cell proliferation.

As used herein, the term "specifically binding," refers to the interaction between binding pairs (e.g., an antibody and an antigen). In various instances, specifically binding can be embodied by an affinity constant of about 10⁻⁶ moles/liter, about 10⁻⁷ moles/liter, or about 10⁻⁸ moles/liter, or less.

As used herein, the term "Flow cytometry" or "FACS" means a technique for examining the physical and chemical properties of particles or cells suspended in a stream of fluid, through optical and electronic detection devices.

As used herein, the terms glycoenzymes refers to at least in part the enzymes in the globoseries biosynthetic pathway; exemplary glycoenzymes include alpha-4GalT; beta-4GalNAcT-I; or beta-3GalT-V enzymes.

As used herein, the term "globoseries pathway" refers to a biochemical pathway described in Figure 1.

An "isolated" antibody is one which has been identified and separated and/or recovered from a component of its natural environment. Contaminant components of its natural environment are materials which would interfere with research, diagnostic or therapeutic uses for the antibody, and may include enzymes, hormones, and other proteinaceous or nonproteinaceous solutes. In one aspect, the antibody will be purified (1) to greater than 95% by weight of antibody as determined by, for example, the Lowry method, and in some embodiments more than 99% by weight, (2) to a degree sufficient to obtain at least 15 residues of N-terminal or internal amino acid sequence by use of, for example, a spinning cup sequenator, or (3) to homogeneity by SDS-PAGE under reducing or nonreducing conditions using, for example, Coomassie blue or silver stain. Isolated antibody includes the antibody in situ within recombinant cells since at least one component of the antibody's natural environment will not be present. Ordinarily, however, isolated antibody will be prepared by at least one purification step.

The phrase "substantially similar," "substantially the same", "equivalent", or "substantially equivalent", as used herein, denotes a sufficiently high degree of similarity between two numeric values (for example, one associated with a molecule and the other associated with a reference/comparator molecule) such that one of skill in the art would consider the difference between the two values to be of little or no biological and/or statistical significance within the context of the biological characteristic measured by said values (e.g., Kd values, anti-viral effects, etc.). The difference between said two values is, for example, less than about 50%, less than about 40%, less than about 30%, less than about 20%, and/or less than about 10% as a function of the value for the reference/comparator molecule.

The phrase "substantially reduced," or "substantially different", as used herein, denotes a sufficiently high degree of difference between two numeric values (generally one associated with a molecule and the other associated with a reference/comparator molecule) such that one of skill in the art would consider the difference between the two values to be of statistical significance within the context of the biological characteristic measured by said values (e.g., Kd values). The difference between said two values is, for example, greater than about 10%, greater than about 20%, greater than about 30%, greater than about 40%, and/or greater than about 50% as a function of the value for the reference/comparator molecule.

"Binding affinity" generally refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein. Low-affinity antibodies generally bind antigen slowly and tend to dissociate readily, whereas high-affinity antibodies generally bind antigen faster and tend to remain bound longer. A variety of methods of measuring binding affinity are known in the art, any of which can be used for purposes of the present disclosure. Specific illustrative aspects are described in the following.

In one aspect, the "Kd" or "Kd value" according to this disclosure is measured by a radiolabeled antigen binding assay (RIA) performed with the Fab version of an antibody of interest and its antigen as described by the following assay. Solution binding affinity of Fabs for antigen is measured by equilibrating Fab with a minimal concentration of (1251)-labeled antigen in the presence of a titration series of unlabeled antigen, then capturing bound antigen with an anti-Fab antibody-coated plate (Chen, et al., (1999) J. Mol Biol 293:865-881). To establish conditions for the assay, microtiter plates (Dynex) are coated overnight with 5 µg/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23° C.). In a non-adsorbent plate (Nunc #269620), 100 pM or 26 pM [125I]-antigen are mixed with serial dilutions of a Fab of interest (e.g., consistent with assessment of an anti-VEGF antibody, Fab-12, in Presta et al., (1997) Cancer Res. 57:4593-4599). The Fab of interest is then incubated overnight; however, the incubation may continue for a longer period (e.g., 65 hours) to insure that equilibrium is reached. Thereafter, the mixtures are transferred to the capture plate for incubation at room temperature (e.g., for one hour). The solution is then removed and the plate washed eight times with 0.1% Tween-20 in PBS. When the plates have dried, 150 µl/well of scintillant (MicroScint-20; Packard) is added, and the plates are counted on a Topcount gamma counter (Packard) for ten minutes. Concentrations of each Fab that give less than or equal to 20% of maximal binding are chosen for use in competitive binding assays. According to another aspect the Kd or Kd value is measured by using surface plasmon resonance assays using a BIAcore™-2000 or a BIAcore™-3000 (BIAcore, Inc., Piscataway, N.J.) at 25° C. with immobilized antigen CM5 chips at ^{∼}10 response units (RU). Briefly, carboxymethylated dextran biosensor chips (CM5, BIAcore Inc.) are activated with N-ethyl-N'-(3-dimethylaminopropyl)-carbodiimide hydrochloride (EDC) and N-hydroxysuccinimide (NHS) according to the supplier's instructions. Antigen is diluted with 10 mM sodium acetate, pH 4.8, to 5 µg/ml (^{∼}0.2 µM) before injection at a flow rate of 5 µl/minute to achieve approximately 10 response units (RU) of coupled protein. Following the injection of antigen, 1 M ethanolamine is injected to block unreacted groups. In each experiment, a spot was activated and ethanolamine blocked without immobilizing protein, to be used for reference subtraction. For kinetics measurements, two-fold serial dilutions of Fab (0.78 nM to 500 nM) are injected in PBS with 0.05% Tween 20 (PBST) at 25° C. at a flow rate of approximately 25 µl/min. Association rates (kon) and dissociation rates (koff) are calculated using a simple one-to-one Langmuir binding model (BIAcore Evaluation Software version 3.2) by simultaneously fitting the association and dissociation sensorgrams. The equilibrium dissociation constant (Kd) is calculated as the ratio koff/kon. See, e.g., Chen, Y., et al., (1999) J. Mol Biol 293:865-881. If the on-rate exceeds 106 M-1s-1 by the surface plasmon resonance assay above, then the on-rate can be determined by using a fluorescent quenching technique that measures the increase or decrease in fluorescence emission intensity (excitation=295 nm; emission=340 nm, 16 nm band-pass) at 25° C. of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-Aminco spectrophotometer (ThermoSpectronic) with a stirred cuvette.

An "on-rate" or "rate of association" or "association rate" or "kon" according to this disclosure can also be determined with the same surface plasmon resonance technique described above using a BIAcore™-2000 or a BIAcore™-3000 (BIAcore, Inc., Piscataway, N.J.) at 25° C. with immobilized antigen CM5 chips at "10 response units (RU). Briefly, carboxymethylated dextran biosensor chips (CM5, BIAcore Inc.) are activated with N-ethyl-N '-(3-dimethylaminopropyl)-carbodiimide hydrochloride (EDC) and N-hydroxysuccinimide (NHS) according to the supplier's instructions. Antigen is diluted with 10 mM sodium acetate, pH 4.8, to 5 µg/ml (^{∼}0.2 µM) before injection at a flow rate of 5 µl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% Tween 20 (PBST) at 25° C. at a flow rate of approximately 25 µl/min. Association rates (kon) and dissociation rates (koff) are calculated using a simple one-to-one Langmuir binding model (BIAcore Evaluation Software version 3.2) by simultaneously fitting the association and dissociation sensorgram. The equilibrium dissociation constant (Kd) was calculated as the ratio koff/kon. See, e.g., Chen, Y., et al., (1999)J. Mol Biol 293:865-881. However, if the on-rate exceeds 106 M-1s-1 by the surface plasmon resonance assay above, then the on-rate can be determined by using a fluorescent quenching technique that measures the increase or decrease in fluorescence emission intensity (excitation=295 nm; emission=340 nm, 16 nm band-pass) at 25° C. of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-Aminco spectrophotometer (ThermoSpectronic) with a stirred cuvette.

The term "vector," as used herein, is intended to refer to a nucleic acid molecule capable of transporting another nucleic acid to which it has been linked. One type of vector is a "plasmid", which refers to a circular double stranded DNA loop into which additional DNA segments may be ligated. Another type of vector is a phage vector. Another type of vector is a viral vector, wherein additional DNA segments may be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "recombinant vectors"). In general, expression vectors of utility in recombinant DNA techniques are often in the form of plasmids. In the present specification, "plasmid" and "vector" may be used interchangeably as the plasmid is the most commonly used form of vector.

"Polynucleotide," or "nucleic acid," as used interchangeably herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase, or by a synthetic reaction. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may be further modified after synthesis, such as by conjugation with a label. Other types of modifications include, for example, "caps," substitution of one or more of the naturally occurring nucleotides with an analog, internucleotide modifications such as, for example, those with uncharged linkages (e.g., methyl phosphonates, phosphotriesters, phosphoamidates, carbamates, etc.) and with charged linkages (e.g., phosphorothioates, phosphorodithioates, etc.), those containing pendant moieties, such as, for example, proteins (e.g., nucleases, toxins, antibodies, signal peptides, ply-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotides(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5 ' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-, 2'-O-allyl, 2'-fluoro- or 2'-azido-ribose, carbocyclic sugar analogs, α-anomeric sugars, epimeric sugars such as arabinose, xyloses or lyxoses, pyranose sugars, furanose sugars, sedoheptuloses, acyclic analogs and basic nucleoside analogs such as methyl riboside. One or more phosphodiester linkages may be replaced by alternative linking groups. These alternative linking groups include, but are not limited to, embodiments wherein phosphate is replaced by P(O)S ("thioate"), P(S)S ("dithioate"), "(O)NR2 ("amidate"), P(O)R, P(O)OR', CO or CH2 ("formacetal"), in which each R or R' is independently H or substituted or unsubstituted alkyl (1-20 C) optionally containing an ether (-O-) linkage, aryl, alkenyl, cycloalkyl, cycloalkenyl or araldyl. Not all linkages in a polynucleotide need be identical. The preceding description applies to all polynucleotides referred to herein, including RNA and DNA.

"Oligonucleotide," as used herein, generally refers to short, generally single-stranded, generally synthetic polynucleotides that are generally, but not necessarily, less than about 200 nucleotides in length. The terms "oligonucleotide" and "polynucleotide" are not mutually exclusive. The description above for polynucleotides is equally and fully applicable to oligonucleotides.

"Antibodies" (Abs) and "immunoglobulins" (Igs) are glycoproteins having the same structural characteristics. While antibodies exhibit binding specificity to a specific antigen, immunoglobulins include both antibodies and other antibody-like molecules which generally lack antigen specificity. Polypeptides of the latter kind are, for example, produced at low levels by the lymph system and at increased levels by myelomas.

The terms "antibody" and "immunoglobulin" are used interchangeably in the broadest sense and include monoclonal antibodies (e.g., full length or intact monoclonal antibodies), polyclonal antibodies, monovalent, multivalent antibodies, multispecific antibodies (e.g., bispecific antibodies so long as they exhibit the desired biological activity) and may also include certain antibody fragments (as described in greater detail herein). An antibody can be chimeric, human, humanized and/or affinity matured.

The "variable region" or "variable domain" of an antibody refers to the amino-terminal domains of heavy or light chain of the antibody. These domains are generally the most variable parts of an antibody and contain the antigen-binding sites.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called complementarity-determining regions (CDRs) or hypervariable regions both in the light-chain and the heavy-chain variable domains. The more highly conserved portions of variable domains are called the framework (FR). The variable domains of native heavy and light chains each comprise four FR regions, largely adopting a beta-sheet configuration, connected by three CDRs, which form loops connecting, and in some cases forming part of, the beta-sheet structure. The CDRs in each chain are held together in close proximity by the FR regions and, with the CDRs from the other chain, contribute to the formation of the antigen-binding site of antibodies (see Kabat et al., Sequences of Proteins of Immunological Interest, Fifth Edition, National Institute of Health, Bethesda, Md. (1991)). The constant domains are not involved directly in binding an antibody to an antigen, but exhibit various effector functions, such as participation of the antibody in antibody-dependent cellular toxicity.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')2 fragment that has two antigen-combining sites and is still capable of cross-linking antigen.

"Fv" is the minimum antibody fragment which contains a complete antigen-recognition and -binding site. In a two-chain Fv species, this region consists of a dimer of one heavy- and one light-chain variable domain in tight, non-covalent association. In a single-chain Fv species, one heavy- and one light-chain variable domain can be covalently linked by a flexible peptide linker such that the light and heavy chains can associate in a "dimeric" structure analogous to that in a two-chain Fv species. It is in this configuration that the three CDRs of each variable domain interact to define an antigen-binding site on the surface of the VH-VL dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (CHI) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab' )2 antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa (κ) and lambda (λ), based on the amino acid sequences of their constant domains.

Depending on the amino acid sequences of the constant domains of their heavy chains, antibodies (immunoglobulins) can be assigned to different classes. There are five major classes of immunoglobulins: IgA, IgD, IgE, IgG and IgM, and several of these may be further divided into subclasses (isotypes), e.g., IgG1, IgG2, IgG3, IgG4, IgA1, and IgA2. The heavy chain constant domains that correspond to the different classes of immunoglobulins are called α, δ, ε, γ, and µ, respectively. The subunit structures and three-dimensional configurations of different classes of immunoglobulins are well known and described generally in, for example, Abbas et al. Cellular and Mol. Immunology, 4th ed. (2000). An antibody may be part of a larger fusion molecule, formed by covalent or non-covalent association of the antibody with one or more other proteins or peptides.

The terms "full length antibody," "intact antibody" and "whole antibody" are used herein interchangeably, to refer to an antibody in its substantially intact form, not antibody fragments as defined below. The terms particularly refer to an antibody with heavy chains that contain the Fc region.

"Antibody fragments" comprise only a portion of an intact antibody, wherein the portion retains at least one, and as many as most or all, of the functions normally associated with that portion when present in an intact antibody. In one embodiment, an antibody fragment comprises an antigen binding site of the intact antibody and thus retains the ability to bind antigen. In another embodiment, an antibody fragment, for example one that comprises the Fc region, retains at least one of the biological functions normally associated with the Fc region when present in an intact antibody, such as FcRn binding, antibody half life modulation, ADCC function and complement binding. In one embodiment, an antibody fragment is a monovalent antibody that has an in vivo half life substantially similar to an intact antibody. For example, such an antibody fragment may comprise an antigen binding arm linked to an Fc sequence capable of conferring in vivo stability to the fragment.

The term "monoclonal antibody" as used herein refers to an antibody obtained from a population of substantially homogeneous antibodies, i.e., the individual antibodies comprising the population are identical except for possible naturally occurring mutations that may be present in minor amounts. Thus, the modifier "monoclonal" indicates the character of the antibody as not being a mixture of discrete antibodies. Such monoclonal antibody typically includes an antibody comprising a polypeptide sequence that binds a target, wherein the target-binding polypeptide sequence was obtained by a process that includes the selection of a single target binding polypeptide sequence from a plurality of polypeptide sequences. For example, the selection process can be the selection of a unique clone from a plurality of clones, such as a pool of hybridoma clones, phage clones or recombinant DNA clones. It should be understood that the selected target binding sequence can be further altered, for example, to improve affinity for the target, to humanize the target binding sequence, to improve its production in cell culture, to reduce its immunogenicity in vivo, to create a multispecific antibody, etc., and that an antibody comprising the altered target binding sequence is also a monoclonal antibody of this invention. In contrast to polyclonal antibody preparations which typically include different antibodies directed against different determinants (epitopes), each monoclonal antibody of a monoclonal antibody preparation is directed against a single determinant on an antigen. In addition to their specificity, the monoclonal antibody preparations are advantageous in that they are typically uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including, for example, the hybridoma method (e.g., Kohler et al., Nature, 256: 495 (1975); Harlow et al., Antibodies: A Laboratory Manual, (Cold Spring Harbor Laboratory Press, 2nd ed. 1988); Hammerling et al., in: Monoclonal Antibodies and T-Cell hybridomas 563-681 (Elsevier, N.Y., 1981)), recombinant DNA methods (see, e.g., U.S. Pat. No. 4,816,567), phage display technologies (See, e.g., Clackson et al., Nature, 352: 624-628 (1991); Marks et al., J. Mol. Biol. 222: 581-597 (1992); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132 (2004), and technologies for producing human or human-like antibodies in animals that have parts or all of the human immunoglobulin loci or genes encoding human immunoglobulin sequences (see, e.g., WO98/24893; WO96/34096; WO96/33735; WO91/10741; Jakobovits et al., Proc. Natl. Acad. Sci. USA 90: 2551 (1993); Jakobovits et al., Nature 362: 255-258 (1993); Bruggemann et al., Year in Immunol. 7:33 (1993); U.S. Pat. Nos. 5,545,807; 5,545,806; 5,569,825; 5,625,126; 5,633,425; 5,661,016; Marks et al., Bio. Technology 10: 779-783 (1992); Lonberg et al., Nature 368: 856-859 (1994); Morrison, Nature 368: 812-813 (1994); Fishwild et al., Nature Biotechnol. 14: 845-851 (1996); Neuberger, Nature Biotechnol. 14: 826 (1996) and Lonberg and Huszar, Intern. Rev. Immunol. 13: 65-93 (1995).

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Pat. No. 4,816,567; and Morrison et al., Proc. Natl. Acad. Sci. USA 81:6851-6855 (1984)).

Humanized" forms of non-human (e.g., murine) antibodies are chimeric antibodies that contain minimal sequence derived from non-human immunoglobulin. In one embodiment, a humanized antibody is a human immunoglobulin (recipient antibody) in which residues from a hypervariable region of the recipient are replaced by residues from a hypervariable region of a non-human species (donor antibody) such as mouse, rat, rabbit or nonhuman primate having the desired specificity, affinity, and/or capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally will also comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992). See also the following review articles and references cited therein: Vaswani and Hamilton, Ann. Allergy, Asthma & Immunol. 1:105-115 (1998); Harris, Biochem. Soc. Transactions 23:1035-1038 (1995); Hurle and Gross, Curr. Op. Biotech. 5:428-433 (1994).

The term "hypervariable region", "HVR", or "HV", when used herein refers to the regions of an antibody variable domain which are hypervariable in sequence and/or form structurally defined loops. Generally, antibodies comprise six hypervariable regions; three in the VH (HI, H2, H3), and three in the VL (LI, L2, L3). A number of hypervariable region delineations are in use and are encompassed herein. The Kabat Complementarity Determining Regions (CDRs) are based on sequence variability and are the most commonly used (Kabat et al., Sequences of Proteins of Immunological Interest, 5th Ed. Public Health Service, National Institutes of Health, Bethesda, Md. (1991)). Chothia refers instead to the location of the structural loops (Chothia and Lesk J. Mol. Biol. 196:901-917 (1987)). The AbM hypervariable regions represent a compromise between the Kabat CDRs and Chothia structural loops, and are used by Oxford Molecular's AbM antibody modeling software. The "contact" hypervariable regions are based on an analysis of the available complex crystal structures. The residues from each of these hypervariable regions are noted below.

### Loop Kabat AbM Chothia Contact

L1 L24-L34 L24-L34 L26-L32 L30-L36
L2 L50-L56 L50-L56 L50-L52 L46-L55
L3 L89-L97 L89-L97 L91-L96 L89-L96
H1 H31-H35B H26-H35B H26-H32 H30-H35B

### (Kabat Numbering)

H1 H31-H35 H26-H35 H26-H32 H30-H35

### (Chothia Numbering)

H2 H50-H65 H50-H58 H53-H55 H47-H58
H3 H95-H102 H95-H102 H96-H101 H93-H101
Hypervariable regions may comprise "extended hypervariable regions" as follows: 24-36 or 24-34 (LI), 46-56 or 50-56 or 49-56 (L2) and 89-97 or 89-96 (L3) in the VL and 26-35 (HI), 50-65 or 49-65 (H2) and 93-102, 94-102, or 95-102 (H3) in the VH. The variable domain residues are numbered according to Kabat et al., supra, for each of these definitions.

"Framework" or "FR" residues are those variable domain residues other than the hypervariable region residues as herein defined.

The term "variable domain residue numbering as in Kabat" or "amino acid position numbering as in Kabat," and variations thereof, refers to the numbering system used for heavy chain variable domains or light chain variable domains of the compilation of antibodies in Kabat et al., Sequences of Proteins of Immunological Interest, 5th Ed. Public Health Service, National Institutes of Health, Bethesda, Md. (1991). Using this numbering system, the actual linear amino acid sequence may contain fewer or additional amino acids corresponding to a shortening of, or insertion into, a FR or HVR of the variable domain. For example, a heavy chain variable domain may include a single amino acid insert (residue 52a according to Kabat) after residue 52 of H2 and inserted residues (e.g. residues 82a, 82b, and 82c, etc. according to Kabat) after heavy chain FR residue 82. The Kabat numbering of residues may be determined for a given antibody by alignment at regions of homology of the sequence of the antibody with a "standard" Kabat numbered sequence.

"Single-chain Fv" or "scFv" antibody fragments comprise the VH and VL domains of antibody, wherein these domains are present in a single polypeptide chain. Generally, the scFv polypeptide further comprises a polypeptide linker between the VH and VL domains which enables the scFv to form the desired structure for antigen binding. For a review of scFv see Pluckthun, in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites. Diabodies are described more fully in, for example, EP 404,097; WO93/1161; and Hollinger et al., Proc. Natl. Acad. Sci. USA 90: 6444-6448 (1993).

A "human antibody" is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen-binding residues.

An "affinity matured" antibody is one with one or more alterations in one or more HVRs thereof which result in an improvement in the affinity of the antibody for antigen, compared to a parent antibody which does not possess those alteration(s). In one embodiment, an affinity matured antibody has nanomolar or even picomolar affinities for the target antigen. Affinity matured antibodies are produced by procedures known in the art. Marks et al. Bio/Technology 10:779-783 (1992) describes affinity maturation by VH and VL domain shuffling. Random mutagenesis of CDR and/or framework residues is described by: Barbas et al. Proc Nat. Acad. Sci. USA 91:3809-3813 (1994); Schier et al. Gene 169:147-155 (1995); Yelton et al. J. Immunol. 155:1994-2004 (1995); Jackson et al., J. Immunol. 154(7):3310-9 (1995); and Hawkins et al, J. Mol. Biol. 226:889-896 (1992).

A "blocking" antibody or an "antagonist" antibody is one which inhibits or reduces biological activity of the antigen it binds. Certain blocking antibodies or antagonist antibodies substantially or completely inhibit the biological activity of the antigen.

An "agonist antibody", as used herein, is an antibody which mimics at least one of the functional activities of a polypeptide of interest.

A "disorder" is any condition that would benefit from treatment with an antibody of the invention. This includes chronic and acute disorders or diseases including those pathological conditions which predispose the mammal to the disorder in question. Nonlimiting examples of disorders to be treated herein include cancer.

The terms "cell proliferative disorder" and "proliferative disorder" refer to disorders that are associated with some degree of abnormal cell proliferation. In one aspect, the cell proliferative disorder is cancer.

"Tumor," as used herein, refers to all neoplastic cell growth and proliferation, whether malignant or benign, and all pre-cancerous and cancerous cells and tissues. The terms "cancer," "cancerous," "cell proliferative disorder," "proliferative disorder" and "tumor" are not mutually exclusive as referred to herein.

The terms "cancer" and "cancerous" refer to or describe the physiological condition in mammals that is typically characterized by unregulated cell growth/proliferation. Examples of cancer include carcinoma, lymphoma (e.g., Hodgkin's and non-Hodgkin's lymphoma), blastoma, sarcoma, and leukemia. More particular examples of such cancers include squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney cancer, liver cancer, prostate cancer, vulval cancer, thyroid cancer, hepatic carcinoma, leukemia and other lymphoproliferative disorders, and various types of head and neck cancer.

The term "muscular disorder" refers to or describes the physiological condition in muscle-containing animals that is typically characterized by deterioration or weakening of skeletal and/or smooth muscle such that normal muscular function is significantly reduced. Examples of muscular disorders include muscular dystrophy, multiple
sclerosis, amyotrophic lateral sclerosis, Isaac's syndrome; stiff-person syndrome; familiar periodic paralyses, myopathy, myotonia, rhabdomyolyses, muscle atrophy, and various types of muscle weakness and muscle rigidity.

The term "globoseries -related disorder" refers to or describes a disorder that is typically characterized by or contributed to by aberrant functioning or presentation of the pathway. Examples of such disorders include hyperproliferative
diseases, including cancer.

The terms "neurological disorder" or "neurological disease" refer to or describe a disease or disorder of the central and/or peripheral nervous system in mammals that is typically characterized by deterioration of nervous tissue or deterioration of communication between cells in nervous tissue. Examples of neurological disorders include neurodegenerative diseases (including Lewy body disease,
postpoliomyelitis syndrome, Shy-Draeger syndrome, olivopontocerebellar atrophy, Parkinson's disease, multiple system atrophy, striatonigral degeneration, tauopathies (including Alzheimer disease and supranuclear palsy), prion diseases
(including bovine spongiform encephalopathy, scrapie, Creutzfeldt-Jakob syndrome, kuru, Gerstmann-Straussler-Scheinker disease, chronic wasting disease, and fatal familial insomnia), bulbar palsy, motor neuron disease, and nervous system heterodegenerative disorders (including Canavan disease, Huntington's disease, neuronal ceroid-lipofuscinosis, Alexander's disease, Tourette's syndrome, Menkes kinky hair syndrome, Cockayne syndrome, Halervorden-Spatz syndrome, lafora disease, Rett syndrome, hepatolenticular degeneration, Lesch-Nyhan syndrome, and Unverricht-Lundborg
syndrome), dementia (including Pick's disease, and spinocerebellar ataxia.

The terms "inflammatory disorder" and "immune disorder" refer to or describe disorders caused by aberrant immunologic mechanisms and/or aberrant cytokine signaling. Examples of inflammatory and immune disorders include autoimmune
diseases, immunologic deficiency syndromes, and hypersensitivity. An "autoimmune disease" herein is a non-malignant disease or disorder arising from and directed against an individual's own tissues. The autoimmune diseases herein specifically exclude malignant or cancerous diseases or conditions, especially excluding B cell lymphoma, acute lymphoblastic leukemia (ALL), chronic lymphocytic leukemia (CLL), Hairy cell leukemia and chronic myeloblastic leukemia. Examples of autoimmune diseases or disorders include
inflammatory responses such as inflammatory skin diseases including psoriasis and dermatitis (e.g. atopic dermatitis); systemic scleroderma and sclerosis; responses associated with inflammatory bowel disease (such as Crohn's disease and ulcerative colitis); respiratory distress syndrome (including adult respiratory distress syndrome; ARDS); dermatitis; meningitis; encephalitis; uveitis; colitis; glomerulonephritis; allergic conditions such as eczema and asthma and other conditions involving infiltration of T cells and chronic inflammatory responses; atherosclerosis; leukocyte adhesion deficiency; rheumatoid arthritis; systemic lupus erythematosus (SLE) (including lupus nephritis, cutaneous lupus); diabetes mellitus (e.g. Type I diabetes mellitus or insulin dependent diabetes mellitus); multiple sclerosis; Reynaud's syndrome; autoimmune thyroiditis; Hashimoto's thyroiditis; allergic encephalomyelitis; Sjogren's syndrome; juvenile onset diabetes; and immune responses associated with acute and delayed hypersensitivity mediated by cytokines and T-lymphocytes typically found in tuberculosis, sarcoidosis, polymyositis, granulomatosis and vasculitis; pernicious anemia (Addison's disease); diseases involving leukocyte diapedesis; central nervous system (CNS) inflammatory disorder; multiple organ injury syndrome; hemolytic anemia (including cryoglobinemia or Coombs positive anemia);
myasthenia gravis; antigen-antibody complex mediated diseases; anti-glomerular basement membrane disease; antiphospholipid syndrome; allergic neuritis; Graves' disease; Lambert-Eaton myasthenic syndrome; pemphigoid bullous; pemphigus; autoimmune polyendocrinopathies; Reiter's disease; stiff-man syndrome; Behcet disease; giant cell arteritis; immune complex nephritis; IgA nephropathy; IgM polyneuropathies; immune thrombocytopenic purpura (ITP) or autoimmune thrombocytopenia, etc.

Examples of immunologic deficiency syndromes include
ataxia telangiectasia, leukocyte-adhesion deficiency syndrome, lymphopenia, dysgammaglobulinemia, HIV or deltaretrovirus infections, common variable immunodeficiency, severe combined immunodeficiency, phagocyte bactericidal dysfunction, agammaglobulinemia, DiGeorge syndrome, and Wiskott-Aldrich syndrome. Examples of hypersensitivity include allergies, asthma, dermatitis, hives,
anaphylaxis, Wissler's syndrome, and thrombocytopenic purpura.

As used herein, "treatment" refers to clinical intervention in an attempt to alter the natural course of the individual or cell being treated, and can be performed either for prophylaxis or during the course of clinical pathology. Desirable effects of treatment include preventing occurrence or recurrence of disease, alleviation of symptoms, diminishment of any direct or indirect pathological consequences of the disease, preventing or decreasing inflammation and/or tissue/organ damage, decreasing the rate of disease progression, amelioration or palliation of the disease state, and remission or improved prognosis. In some embodiments, antibodies of the invention are used to delay development of a disease or disorder.

An "individual" or a "subject" is a vertebrate. In certain embodiments, the vertebrate is a mammal. Mammals include farm animals (such as cows), sport
animals, pets (such as cats, dogs, and horses), primates, mice and rats. In certain embodiments, the vertebrate is a human.

"Mammal" for purposes of treatment refers to any animal classified as a mammal, including humans, domestic and farm animals, and zoo, sports, or pet animals, such as dogs, horses, cats, cows, etc. In certain embodiments, the mammal is human.

An "effective amount" refers to an amount effective, at dosages and for periods of time necessary, to achieve the desired therapeutic or prophylactic result.

A "therapeutically effective amount" of a substance/molecule of the invention may vary according to factors such as the disease state, age, sex, and weight of the individual, and the ability of the substance/molecule, to elicit a desired response in the individual. A therapeutically effective amount is also one in which any toxic or detrimental effects of the substance/molecule are outweighed by the therapeutically beneficial effects. A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount would be less than the therapeutically effective amount.

The term "cytotoxic agent" as used herein refers to a substance that inhibits or prevents the function of cells and/or causes destruction of cells. The term is intended to include radioactive isotopes (e.g., At211, 1131, 1125, Y90, Re186, Re188, Sm153, Bi212, P32, Pb212 and radioactive isotopes of Lu), chemotherapeutic agents (e.g., methotrexate, adriamicin, vinca alkaloids (vincristine, vinblastine, etoposide), doxorubicin, melphalan, mitomycin C, chlorambucil, daunorubicin or other intercalating agents, enzymes and fragments thereof such as nucleolyticenzymes, antibiotics, and toxins such as small molecule toxins or enzymatically active toxins of bacterial, fungal, plant or animal origin, including fragments and/or variants thereof, and the various antitumor or anticancer agents disclosed below. Other cytotoxic agents are described below. A tumoricidal agent causes destruction of tumor cells.

A "chemotherapeutic agent" is a chemical compound useful in the treatment of cancer. Examples of chemotherapeutic agents include alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); delta-9-tetrahydrocannabinol (dronabinol, MARINOL®); beta-lapachone; lapachol; colchicines; betulinic acid; a camptothecin (including the synthetic analogue topotecan (HYCAMTIN®), CPT-11 (irinotecan, CAMPTOSAR®), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlomaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g., calicheamicin, especially calicheamicin gamma1I and calicheamicin omegaI1 (see, e.g., Agnew, Chem. Intl. Ed. Engl., 33: 183-186 (1994)); dynemicin, including dynemicin A; an esperamicin; as well as neocarzinostatin chromophore and related chromoprotein enediyne antiobiotic chromophores), aclacinomysins, actinomycin, authramycin, azaserine, bleomycins, cactinomycin, carabicin, caminomycin, carzinophilin, chromomycinis, dactinomycin, daunorubicin, detorubicin, 6-diazo-5-oxo-L-norleucine, ADRIAMYCIN® doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK® polysaccharide complex (JHS Natural Products, Eugene, Oreg.); razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine (ELDISINE®, FILDESIN®); dacarbazine; mannomustine; mitobronitol; mitolactol; pipobroman; gacytosine; arabinoside ("Ara-C"); thiotepa; taxoids, e.g., TAXOL® paclitaxel (Bristol-Myers Squibb Oncology, Princeton, N.J.), ABRAXANE™ Cremophor-free, albumin-engineered nanoparticle formulation of paclitaxel (American Pharmaceutical Partners, Schaumberg, Ill.), and TAXOTERE® doxetaxel (Rhône-Poulenc Rorer, Antony, France); chloranbucil; gemcitabine (GEMZAR®); 6-thioguanine; mercaptopurine; methotrexate; platinum analogs such as cisplatin and carboplatin; vinblastine (VELBAN®); platinum; etoposide (VP-16); ifosfamide; mitoxantrone; vincristine (ONCOVIN®); oxaliplatin; leucovovin; vinorelbine (NAVELBINE®); novantrone; edatrexate; daunomycin; aminopterin; ibandronate; topoisomerase inhibitor RFS 2000; difluoromethylornithine (DMFO); retinoids such as retinoic acid; capecitabine (XELODA®); pharmaceutically acceptable salts, acids or derivatives of any of the above; as well as combinations of two or more of the above such as CHOP, an abbreviation for a combined therapy of cyclophosphamide, doxorubicin, vincristine, and prednisolone, and FOLFOX, an abbreviation for a treatment regimen with oxaliplatin (ELOXATIN™) combined with 5-FU and leucovovin.

Also included in this definition are anti-hormonal agents that act to regulate, reduce, block, or inhibit the effects of hormones that can promote the growth of cancer, and are often in the form of systemic, or whole-body treatment. They may be hormones themselves. Examples include anti-estrogens and selective estrogen receptor modulators (SERMs), including, for example, tamoxifen (including NOLVADEX® tamoxifen), EVISTA® raloxifene, droloxifene, 4-hydroxytamoxifen, trioxifene, keoxifene, LY117018, onapristone, and FARESTON® toremifene; anti-progesterones; estrogen receptor down-regulators (ERDs); agents that function to suppress or shut down the ovaries, for example, leutinizing hormone-releasing hormone (LHRH) agonists such as LUPRON® and ELIGARD® leuprolide acetate, goserelin acetate, buserelin acetate and tripterelin; other anti-androgens such as flutamide, nilutamide and bicalutamide; and aromatase inhibitors that inhibit the enzyme aromatase, which regulates estrogen production in the adrenal glands, such as, for example, 4(5)-imidazoles, aminoglutethimide, MEGASE® megestrol acetate, AROMASIN® exemestane, formestanie, fadrozole, RIVISOR® vorozole, FEMARA® letrozole, and ARIMIDEX® anastrozole. In addition, such definition of chemotherapeutic agents includes bisphosphonates such as clodronate (for example, BONEFOS® or OSTAC®), DIDROCAL® etidronate, NE-58095, ZOMETA® zoledronic acid/zoledronate, FOSAMAX® alendronate, AREDIA® pamidronate, SKELID® tiludronate, or ACTONEL® risedronate; as well as troxacitabine (a 1,3-dioxolane nucleoside cytosine analog); antisense oligonucleotides, particularly those that inhibit expression of genes in signaling pathways implicated in aberrant cell proliferation, such as, for example, PKC-alpha, Raf, H-Ras, and epidermal growth factor receptor (EGF-R); vaccines such as TFIERATOPE® vaccine and gene therapy vaccines, for example, ALLOVECTIN® vaccine, LEUVECTIN® vaccine, and VAXID® vaccine; LURTOTECAN® topoisomerase 1 inhibitor; ABARELIX® rmRH; lapatinib ditosylate (an ErbB-2 and EGFR dual tyrosine kinase small-molecule inhibitor also known as GW572016); and pharmaceutically acceptable salts, acids or derivatives of any of the above.

Unless defined otherwise, all technical and scientific terms used herein have the same meanings as commonly understood by one of ordinary skill in the art to which this invention belongs. Although any methods and materials similar or equivalent to those described herein can be used in the practice or testing of the present invention, the preferred methods and materials are now described.
All references cited in this specification are to be taken as indicative of the level of skill in the art. Nothing herein is to be construed as an admission that the invention is not entitled to antedate such disclosure by virtue of prior invention.

Most recently, Wong et al25 attempts to improve the immunogenicity of the vaccine, the research team synthesized a variety of GH-derivatives containing modifications at either the reducing end or at the non-reducing end of the carbohydrate, and found that vaccines containing GH modified with a fluoro, azido, or phenyl group at the reducing end or an azido group at the non-reducing end could stimulate the production of GH, SSEA3, and SSEA4-targeting antibodies, with the latter vaccine eliciting an especially favorable high ratio of IgG:IgM antibodies not usually achieved in anticancer vaccines. Encouragingly, the antibodies produced in response to these vaccines mediated complement-dependent cytotoxicity towards cultured GH-positive human breast cancer cells.

The present disclosure is based on the surprising discovery that the modification of the stage-specific embryonic antigens (SSEA3 and SSEA4) with certain groups elicited robust IgG antibody response to specifically recognize SSEA3 and SSEA4, respectively.

In some examples, the modification of SSEA-3 comprises a fluoro, an azido or an O-phenyl group at the one or more positions of the glucose of SSEA-3. In some examples, the modification of SSEA-3 comprises a fluoro, an azido or an O-phenyl group at the one or more positions of the non-reducing end galactose. In some examples, the modification of SSEA-4 comprises a fluoro, an azido or an O-phenyl group at one or more positions of the glucose of SSEA-4. In some examples, the modification of SSEA-4 comprises a fluoro, an azido or an O-phenyl group at one or more positions of the sialic acid residue.

Described herein are SSEA3 and SSEA4 derivatives having the modification at the reducing and/or non-reducing end. Such SSEA3 and SSEA4 derivatives can elicit a stronger immune response (e.g., induction of IgG antibodies against SSEA3 and/or SSEA4) as compared to the native SSEA3 and SSEA4. The antibodies induced by an immmunogenic composition comprising such unnatural glycan moiety are able to mediate the complement-dependent cell cytotoxicity against tumor cells.

### Compounds

Accordingly, the present disclosure also features novel compounds consisting of the modified carbohydrate antigens (SSEA3 and SSEA4), glycan conjugates comprising such, and immmunogenic compositions and vaccines thereof.

In one aspect, the present disclosure provides a compound of formula (I): 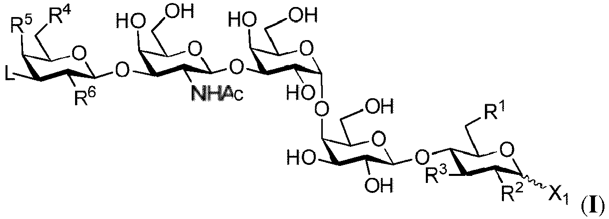 or a salt thereof, wherein:
X₁ is -OR or -SR, wherein R is hydrogen, a oxygen or sulfur protecting group, optionally substituted C₁₋₁₀ alkyl, optionally substituted aryl, optionally substituted acyl, or optionally substituted imidoyl;
each instance of R¹, R², R³, R⁴, R⁵, R⁶ and L is independently selected from hydrogen, halogen, optionally substituted alkyl, optionally substituted alkenyl, optionally substituted alkynyl, optionally substituted heterocyclyl, optionally substituted aryl, -N₃, -NO₂, -N(R^{B})₂, -N(R^{A})C(O)R^{A}, -OR^{A}, -OC(O)R^{A}, -SR^{A}, -C(O)N(R^{B})₂, -CN, -C(O)R^{A}, -C(O)OR^{A}, - S(O)R^{A}, -SO₂R^{A}, -SO₂N(R^{b})₂, and -NHSO₂R^{B};
each instance of R^{A} is independently selected from hydrogen, optionally substituted alkyl, optionally substituted alkenyl, optionally substituted alkynyl, optionally substituted heterocyclyl, and optionally substituted aryl;
each instance of R^{B} is independently selected from hydrogen, optionally substituted alkyl, optionally substituted alkenyl, optionally substituted alkynyl, optionally substituted heterocyclyl, and optionally substituted aryl; and
provided the compound is not of the formula:

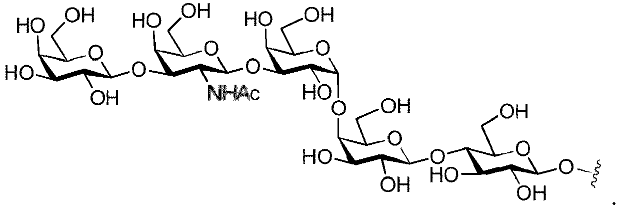

In certain aspects, X₁ is in the alpha configuration. In certain aspects, X₁ is in the beta configuration.

In some aspects, X₁ is -OR^{A}. In some aspects, X₁ is -OH. In some aspects, X₁ is - O(protecting group). In some aspects, X₁ is -OR^{A}, wherein R^{A} is unsubstituted C₁₋₁₀ alkyl. In some aspects, X₁ is -OR^{A}, wherein R^{A} is substituted C₁₋₁₀ alkyl. In some aspects, X₁ is -OR^{A}, wherein R^{A} is unsubstituted aryl. In some aspects, X₁ is -OR^{A}, wherein R^{A} is substituted aryl. In some aspects, X₁ is - OR^{A}, wherein R^{A} is unsubstituted acyl. In some aspects, X₁ is -OR^{A}, wherein R^{A} is substituted acyl. In some aspects, X₁ is -OR^{A}, wherein R^{A} is unsubstituted imidoyl. In some aspects, X₁ is -OR^{A}, wherein R^{A} is substituted imidoyl.

In some aspects, X₁ is -SR^{A}. In some aspects, X₁ is -SH. In some aspects, X₁ is - S(protecting group). In some aspects, X₁ is -SR^{A}, wherein R^{A} is unsubstituted C₁₋₁₀ alkyl. In some aspects, X₁ is -SR^{A}, wherein R^{A} is substituted C₁₋₁₀ alkyl. In certain aspects, X₁ is - SCH₃. In some aspects, X₁ is -SR^{A}, wherein R^{A} is unsubstituted aryl. In some aspects, X₁ is -SR^{A}, wherein R^{A} is substituted aryl. In some aspects, X₁ is -SR^{A}, wherein R^{A} is unsubstituted acyl. In some aspects, X₁ is -SR^{A}, wherein R^{A} is substituted acyl. In some aspects, X₁ is -SR^{A}, wherein R^{A} is unsubstituted imidoyl. In some aspects, X₁ is -SR^{A}, wherein R^{A} is substituted imidoyl.

In some aspects, X₁ is C₁₋₁₀ alkoxy. In some aspects, X₁ is C₁₋₃ alkoxy. In certain aspects, X₁ is methoxy. In certain aspects, X₁ is alpha-methoxy.

In some aspects, X₁ is selected from the group consisting of alpha-thiomethyl, beta-thiomethyl, alpha-thiocresyl, beta-thiocresyl, alpha-t-butyldiphenylsilyloxy, beta-t-butyldiphenylsilyloxy, and alpha-methoxy.

In some aspects, R¹ is -N₃ or -N(R^{W})₂, wherein each R^{W} is independently hydrogen or a nitrogen protecting group. In certain aspects, R¹ is -N₃. In certain aspects, R¹ is - N(R^{W})₂, wherein each R^{W} is independently hydrogen or a nitrogen protecting group. In certain aspects, R¹ is -NH₂. In certain aspects, R¹ is - NHR^{W}, wherein R^{W} is a nitrogen protecting group. In certain aspects, R¹ is -N(R^{W})₂, wherein each R^{W} is a nitrogen protecting group. In certain aspects, R¹ is selected from the group consisting of -N₃, -NH(Cbz), - NH(Boc), -NH(Fmoc), -NHC(O)CCl₃, -NHC(O)CH₃, and -N(C(O)CH₃)₂. In certain aspects, R¹ is -NH(Cbz). In certain aspects, R¹ is -NH(Fmoc). In certain aspects, R¹ is - NHC(O)CCl₃. In certain aspects, R¹ is -NHC(O)CH₃. In certain aspects, R¹ is - N(C(O)CH₃)₂.

In some aspects, R² is -N₃ or -N(R^{W})₂, wherein each R^{W} is independently hydrogen or a nitrogen protecting group. In certain aspects, R² is -N₃. In certain aspects, R² is - N(R^{W})₂, wherein each R^{W} is independently hydrogen or a nitrogen protecting group. In certain aspects, R² is -NH₂. In certain aspects, R² is - NHR^{W}, wherein R^{W} is a nitrogen protecting group. In certain aspects, R² is -N(R^{W})₂, wherein each R^{W} is a nitrogen protecting group. In certain aspects, R² is selected from the group consisting of -N₃, -NH(Cbz), - NH(Boc), -NH(Fmoc), -NHC(O)CCl₃, -NHC(O)CH₃, and -N(C(O)CH₃)₂. In certain aspects, R² is -NH(Cbz). In certain aspects, R² is -NH(Fmoc). In certain aspects, R² is - NHC(O)CCl₃. In certain aspects, R² is -NHC(O)CH₃. In certain aspects, R² is - N(C(O)CH₃)₂.

In some aspects, R³ is -N₃ or -N(R^{W})₂, wherein each R^{W} is independently hydrogen or a nitrogen protecting group. In certain aspects, R³ is -N₃. In certain aspects, R³ is - N(R^{W})₂, wherein each R^{W} is independently hydrogen or a nitrogen protecting group. In certain aspects, R³ is -NH₂. In certain aspects, R³ is - NHR^{W}, wherein R^{W} is a nitrogen protecting group. In certain aspects, R³ is -N(R^{W})₂, wherein each R^{W} is a nitrogen protecting group. In certain aspects, R³ is selected from the group consisting of -N₃, -NH(Cbz), - NH(Boc), -NH(Fmoc), -NHC(O)CCl₃, -NHC(O)CH₃, and -N(C(O)CH₃)₂. In certain aspects, R³ is -NH(Cbz). In certain aspects, R³ is -NH(Fmoc). In certain aspects, R³ is - NHC(O)CCl₃. In certain aspects, R³ is -NHC(O)CH₃. In certain aspects, R³ is - N(C(O)CH₃)₂.

In some aspects, R⁴ is -N₃ or -N(R^{W})₂, wherein each R⁴ is independently hydrogen or a nitrogen protecting group. In certain aspects, R⁴ is -N₃. In certain aspects, R⁴ is - N(R^{W})₂, wherein each R^{W} is independently hydrogen or a nitrogen protecting group. In certain aspects, R⁴ is -NH₂. In certain aspects, R⁴ is - NHR^{W}, wherein R^{W} is a nitrogen protecting group. In certain aspects, R⁴ is -N(R^{W})₂, wherein each R^{W} is a nitrogen protecting group. In certain aspects, R⁴ is selected from the group consisting of -N₃, -NH(Cbz), - NH(Boc), -NH(Fmoc), -NHC(O)CCl₃, -NHC(O)CH₃, and -N(C(O)CH₃)₂. In certain aspects, R⁴ is -NH(Cbz). In certain aspects, R⁴ is -NH(Fmoc). In certain aspects, R⁴ is - NHC(O)CCl₃. In certain aspects, R⁴ is -NHC(O)CH₃. In certain aspects, R⁴ is - N(C(O)CH₃)₂.

In some aspects, R⁵ is -N₃ or -N(R^{W})₂, wherein each R^{W} is independently hydrogen or a nitrogen protecting group. In certain aspects, R⁵ is -N₃. In certain aspects, R⁵ is - N(R^{W})₂, wherein each R^{W} is independently hydrogen or a nitrogen protecting group. In certain aspects, R⁵ is -NH₂. In certain aspects, R⁵ is - NHR^{W}, wherein R^{W} is a nitrogen protecting group. In certain aspects, R⁵ is -N(R^{W})₂, wherein each R^{W} is a nitrogen protecting group. In certain aspects, R⁵ is selected from the group consisting of -N₃, -NH(Cbz), - NH(Boc), -NH(Fmoc), -NHC(O)CCl₃, -NHC(O)CH₃, and -N(C(O)CH₃)₂. In certain aspects, R⁵ is -NH(Cbz). In certain aspects, R⁵ is -NH(Fmoc). In certain aspects, R⁵ is - NHC(O)CCl₃. In certain aspects, R⁵ is -NHC(O)CH₃. In certain aspects, R⁵ is - N(C(O)CH₃)₂.

In some aspects, R⁶ is -N₃ or -N(R^{W})₂, wherein each R^{W} is independently hydrogen or a nitrogen protecting group. In certain aspects, R⁶ is -N₃. In certain aspects, R⁶ is - N(R^{W})₂, wherein each R^{W} is independently hydrogen or a nitrogen protecting group. In certain aspects, R⁶ is -NH₂. In certain aspects, R⁶ is - NHR^{W}, wherein R^{W} is a nitrogen protecting group. In certain aspects, R⁶ is -N(R^{W})₂, wherein each R^{W} is a nitrogen protecting group. In certain aspects, R⁶ is selected from the group consisting of -N₃, -NH(Cbz), - NH(Boc), -NH(Fmoc), -NHC(O)CCl₃, -NHC(O)CH₃, and -N(C(O)CH₃)₂. In certain aspects, R⁶ is -NH(Cbz). In certain aspects, R⁶ is -NH(Fmoc). In certain aspects, R⁶ is - NHC(O)CCl₃. In certain aspects, R⁶ is -NHC(O)CH₃. In certain aspects, R⁶ is - N(C(O)CH₃)₂.

In some aspects, R¹, R² and R³ are the same. In some aspects, R¹, R² and R³ are - OH. In some aspects, R⁴, R⁵ and R⁶ are the same. In some aspects, R⁴, R⁵ and R⁶ are -OH.

In certain aspects, L is -OH.

In certain aspects, L is -OH and R¹ is -N₃. In certain aspects, L is -OH, R¹ is -N₃, and each instance of R², R³, R⁴, R⁵ and R⁶ is -OH.

In certain aspects, L is -OH and R² is -N₃. In certain aspects, L is -OH, R² is -N₃, and each instance of R¹, R³, R⁴, R⁵ and R⁶ is -OH.

In certain aspects, L is -OH and R³ is -N₃. In certain aspects, L is -OH, R³ is -N₃, and each instance of R¹, R², R⁴, R⁵ and R⁶ is -OH.

In certain aspects, L is -OH and R⁴ is -N₃. In certain aspects, L is -OH, R⁴ is -N₃, and each instance of R¹, R², R³,R⁵ and R⁶ is -OH.

In certain aspects, L is -OH and R⁵ is -N₃. In certain aspects, L is -OH, R⁵ is -N₃, and each instance of R¹, R², R³, R⁴ and R⁶ is -OH.

In certain aspects, L is -OH and R⁶ is -N₃. In certain aspects, L is -OH, R⁶ is -N₃, and each instance of R¹, R², R³, R⁴ and R⁵ is -OH.

In certain aspects, each instance of R¹, R², R³, R⁴, R⁵, R⁶ and L is -F. In certain aspects, R¹ is -F. In certain aspects, R² is -F. In certain aspects, R³ is -F. In certain aspects, R⁴ is -F. In certain aspects, R⁵ is -F. In certain aspects, R⁶ is -F. In certain aspects, L is -F.

In certain aspects, L is of the following structure: 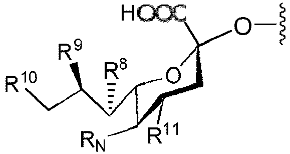 wherein:
each instance of R⁸, R⁹, R¹⁰ and R¹¹ is independently selected from hydrogen, halogen, optionally substituted alkyl, optionally substituted alkenyl, optionally substituted alkynyl, optionally substituted heterocyclyl, optionally substituted aryl, -N₃, -NO₂, -N(R^{B})₂, - N(R^{A})C(O)R^{A}, -OR^{A}, -OC(O)R^{A}, -SR^{A}, -C(O)N(R^{B})₂, -CN, -C(O)R^{A}, -C(O)OR^{A}, -S(O)R^{A}, - SO₂R^{A}, -SO₂N(R^{b})₂, and -NHSO₂R^{B};
R_{N} is selected from -N₃, -NO₂, -N(R^{B})₂, -N(R^{A})C(O)R^{A}, -OR^{A}, -OC(O)R^{A}, -SR^{A}, - C(O)N(R^{B})₂, -CN, -C(O)R^{A}, -C(O)OR^{A}, -S(O)R^{A}, -SO₂R^{A}, -SO₂N(R^{b})₂, and -NHSO₂R^{B};
each instance of R^{A} is independently selected from hydrogen, optionally substituted alkyl, optionally substituted alkenyl, optionally substituted alkynyl, optionally substituted heterocyclyl, and optionally substituted aryl; and
each instance of R^{B} is independently selected from hydrogen, optionally substituted alkyl, optionally substituted alkenyl, optionally substituted alkynyl, optionally substituted heterocyclyl, and optionally substituted aryl.

In some aspects, the compound is of Formula (**II**) 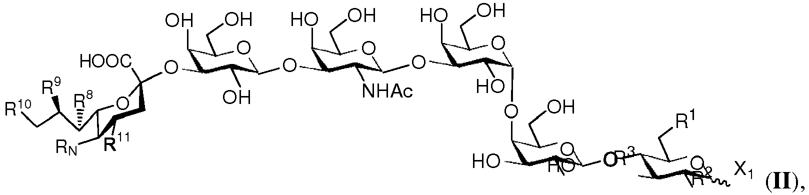 wherein: R¹, R², R³, R⁸, R⁹, R¹⁰, R¹¹ and R_{N} and X₁ are as described herein, and provided the compound is not of the formula:

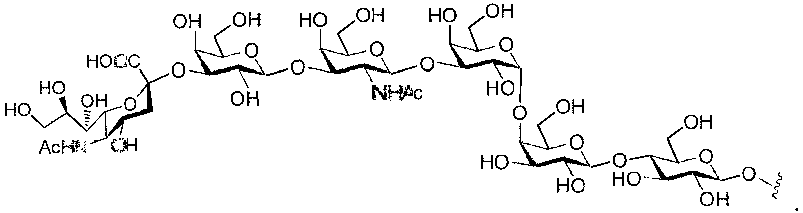

In some aspects, R⁸ is -N₃ or -N(R^{W})₂, wherein each R^{W} is independently hydrogen or a nitrogen protecting group. In certain aspects, R⁸ is -N₃. In certain aspects, R⁸ is -N(R^{W})₂, wherein each R^{W} is independently hydrogen or a nitrogen protecting group. In certain aspects, R⁸ is -NH₂. In certain aspects, R⁸ is - NHR^{W}, wherein R^{W} is a nitrogen protecting group. In certain aspects, R⁸ is -N(R^{W})₂, wherein each R^{W} is a nitrogen protecting group. In certain aspects, R⁸ is selected from
the group consisting of -N₃, -NH(Cbz), -NH(Boc), -NH(Fmoc), -NHC(O)CCl₃, - NHC(O)CH₃, and -N(C(O)CH₃)₂. In certain aspects, R⁸ is -NH(Cbz). In certain aspects, R⁸ is -NH(Fmoc). In certain aspects, R⁸ is -NHC(O)CCl₃. In certain aspects, R⁸ is - NHC(O)CH₃. In certain aspects, R⁸ is -N(C(O)CH₃)₂.

In some aspects, R⁹ is -N₃ or -N(R^{W})₂, wherein each R^{W} is independently hydrogen or a nitrogen protecting group. In certain aspects, R⁹ is -N₃. In certain aspects, R⁹ is - N(R^{W})₂, wherein each R^{W} is independently hydrogen or a nitrogen protecting group. In certain aspects, R⁹ is -NH₂. In certain aspects, R⁹ is - NHR^{W}, wherein R^{W} is a nitrogen protecting group. In certain aspects, R⁹ is -N(R^{W})₂, wherein each R^{W} is a nitrogen protecting group. In certain aspects, R⁹ is selected from the group consisting of -N₃, -NH(Cbz), - NH(Boc), -NH(Fmoc), -NHC(O)CCl₃, -NHC(O)CH₃, and -N(C(O)CH₃)₂. In certain aspects, R⁹ is -NH(Cbz). In certain aspects, R⁹ is -NH(Fmoc). In certain aspects, R⁹ is - NHC(O)CCl₃. In certain aspects, R⁹ is -NHC(O)CH₃. In certain aspects, R⁹ is - N(C(O)CH₃)₂.

In some aspects, R¹⁰ is -N₃ or -N(R^{W})₂, wherein each R^{W} is independently hydrogen or a nitrogen protecting group. In certain aspects, R¹⁰ is -N₃. In certain aspects, R¹⁰ is - N(R^{W})₂, wherein each R^{W} is independently hydrogen or a nitrogen protecting group. In certain aspects, R¹⁰ is -NH₂. In certain aspects, R¹⁰ is - NHR^{W}, wherein R^{W} is a nitrogen protecting group. In certain aspects, R¹⁰ is -N(R^{W})₂, wherein each R^{W} is a nitrogen protecting group. In certain aspects, R¹⁰ is selected from the group consisting of -N₃, -NH(Cbz), - NH(Boc), -NH(Fmoc), -NHC(O)CCl₃, -NHC(O)CH₃, and -N(C(O)CH₃)₂. In certain aspects, R¹⁰ is -NH(Cbz). In certain aspects, R¹⁰ is -NH(Fmoc). In certain aspects, R¹⁰ is - NHC(O)CCl₃. In certain aspects, R¹⁰ is -NHC(O)CH₃. In certain aspects, R¹⁰ is - N(C(O)CH₃)₂.

In some aspects, R¹¹ is -N₃ or -N(R^{W})₂, wherein each R^{W} is independently hydrogen or a nitrogen protecting group. In certain aspects, R¹¹ is -N₃. In certain aspects, R¹¹ is -N(R^{W})₂, wherein each R^{W} is independently hydrogen or a nitrogen protecting group. In certain aspects, R¹¹ is -NH₂. In certain aspects, R¹¹ is - NHR^{W}, wherein R^{W} is a nitrogen protecting group. In certain aspects, R¹¹ is -N(R^{W})₂, wherein each R^{W} is a nitrogen protecting group. In certain aspects, R¹¹ is selected from the group consisting of - N₃, -NH(Cbz), -NH(Boc), -NH(Fmoc), -NHC(O)CCl₃, -NHC(O)CH₃, and -N(C(O)CH₃)₂. In certain aspects, R¹¹ is -NH(Cbz). In certain aspects, R¹¹ is -NH(Fmoc). In certain aspects, R¹¹ is -NHC(O)CCl₃. In certain aspects, R¹¹ is -NHC(O)CH₃. In certain aspects, R¹¹ is - N(C(O)CH₃)₂.

In some aspects, R¹² is -N₃ or -N(R^{W})₂, wherein each R^{W} is independently hydrogen or a nitrogen protecting group. In certain aspects, R¹² is -N₃. In certain aspects, R¹² is - N(R^{W})₂, wherein each R^{W} is independently hydrogen or a nitrogen protecting group. In certain aspects, R¹² is -NH₂. In certain aspects, R¹² is - NHR^{W}, wherein R^{W} is a nitrogen protecting group. In certain aspects, R¹² is -N(R^{W})₂, wherein each R^{W} is a nitrogen protecting group. In certain aspects, R¹² is selected from the group consisting of -N₃, -NH(Cbz), - NH(Boc), -NH(Fmoc), -NHC(O)CCl₃, -NHC(O)CH₃, and -N(C(O)CH₃)₂. In certain aspects, R¹² is -NH(Cbz). In certain aspects, R¹² is -NH(Fmoc). In certain aspects, R¹² is - NHC(O)CCl₃. In certain aspects, R¹² is -NHC(O)CH₃. In certain aspects, R¹² is - N(C(O)CH₃)₂.

In some aspects, R_{N} is -N₃ or -N(R^{W})₂, wherein each R^{W} is independently hydrogen or a nitrogen protecting group. In certain aspects, R_{N} is -N₃. In certain aspects, R_{N} is - N(R^{W})₂, wherein each R^{W} is independently hydrogen or a nitrogen protecting group. In certain aspects, R_{N} is -NH₂. In certain aspects, R_{N} is - NHR^{W}, wherein R^{W} is a nitrogen protecting group. In certain aspects, R_{N} is -N(R^{W})₂, wherein each R^{W} is a nitrogen protecting group. In certain aspects, R_{N} is selected from the group consisting of -N₃, -NH(Cbz), - NH(Boc), -NH(Fmoc), -NHC(O)CCl₃, -NHC(O)CH₃, and -N(C(O)CH₃)₂. In certain aspects, R_{N} is -NH(Cbz). In certain aspects, R_{N} is -NH(Fmoc). In certain aspects, R_{N} is - NHC(O)CCl₃. In certain aspects, R_{N} is -NHC(O)CH₃. In certain aspects, R_{N} is - N(C(O)CH₃)₂.

### Immunogenic Compositions

In another aspect, the present disclosure provides an immunogenic composition, comprising (a) a glycan conjugate including a carrier and one or more glycans, and optionally (b) an adjuvant,
wherein: each of the one or more glycans is conjugated with the carrier through a linker, having the formula (III) or (IV): 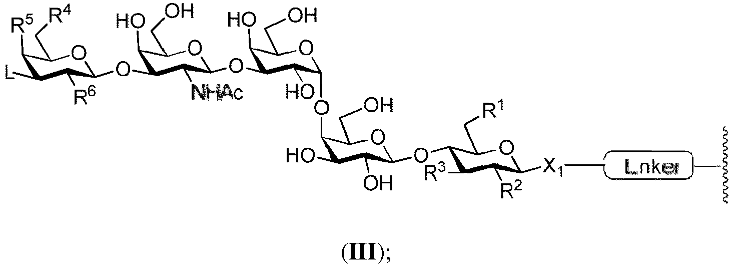 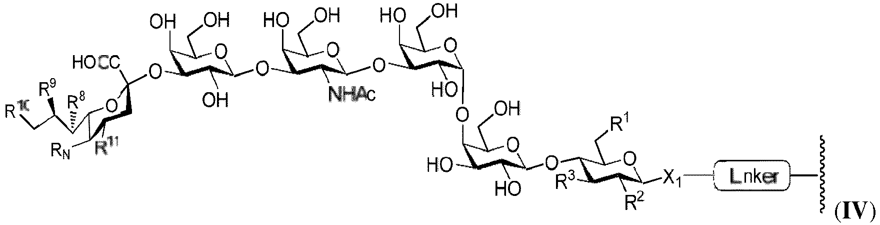 wherein X₁, R¹, R², R³, R⁴, R⁵, R⁶, R⁸, R⁹, R¹⁰, R¹¹, L and R_{N} are as described herein.

In certain aspects, the linker is a hetero- or homo-bifunctional linker.

In certain aspects, the linker includes at least one sulfur atom, carboxylate group, amide group, carbamate group, carbonate group, thiocarbamate group, thiocarbonate group, thioether group, succinamide group, n-hydroxy succinamide group, or any combination thereof.

In certain aspects, the linker is, -L¹-L²- wherein L¹ is a bond, -O-, -S-, - NR^{L1a}-, -C(=O)-, -NR ^{L1a}C(=O)-, -NR ^{L1a}C(=O)O-, -C(=O)NR^{L1a}-, -OC(=O)NR^{L1a}-, -SC(=O)-, -C(=O)S-, -OC(=O)-, -C(=O)O-, -NR^{L1a}C(=S)-, -C(=S)NR^{L1a}-, *trans-* CR^{L1b} =CR^{L1b}-, cis-CR^{L1b}-CR^{L1b}-, -C≡C-, -OC(R^{L1b})₂-, -C(R^{L1b} )₂O-, -NR^{L1A}C(R^{L1b})₂-, - C(R^{L1b})₂NR^{L1a}- -SC(R^{L1b})₂- -C(R^{L1b})₂S-, -S(=O)₂O- -OS(=O)₂-, -S(=O)₂NR^{L1a}-, - NR^{L1a}S(-O)₂-, or an optionally substituted C₁₋₂₀ hydrocarbon chain, optionally wherein one or more carbon units of the hydrocarbon chain is replaced with -O-, -S-, -NR^{L1a}-, -C(-O)-, NR^{L1a}C(=O)-, -NR^{L1a}C(=O)O-, -C(=O)NR^{L1a}-, -OC(=O)NR^{L1a}-, -SC(=O)-, -C(=O)S-, - OC(=O)-, -C(=O)O-, -NR^{L1a}C(=S)-, -C(=S)NR^{L1a}-, *trans*-CR^{L1b}=CR^{L1b}-, *cis-*CR^{L1b}=CR^{L1b}-, -C≡C-, -S(=O)₂O-, -OS(=O)₂-, -S(=O)₂NR^{L1a}-, or -NR^{L1a}S(-O)₂-, wherein R^{L1a} is hydrogen, optionally substituted C₁₋₆ alkyl, or a nitrogen protecting group, or R^{L1a} is joined with the adjacent carbon atom to form an optionally substituted heterocyclic ring, and wherein each occurrence of R^{L1b} is independently selected from the group consisting of hydrogen, halogen, optionally substituted C₁₋₁₀ alkyl, optionally substituted alkenyl, optionally substituted alkynyl, optionally substituted carbocyclyl, optionally substituted heterocyclyl, optionally substituted aryl, and optionally substituted heteroaryl, or R^{L1b} is joined with the adjacent carbon or nitrogen or oxygen atom to form an optionally substituted carbocyclic or heterocyclic ring, or two R^{L1b} groups are joined to form an optionally substituted carbocyclic or optionally substituted heterocyclic ring; and L² is a moiety derived from a crosslinking reagent capable of crosslinking the carrier and L¹.

The carrier can be a protein, a lipid, a lipolized protein, a virus, a peptide, or a dendrimer of glycopeptides. In certain aspects, the carrier is a peptide comprising a T cell epitope.

Examples of carrier proteins which may be used in the present disclosure are tetanus toxoid (TT), diphtheria toxoid (DT), diphtheria toxin cross-reacting material 197 (CRM197), fragment C of TT, Keyhole limpet hemocyanin (KLH), bovine serum albumin (BSA), protein D, outer-membrane protein (OMP) and pneumolysin, diphtheria toxin cross-reacting material 197 (CRM197) or other DT point mutants, such as CRM176, CRM228, CRM 45 (Uchida et al J. Biol. Chem. 218; 3838-3844, 1973); CRM 9, CRM 45, CRM102, CRM 103 and CRM107 and other mutations described in the art.

In certain aspects, the glycan conjugate is of the formula (IV-a) or (IV-b): 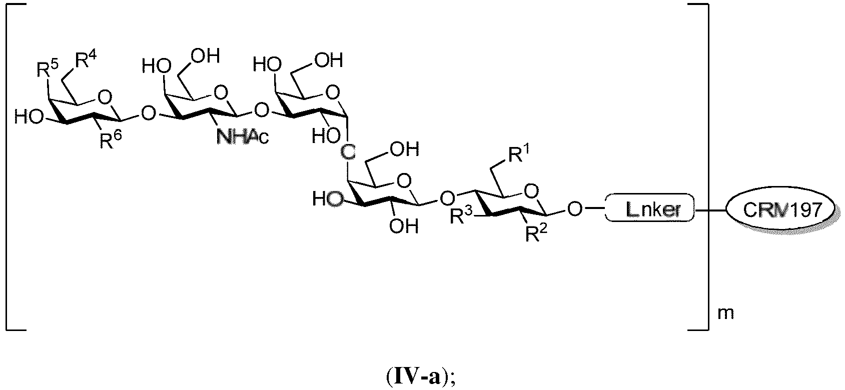 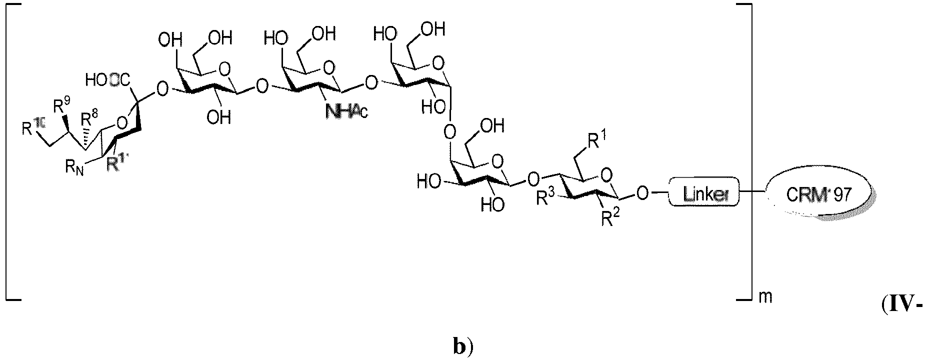 wherein m is an integer of 1 to 40, inclusive.

In certain aspects, m is an integer of 1 to 30, inclusive. As generally defined herein, m is an integer of 1 to 20 inclusive. In certain aspects, m is 1. In certain aspects, m is 2. In certain aspects, m is 4. In certain aspects, m is 6. In certain aspects, m is 8. In certain aspects, m is 10. In certain aspects, m is 15. In certain aspects, m is 20. In certain aspects, m is 30. In certain aspects, m is 40.

In another aspect, the present disclosure provides a glycan conjugate mixture comprising at least two of the glycan conjugates as described herein. In certain aspects, the average value of w in the glycan mixture is from about 1.0 to about 40.0. In certain aspects, the average value of w in the glycan mixture is from about 1.0 to 10.0. In certain aspects, the average value of w in the glycan mixture is about 5.7, 4.9, 2.9, 2.8, or 3.1. In certain aspects, the average value of w in the glycan mixture is about 4.9, 2.9, 2.8, or 3.1.

In certain aspects, the immmunogenic compositions described herein include an immmunogenically effective amount of a glycan conjugate disclosed herein.

The compounds disclosed herein can be synthesized using procedures known in the art or described herein. Also see US20140051127.

The immunogenic conjugate disclosed herein may include one or more molecules (e.g., 1-40, 1-20, 1-25, 1-30, 5-20, 5-25, 5-30, or 5-35) of the same or different SSEA-33
and/or SSEA-4 derivatives. Procedures for generating glycan conjugates are known in the art and described below. Also see US Patent No. 8,268,969.

In certain aspects, the immmunogenic composition disclosed herein may include one or more adjuvants. Suitable adjuvants are known in the art (e.g., C34, 7DW8-5, C17, C23, Gluco-C34, Aluminum salt, Squalene, MF59, and QS-21).

As used herein, the term "alum adjuvant" refers to an aluminum salt with immune adjuvant activity. This agent adsorbs and precipitates protein antigens in solution; the resulting precipitate improves vaccine immunogenicity by facilitating the slow release of antigen from the vaccine depot formed at the site of inoculation.

As used herein, the term "immunologic adjuvant" refers to a substance used in conjunction with an immunogen which enhances or modifies the immune response to the immunogen. The α-GalCer analogs of the present disclosure are used as immunologic adjuvants to modify or augment the effects of a vaccine by stimulating the immune system of a patient who is administered the vaccine to respond to the vaccine more vigorously. In an exemplary implementation, the analog C34 is used as an adjuvant. The structures of C34 and other alpha-galactosyl ceramide analogs and their use as adjuvants are disclosed in detail in US patent No. 7,928,077.

As used herein, the term "glycolipid" refers to a carbohydrate-attached lipid that serves as a marker for cellular recognition.

The glycolipids C34, Gluco-C34, C23 and 7DW8-5 have the following structures:

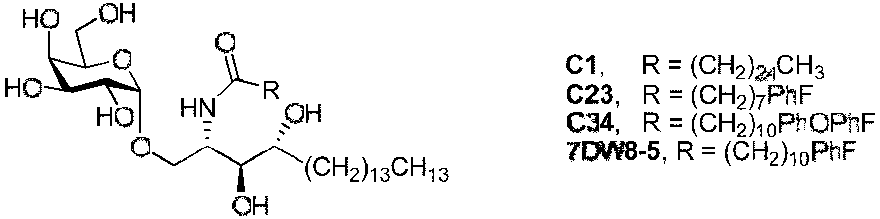

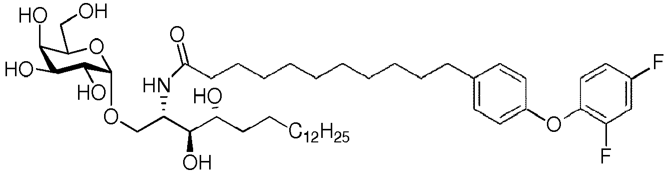

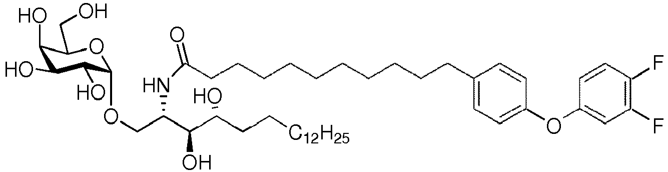

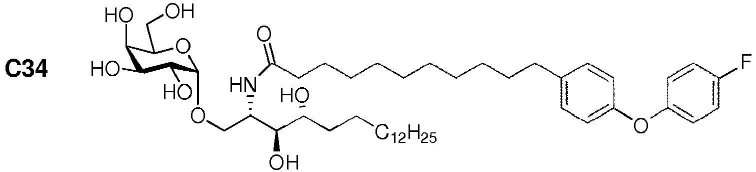

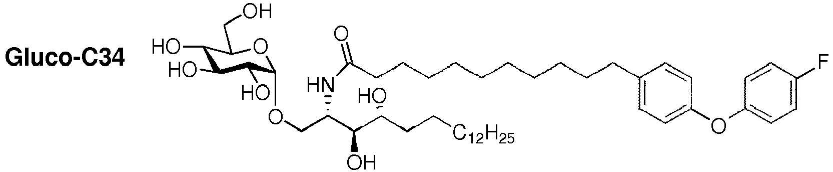

The immunogenic composition can further include a pharmaceutically acceptable excipient. In certain aspects, the immmunogenic compositions described herein include an pharmaceutically effective amount of a glycan conjugate disclosed herein.

In another aspect, the present disclosure provides a cancer vaccine comprising an immmunogenic composition described herein and a pharmaceutically acceptable excipient.

The cancer vaccines disclosed herein may include a single dose or multiple doses of the inventive glycan conjugates, a glycan conjugate mixture thereof, or immmunogenic compositions thereof. The provided cancer vaccines may be useful for treating or reducing the risk of cancers. The cancer vaccines may also include packaging information describing the use or prescribing information for the subject or a health care professional. Such information may be required by a regulatory agency such as the U.S. Food and Drug Administration (FDA). The cancer vaccine may also optionally include a device for administration of the compound or composition, for example, a syringe for parenteral administration.

### Pharmaceutical Formulations

The immune composition is administered in a manner compatible with the dosage formulation, and in an amount that is therapeutically effective, protective and immunogenic. The quantity to be administered depends on the subject to be treated, including, for example, the capacity of the individual's immune system to synthesize antibodies, and if needed, to produce a cell-mediated immune response. Precise amounts of active ingredient required to be administered depend on the judgment of the practitioner. However, suitable dosage ranges are readily determinable by one skilled in the art. Suitable regimes for initial administration and booster doses are also variable, but may include an initial administration followed by subsequent administrations. The dosage of the vaccine may also depend on the route of administration and varies according to the size of the host.

The immune composition disclosed herein can also be used to generate antibodies in animals for production of antibodies, which can be used in both cancer treatment and diagnosis. Methods of making monoclonal and polyclonal antibodies and fragments thereof in animals (e.g., mouse, rabbit, goat, sheep, or horse) are well known in the art. See, for example, Harlow and Lane, (1988) Antibodies: A Laboratory Manual, Cold Spring Harbor Laboratory, New York. The term "antibody" includes intact immunoglobulin molecules as well as fragments thereof, such as Fab, F(ab')₂, Fv, scFv (single chain antibody), and dAb (domain antibody; Ward, et. al. (1989) Nature, 341, 544).

The compositions disclosed herein can be included in a pharmaceutical composition together with additional active agents, carriers, vehicles, excipients, or auxiliary agents identifiable by a person skilled in the art upon reading of the present disclosure.

The pharmaceutical compositions preferably comprise at least one pharmaceutically acceptable carrier. In such pharmaceutical compositions, the compositions disclosed herein form the "active compound," also referred to as the "active agent." As used herein the language "pharmaceutically acceptable carrier" includes solvents, dispersion media, coatings, antibacterial and antifungal agents, isotonic and absorption delaying agents, and the like, compatible with pharmaceutical administration. Supplementary active compounds can also be incorporated into the compositions. A pharmaceutical composition is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol, or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates, or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes, or multiple dose vials made of glass or plastic.

### Clinical Applications

The present disclosure provides glycan conjugates, immunogenic compositions or vaccines useful for the treatment of a proliferative disease such as cancer (*e.g.* lung cancer, large bowel cancer, pancreas cancer, biliary tract cancer, or endometrial cancer), benign neoplasm, or angiogenesis in a subject.

The immunogenic compositions or vaccines described herein can also be used to generate antibodies in human or animals for production of antibodies, which can be used in both cancer treatment and diagnosis. In some aspects, the immunogenic compositions or vaccines described herein can also be used to generate antibodies for production of Globo H, SSEA-3 and/or SSEA-4 antibodies. Methods of making monoclonal and polyclonal antibodies and fragments thereof in human and/or animals (e.g., mouse, rabbit, goat, sheep, or horse) are well known in the art. See, for example, Harlow and Lane, (1988) Antibodies: A Laboratory Manual, Cold Spring Harbor Laboratory, New York. The term "antibody" includes intact immunoglobulin molecules as well as fragments thereof, such as Fab, F(ab').sub.2, Fv, scFv (single chain antibody), and dAb (domain antibody; Ward, et. al. (1989) Nature, 341, 544).

Compositions comprising at least one anti-SSEA-3/SSEA-4/GLOBO H antibody or at least one polynucleotide comprising sequences encoding an anti-SSEA-3/SSEA-4/GLOBO H antibody are provided. In certain aspects, a composition may be a pharmaceutical composition. As used herein, compositions comprise one or more antibodies that bind to one or more SSEA-3/SSEA-4/GLOBO H and/or one or more polynucleotides comprising sequences encoding one or more antibodies that bind to one or more SSEA-3/SSEA-4/GLOBO H. These compositions may further comprise suitable carriers, such as pharmaceutically acceptable excipients including buffers, which are well known in the art.

Isolated antibodies and polynucleotides are also provided. In certain aspects, the isolated antibodies and polynucleotides are substantially pure.

In one aspect, anti-SSEA-3/SSEA-4/GLOBO H antibodies are monoclonal. In another aspect, fragments of the anti-SSEA-3/SSEA-4/GLOBO H antibodies (e.g., Fab, Fab'-SH and F(ab')2 fragments) are provided. These antibody fragments can be created by traditional means, such as enzymatic digestion, or may be generated by recombinant techniques. Such antibody fragments may be chimeric, humanized, or human. These fragments are useful for the diagnostic and therapeutic purposes set forth below.

Example of Generation of Anti-SSEA-3/SSEA-4/GLOBO H Antibodies Using a Phage Display Library

A variety of methods are known in the art for generating phage display libraries from which an antibody of interest can be obtained. One method of generating antibodies of interest is through the use of a phage antibody library as described in Lee et al., J. Mol. Biol. (2004), 340(5): 1073-93.

The anti-SSEA-3/SSEA-4/GLOBO H antibodies disclosed herein can be made by using combinatorial libraries to screen for synthetic antibody clones with the desired activity or activities. In principle, synthetic antibody clones are selected by screening phage libraries containing phage that display various fragments of antibody variable region (Fv) fused to phage coat protein. Such phage libraries are panned by affinity chromatography against the desired antigen. Clones expressing Fv fragments capable of binding to the desired antigen are adsorbed to the antigen and thus separated from the non-binding clones in the library. The binding clones are then eluted from the antigen, and can be further enriched by additional cycles of antigen adsorption/elution. Any of the anti-SSEA-3/SSEA-4/GLOBO H antibodies disclosed herein can be obtained by designing a suitable antigen screening procedure to select for the phage clone of interest followed by construction of a full length anti-SSEA-3/SSEA-4/GLOBO H antibody clone using the Fv sequences from the phage clone of interest and suitable constant region (Fc) sequences described in Kabat et al., Sequences of Proteins of Immunological Interest, Fifth Edition, NIH Publication 91-3242, Bethesda Md. (1991), vols. 1-3.

The antigen-binding domain of an antibody is formed from two variable (V) regions of about 110 amino acids, one each from the light (VL) and heavy (VH) chains, that both present three hypervariable loops or complementarity-determining regions (CDRs). Variable domains can be displayed functionally on phage, either as single-chain Fv (scFv) fragments, in which VH and VL are covalently linked through a short, flexible peptide, or as Fab fragments, in which they are each fused to a constant domain and interact non-covalently, as described in Winter et al., Ann. Rev. Immunol., 12: 433-455 (1994). As used herein, scFv encoding phage clones and Fab encoding phage clones are collectively referred to as "Fv phage clones" or "Fv clones".

Repertoires of VH and VL genes can be separately cloned by polymerase chain reaction (PCR) and recombined randomly in phage libraries, which can then be searched for antigen-binding clones as described in Winter et al., Ann. Rev. Immunol., 12: 433-455 (1994). Libraries from immunized sources provide high-affinity antibodies to the immunogen without the requirement of constructing hybridomas. Alternatively, the naive repertoire can be cloned to provide a single source of human antibodies to a wide range of non-self and also self antigens without any immunization as described by Griffiths et al., EMBO J, 12: 725-734 (1993). Finally, naive libraries can also be made synthetically by cloning the unrearranged V-gene segments from stem cells, and using PCR primers containing random sequence to encode the highly variable CDR3 regions and to accomplish rearrangement in vitro as described by Hoogenboom and Winter, J. Mol. Biol., 227: 381-388 (1992).

Filamentous phage is used to display antibody fragments by fusion to the minor coat protein pIII. The antibody fragments can be displayed as single chain Fv fragments, in which VH and VL domains are connected on the same polypeptide chain by a flexible polypeptide spacer, e.g. as described by Marks et al., J. Mol. Biol., 222: 581-597 (1991), or as Fab fragments, in which one chain is fused to pIII and the other is secreted into the bacterial host cell periplasm where assembly of a Fab-coat protein structure which becomes displayed on the phage surface by displacing some of the wild type coat proteins, e.g. as described in Hoogenboom et al., Nucl. Acids Res., 19: 4133-4137 (1991).

In general, nucleic acids encoding antibody gene fragments are obtained from immune cells harvested from humans or animals. If a library biased in favor of anti-SSEA-3/SSEA-4/GLOBO H clones is desired, the subject is immunized with SSEA-3/SSEA-4/GLOBO H to generate an antibody response, and spleen cells and/or circulating B cells or other peripheral blood lymphocytes (PBLs) are recovered for library construction. In one aspect, a human antibody gene fragment library biased in favor of anti-human SSEA-3/SSEA-4/GLOBO H clones is obtained by generating an anti-human SSEA-3/SSEA-4/GLOBO H antibody response in transgenic mice carrying a functional human immunoglobulin gene array (and lacking a functional endogenous antibody production system) such that SSEA-3/SSEA-4/GLOBO H immunization gives rise to B cells producing human antibodies against SSEA-3/SSEA-4/GLOBO H. The generation of human antibody-producing transgenic mice is described below.

Additional enrichment for anti-SSEA-3/SSEA-4/GLOBO H reactive cell populations can be obtained by using a suitable screening procedure to isolate B cells expressing SSEA-3/SSEA-4/GLOBO H-specific antibody, e.g., by cell separation with SSEA-3/SSEA-4/GLOBO H affinity chromatography or adsorption of cells to fluorochrome-labeled SSEA-3/SSEA-4/GLOBO H followed by flow-activated cell sorting (FACS).

Alternatively, the use of spleen cells and/or B cells or other PBLs from an unimmunized donor provides a better representation of the possible antibody repertoire, and also permits the construction of an antibody library using any animal (human or non-human) species in which SSEA-3/SSEA-4/GLOBO H is not antigenic. For libraries incorporating in vitro antibody gene construction, stem cells are harvested from the subject to provide nucleic acids encoding unrearranged antibody gene segments. The immune cells of interest can be obtained from a variety of animal species, such as human, mouse, rat, lagomorpha, luprine, canine, feline, porcine, bovine, equine, and avian species, etc.

Nucleic acid encoding antibody variable gene segments (including VH and VL segments) are recovered from the cells of interest and amplified. In the case of rearranged VH and VL gene libraries, the desired DNA can be obtained by isolating genomic DNA or mRNA from lymphocytes followed by polymerase chain reaction (PCR) with primers matching the 5 ' and 3' ends of rearranged VH and VL genes as described in Orlandi et al., Proc. Natl. Acad. Sci. (USA), 86: 3833-3837 (1989), thereby making diverse V gene repertoires for expression. The V genes can be amplified from cDNA and genomic DNA, with back primers at the 5' end of the exon encoding the mature V-domain and forward primers based within the J-segment as described in Orlandi et al. (1989) and in Ward et al., Nature, 341: 544-546 (1989). However, for amplifying from cDNA, back primers can also be based in the leader exon as described in Jones et al., Biotechnol., 9: 88-89 (1991), and forward primers within the constant region as described in Sastry et al., Proc. Natl. Acad. Sci. (USA), 86: 5728-5732 (1989). To maximize complementarity, degeneracy can be incorporated in the primers as described in Orlandi et al. (1989) or Sastry et al. (1989). In certain aspects, the library diversity is maximized by using PCR primers targeted to each V-gene family in order to amplify all available VH and VL arrangements present in the immune cell nucleic acid sample, e.g. as described in the method of Marks et al., J. Mol. Biol., 222: 581-597 (1991) or as described in the method of Orum et al., Nucleic Acids Res., 21: 4491-4498 (1993). For cloning of the amplified DNA into expression vectors, rare restriction sites can be introduced within the PCR primer as a tag at one end as described in Orlandi et al. (1989), or by further PCR amplification with a tagged primer as described in Clackson et al., Nature, 352: 624-628 (1991).

Repertoires of synthetically rearranged V genes can be derived in vitro from V gene segments. Most of the human VH-gene segments have been cloned and sequenced (reported in Tomlinson et al., J. Mol. Biol., 227: 776-798 (1992)), and mapped (reported in Matsuda et al., Nature Genet., 3: 88-94 (1993); these cloned segments (including all the major conformations of the H1 and H2 loop) can be used to generate diverse VH gene repertoires with PCR primers encoding H3 loops of diverse sequence and length as described in Hoogenboom and Winter, J. Mol. Biol., 227: 381-388 (1992). VH repertoires can also be made with all the sequence diversity focused in a long H3 loop of a single length as described in Barbas et al., Proc. Natl. Acad. Sci. USA, 89: 4457-4461 (1992). Human Vκ and Vλ segments have been cloned and sequenced (reported in Williams and Winter, Eur. J. Immunol., 23: 1456-1461 (1993)) and can be used to make synthetic light chain repertoires. Synthetic V gene repertoires, based on a range of VH and VL folds, and L3 and H3 lengths, will encode antibodies of considerable structural diversity. Following amplification of V-gene encoding DNAs, germline V-gene segments can be rearranged in vitro according to the methods of Hoogenboom and Winter, J. Mol. Biol., 227: 381-388 (1992).

Repertoires of antibody fragments can be constructed by combining VH and VL gene repertoires together in several ways. Each repertoire can be created in different vectors, and the vectors recombined in vitro, e.g., as described in Hogrefe et al., Gene, 128: 119-126 (1993), or in vivo by combinatorial infection, e.g., the loxP system described in Waterhouse et al., Nucl. Acids Res., 21: 2265-2266 (1993). The in vivo recombination approach exploits the two-chain nature of Fab fragments to overcome the limit on library size imposed by E. coli transformation efficiency. Naive VH and VL repertoires are cloned separately, one into a phagemid and the other into a phage vector. The two libraries are then combined by phage infection of phagemid-containing bacteria so that each cell contains a different combination and the library size is limited only by the number of cells present (about 1012 clones). Both vectors contain in vivo recombination signals so that the VH and VL genes are recombined onto a single replicon and are co-packaged into phage virions. These huge libraries provide large numbers of diverse antibodies of good affinity (Kd -1 of about 10-8 M).

Alternatively, the repertoires may be cloned sequentially into the same vector, e.g. as described in Barbas et al., Proc. Natl. Acad. Sci. USA, 88: 7978-7982 (1991), or assembled together by PCR and then cloned, e.g. as described in Clackson et al., Nature, 352: 624-628 (1991). PCR assembly can also be used to join VH and VL DNAs with DNA encoding a flexible peptide spacer to form single chain Fv (scFv) repertoires. In yet another technique, "in cell PCR assembly" is used to combine VH and VL genes within lymphocytes by PCR and then clone repertoires of linked genes as described in Embleton et al., Nucl. Acids Res., 20: 3831-3837 (1992).

Screening of the libraries can be accomplished by any art-known technique. For example, SSEA-3/SSEA-4/GLOBO H targets can be used to coat the wells of adsorption plates, expressed on host cells affixed to adsorption plates or used in cell sorting, or conjugated to biotin for capture with streptavidin-coated beads, or used in any other art-known method for panning phage display libraries.

The phage library samples are contacted with immobilized SSEA-3/SSEA-4/GLOBO H under conditions suitable for binding of at least a portion of the phage particles with the adsorbent. Normally, the conditions, including pH, ionic strength, temperature and the like are selected to mimic physiological conditions. The phages bound to the solid phase are washed and then eluted by acid, e.g. as described in Barbas et al., Proc. Natl. Acad. Sci. USA, 88: 7978-7982 (1991), or by alkali, e.g. as described in Marks et al., J. Mol. Biol., 222: 581-597 (1991), or by SSEA-3/SSEA-4/GLOBO H antigen competition, e.g. in a procedure similar to the antigen competition method of Clackson et al., Nature, 352: 624-628 (1991). Phages can be enriched 20-1,000-fold in a single round of selection. Moreover, the enriched phages can be grown in bacterial culture and subjected to further rounds of selection.

The efficiency of selection depends on many factors, including the kinetics of dissociation during washing, and whether multiple antibody fragments on a single phage can simultaneously engage with antigen. Antibodies with fast dissociation kinetics (and weak binding affinities) can be retained by use of short washes, multivalent phage display and high coating density of antigen in solid phase. The high density not only stabilizes the phage through multivalent interactions, but favors rebinding of phage that has dissociated. The selection of antibodies with slow dissociation kinetics (and good binding affinities) can be promoted by use of long washes and monovalent phage display as described in Bass et al., Proteins, 8: 309-314 (1990) and in WO 92/09690, and a low coating density of antigen as described in Marks et al., Biotechnol., 10: 779-783 (1992).

It is possible to select between phage antibodies of different affinities, even with affinities that differ slightly, for SSEA-3/SSEA-4/GLOBO H. However, random mutation of a selected antibody (e.g. as performed in some of the affinity maturation techniques described above) is likely to give rise to many mutants, most binding to antigen, and a few with higher affinity. With limiting SSEA-3/SSEA-4/GLOBO H, rare high affinity phage could be competed out. To retain all the higher affinity mutants, phages can be incubated with excess biotinylated SSEA-3/SSEA-4/GLOBO H, but with the biotinylated SSEA-3/SSEA-4/GLOBO H at a concentration of lower molarity than the target molar affinity constant for SSEA-3/SSEA-4/GLOBO H. The high affinity-binding phages can then be captured by streptavidin-coated paramagnetic beads. Such "equilibrium capture" allows the antibodies to be selected according to their affinities of binding, with sensitivity that permits isolation of mutant clones with as little as two-fold higher affinity from a great excess of phages with lower affinity. Conditions used in washing phages bound to a solid phase can also be manipulated to discriminate on the basis of dissociation kinetics.

Anti-SSEA-3/SSEA-4/GLOBO H clones may be activity selected. In one aspect, the disclosure provides anti-SSEA-3/SSEA-4/GLOBO H antibodies that block the binding between a SSEA-3/SSEA-4/GLOBO H ligand and SSEA-3/SSEA-4/GLOBO H, but do not block the binding between a SSEA-3/SSEA-4/GLOBO H ligand and a second protein. Fv clones corresponding to such anti-SSEA-3/SSEA-4/GLOBO H antibodies can be selected by (1) isolating anti-SSEA-3/SSEA-4/GLOBO H clones from a phage library as described in Section B(I)(2) above, and optionally amplifying the isolated population of phage clones by growing up the population in a suitable bacterial host; (2) selecting SSEA-3/SSEA-4/GLOBO H and a second protein against which blocking and non-blocking activity, respectively, is desired; (3) adsorbing the anti-SSEA-3/SSEA-4/GLOBO H phage clones to immobilized SSEA-3/SSEA-4/GLOBO H; (4) using an excess of the second protein to elute any undesired clones that recognize SSEA-3/SSEA-4/GLOBO H-binding determinants which overlap or are shared with the binding determinants of the second protein; and (5) eluting the clones which remain adsorbed following step (4). Optionally, clones with the desired blocking/non-blocking properties can be further enriched by repeating the selection procedures described herein one or more times.

DNA encoding the Fv clones of the invention is readily isolated and sequenced using conventional procedures (e.g. by using oligonucleotide primers designed to specifically amplify the heavy and light chain coding regions of interest from hybridoma or phage DNA template). Once isolated, the DNA can be placed into expression vectors, which are then transfected into host cells such as E. coli cells, simian COS cells, Chinese hamster ovary (CHO) cells, or myeloma cells that do not otherwise produce immunoglobulin protein, to obtain the synthesis of the desired monoclonal antibodies in the recombinant host cells. Review articles on recombinant expression in bacteria of antibody-encoding DNA include Skerra et al., Curr. Opinion in Immunol., 5: 256 (1993) and Pluckthun, Immunol. Revs, 130: 151 (1992).

DNA encoding the Fv clones disclosed herein can be combined with known DNA sequences encoding heavy chain and/or light chain constant regions (e.g. the appropriate DNA sequences can be obtained from Kabat et al., supra) to form clones encoding full or partial length heavy and/or light chains. It will be appreciated that constant regions of any isotype can be used for this purpose, including IgG, IgM, IgA, IgD, and IgE constant regions, and that such constant regions can be obtained from any human or animal species. A Fv clone derived from the variable domain DNA of one animal (such as human) species and then fused to constant region DNA of another animal species to form coding sequence(s) for "hybrid", full length heavy chain and/or light chain is included in the definition of "chimeric" and "hybrid" antibody as used herein. In one aspect, a Fv clone derived from human variable DNA is fused to human constant region DNA to form coding sequence(s) for all human, full or partial length heavy and/or light chains.

The antibodies produced by naive libraries (either natural or synthetic) can be of moderate affinity (Kd -1 of about 106 to 107 M-1), but affinity maturation can also be mimicked in vitro by constructing and reselecting from secondary libraries as described in Winter et al. (1994), supra. For example, mutation can be introduced at random in vitro by using error-prone polymerase (reported in Leung et al., Technique, 1: 11-15 (1989)) in the method of Hawkins et al., J. Mol. Biol., 226: 889-896 (1992) or in the method of Gram et al., Proc. Natl. Acad. Sci. USA, 89: 3576-3580 (1992). Additionally, affinity maturation can be performed by randomly mutating one or more CDRs, e.g. using PCR with primers carrying random sequence spanning the CDR of interest, in selected individual Fv clones and screening for higher affinity clones. WO 9607754 (published 14 Mar. 1996) described a method for inducing mutagenesis in a complementarity determining region of an immunoglobulin light chain to create a library of light chain genes. Another effective approach is to recombine the VH or VL domains selected by phage display with repertoires of naturally occurring V domain variants obtained from unimmunized donors and screen for higher affinity in several rounds of chain reshuffling as described in Marks et al., Biotechnol., 10: 779-783 (1992). This technique allows the production of antibodies and antibody fragments with affinities in the 10-9 M range.

Other Methods of Generating Anti-SSEA-3/SSEA-4/GLOBO H Antibodies

Other methods of generating and assessing the affinity of antibodies are well known in the art and are described, e.g., in Kohler et al., Nature 256: 495 (1975); U.S. Pat. No. 4,816,567; Goding, Monoclonal Antibodies: Principles and Practice, pp. 59-103 (Academic Press, 1986; Kozbor, J. Immunol., 133:3001 (1984); Brodeur et al., Monoclonal Antibody Production Techniques and Applications, pp. 51-63 (Marcel Dekker, Inc., New York, 1987; Munson et al., Anal. Biochem., 107:220 (1980); Engels et al., Agnew. Chem. Int. Ed. Engl., 28: 716-734 (1989); Abrahmsen et al., EMBO J., 4: 3901 (1985); Methods in Enzymology, vol. 44 (1976); Morrison et al., Proc. Natl. Acad. Sci. USA, 81: 6851-6855 (1984).

### General Methods

In general, the disclosure provides affinity-matured SSEA-3/SSEA-4/GLOBO H antibodies. These antibodies have increased affinity and specificity for SSEA-3/SSEA-4/GLOBO H. This increase in affinity and sensitivity permits the molecules disclosed herein to be used for applications and methods that are benefited by (a) the increased sensitivity of the molecules disclosed herein and/or (b) the tight binding of SSEA-3/SSEA-4/GLOBO H by the molecules disclosed herein.

In one aspect, SSEA-3/SSEA-4/GLOBO H antibodies that are useful for treatment of SSEA-3/SSEA-4/GLOBO H-mediated disorders in which a partial or total blockade of one or more SSEA-3/SSEA-4/GLOBO H activities is desired. In one aspect, the anti SSEA-3/SSEA-4/GLOBO H antibodies disclosed herein are used to treat cancer.

The anti- SSEA-3/SSEA-4/GLOBO H antibodies disclosed herein permit the sensitive and specific detection of the epitopes in straightforward and routine biomolecular assays such as immunoprecipitations, ELISAs, or immunomicroscopy without the need for mass spectrometry or genetic manipulation. In turn, this provides a significant advantage in both observing and elucidating the normal functioning of these pathways and in detecting when the pathways are functioning aberrantly.

The SSEA-3/SSEA-4/GLOBO H antibodies disclosed herein can also be used to determine the role in the development and pathogenesis of disease. For example, as described above, the SSEA-3/SSEA-4/GLOBO H antibodies disclosed herein can be used to determine whether the TACAs are normally temporally expressed which can be correlated with one or more disease states.

The SSEA-3/SSEA-4/GLOBO H antibodies disclosed herein can further be used to treat diseases in which one or more SSEA-3/SSEA-4/GLOBO Hs are aberrantly regulated or aberrantly functioning without interfering with the normal activity of SSEA-3/SSEA-4/GLOBO Hs for which the anti-SSEA-3/SSEA-4/GLOBO H antibodies disclosed herein are not specific.

In another aspect, the anti- SSEA-3/SSEA-4/GLOBO H antibodies disclosed herein find utility as reagents for detection of cancer states in various cell types and tissues.

In yet another aspect, the present anti- SSEA-3/SSEA-4/GLOBO H antibodies are
useful for the development of SSEA-3/SSEA-4/GLOBO H antagonists with blocking activity patterns similar to those of the subject antibodies disclosed herein. For example, anti- SSEA-3/SSEA-4/GLOBO H antibodies disclosed herein can be used to determine and identify other antibodies that have the same SSEA-3/SSEA-4/GLOBO H binding characteristics and/or capabilities of blocking SSEA-3/SSEA-4/GLOBO H- pathways.

As a further example, anti- SSEA-3/SSEA-4/GLOBO H antibodies disclosed herein can be used to identify other anti-SSEA-3/SSEA-4/GLOBO H antibodies that bind substantially the same antigenic determinant(s) of SSEA-3/SSEA-4/GLOBO H as the antibodies exemplified herein, including linear and conformational epitopes.

The anti-SSEA-3/SSEA-4/GLOBO H antibodies disclosed herein can be used in assays based on the physiological pathways in which SSEA-3/SSEA-4/GLOBO H is involved to screen for small molecule antagonists of SSEA-3/SSEA-4/GLOBO H which will exhibit similar pharmacological effects in blocking the binding of one or more binding partners to SSEA-3/SSEA-4/GLOBO H as the antibody does.

Generation of antibodies can be achieved using routine skills in the art, including those described herein, such as the hybridoma technique and screening of phage displayed libraries of binder molecules. These methods are well-established in the art.

Briefly, the anti-SSEA-3/SSEA-4/GLOBO H antibodies disclosed herein can be made by using combinatorial libraries to screen for synthetic antibody clones with the desired activity or activities. In principle, synthetic antibody clones are selected by screening phage libraries containing phage that display various fragments of antibody variable region (Fv) fused to phage coat protein. Such phage libraries are panned by affinity chromatography against the desired antigen. Clones expressing Fv fragments capable of binding to the desired antigen are adsorbed to the antigen and thus separated from the non-binding clones in the library. The binding clones are then eluted from the antigen, and can be further enriched by additional cycles of antigen adsorption/elution. Any of the anti-SSEA-3/SSEA-4/GLOBO H antibodies disclosed herein can be obtained by designing a suitable antigen screening procedure to select for the phage clone of interest followed by construction of a full length anti-SSEA-3/SSEA-4/GLOBO H antibody clone using the Fv sequences from the phage clone of interest and suitable constant region (Fc) sequences described in Kabat et al., Sequences of Proteins of Immunological Interest, Fifth Edition, NIH Publication 91-3242, Bethesda Md. (1991), vols. 1-3.

In one embodiment, anti-SSEA-3/SSEA-4/GLOBO H antibodies disclosed herein are monoclonal. Also encompassed within the scope of the invention are antibody fragments such as Fab, Fab', Fab'-SH and F(ab')2 fragments, and variations thereof, of the anti-SSEA-3/SSEA-4/GLOBO H antibodies provided herein. These antibody fragments can be created by traditional means, such as enzymatic digestion, or may be generated by recombinant techniques. Such antibody fragments may be chimeric, human or humanized. These fragments are useful for the experimental, diagnostic, and therapeutic purposes set forth herein.

Monoclonal antibodies can be obtained from a population of substantially homogeneous antibodies, i.e., the individual antibodies comprising the population are identical except for possible naturally occurring mutations that may be present in minor amounts. Thus, the modifier "monoclonal" indicates the character of the antibody as not being a mixture of discrete antibodies.

The anti-SSEA-3/SSEA-4/GLOBO H monoclonal antibodies disclosed herein can be made using a variety of methods known in the art, including the hybridoma method first described by Kohler et al., Nature, 256:495 (1975), or alternatively they may be made by recombinant DNA methods (e.g., U.S. Pat. No. 4,816,567).

### Vectors, Host Cells and Recombinant Methods

For recombinant production of an antibody of the invention, the nucleic acid encoding it is isolated and inserted into a replicable vector for further cloning (amplification of the DNA) or for expression. DNA encoding the antibody is readily isolated and sequenced using conventional procedures (e.g., by using oligonucleotide probes that are capable of binding specifically to genes encoding the heavy and light chains of the antibody). Many vectors are available. The choice of vector depends in part on the host cell to be used. Host cells include, but are not limited to, cells of either prokaryotic or eukaryotic (generally mammalian) origin. It will be appreciated that constant regions of any isotype can be used for this purpose, including IgG, IgM, IgA, IgD, and IgE constant regions, and that such constant regions can be obtained from any human or animal species.

### Generating Antibodies Using Prokaryotic Host Cells

### Vector Construction

Polynucleotide sequences encoding polypeptide components of the antibody disclosed herein can be obtained using standard recombinant techniques. Desired polynucleotide sequences may be isolated and sequenced from antibody producing cells such as hybridoma cells. Alternatively, polynucleotides can be synthesized using nucleotide synthesizer or PCR techniques. Once obtained, sequences encoding the polypeptides are inserted into a recombinant vector capable of replicating and expressing heterologous polynucleotides in prokaryotic hosts. Many vectors that are available and known in the art can be used for the purpose of the present invention. Selection of an appropriate vector will depend mainly on the size of the nucleic acids to be inserted into the vector and the particular host cell to be transformed with the vector. Each vector contains various components, depending on its function (amplification or expression of heterologous polynucleotide, or both) and its compatibility with the particular host cell in which it resides. The vector components generally include, but are not limited to: an origin of replication, a selection marker gene, a promoter, a ribosome binding site (RBS), a signal sequence, the heterologous nucleic acid insert and a transcription termination sequence.

In general, plasmid vectors containing replicon and control sequences which are derived from species compatible with the host cell are used in connection with these hosts. The vector ordinarily carries a replication site, as well as marking sequences which are capable of providing phenotypic selection in transformed cells. For example, E. coli is typically transformed using pBR322, a plasmid derived from an E. coli species. pBR322 contains genes encoding ampicillin (Amp) and tetracycline (Tet) resistance and thus provides easy means for identifying transformed cells. pBR322, its derivatives, or other microbial plasmids or bacteriophage may also contain, or be modified to contain, promoters which can be used by the microbial organism for expression of endogenous proteins. Examples of pBR322 derivatives used for expression of particular antibodies are described in detail in Carter et al., U.S. Pat. No. 5,648,237.

In addition, phage vectors containing replicon and control sequences that are compatible with the host microorganism can be used as transforming vectors in connection with these hosts. For example, bacteriophage such as λGEM™-11 may be utilized in making a recombinant vector which can be used to transform susceptible host cells such as E. coli LE392.

The expression vector disclosed herein may comprise two or more promoter-cistron pairs, encoding each of the polypeptide components. A promoter is an untranslated regulatory sequence located upstream (5') to a cistron that modulates its expression. Prokaryotic promoters typically fall into two classes, inducible and constitutive. Inducible promoter is a promoter that initiates increased levels of transcription of the cistron under its control in response to changes in the culture condition, e.g. the presence or absence of a nutrient or a change in temperature.

A large number of promoters recognized by a variety of potential host cells are well known. The selected promoter can be operably linked to cistron DNA encoding the light or heavy chain by removing the promoter from the source DNA via restriction enzyme digestion and inserting the isolated promoter sequence into the vector disclosed herein. Both the native promoter sequence and many heterologous promoters may be used to direct amplification and/or expression of the target genes. In some aspects, heterologous promoters are utilized, as they generally permit greater transcription and higher yields of expressed target gene as compared to the native target polypeptide promoter.

Promoters suitable for use with prokaryotic hosts include the PhoA promoter, the β-galactamase and lactose promoter systems, a tryptophan (trp) promoter system and hybrid promoters such as the tac or the trc promoter. However, other promoters that are functional in bacteria (such as other known bacterial or phage promoters) are suitable as well. Their nucleotide sequences have been published, thereby enabling a skilled worker operably to ligate them to cistrons encoding the target light and heavy chains (Siebenlist et al. (1980) Cell 20: 269) using linkers or adaptors to supply any required restriction sites.

In one aspect of the disclosure, each cistron within the recombinant vector comprises a secretion signal sequence component that directs translocation of the expressed polypeptides across a membrane. In general, the signal sequence may be a component of the vector, or it may be a part of the target polypeptide DNA that is inserted into the vector. The signal sequence selected for the purpose of this disclosure should be one that is recognized and processed (i.e. cleaved by a signal peptidase) by the host cell. For prokaryotic host cells that do not recognize and process the signal sequences native to the heterologous polypeptides, the signal sequence is substituted by a prokaryotic signal sequence selected, for example, from the group consisting of the alkaline phosphatase, penicillinase, Ipp, or heat-stable enterotoxin II (STII) leaders, LamB, PhoE, PelB, OmpA and MBP. In one aspect of the disclosure, the signal sequences used in both cistrons of the expression system are STII signal sequences or variants thereof.

In another aspect, the production of the immunoglobulins disclosed herein can occur in the cytoplasm of the host cell, and therefore does not require the presence of
secretion signal sequences within each cistron. In that regard, immunoglobulin light and heavy chains are expressed, folded and assembled to form functional immunoglobulins within the cytoplasm. Certain host strains (e.g., the E. coli trxB- strains) provide cytoplasm conditions that are favorable for disulfide bond formation, thereby permitting proper folding and assembly of expressed protein subunits. Proba and Pluckthun Gene, 159:203 (1995).

Antibodies disclosed herein can also be produced by using an expression system inwhich the quantitative ratio of expressed polypeptide components can be modulated in order to maximize the yield of secreted and properly assembled antibodies disclosed herein. Such modulation is accomplished at least in part by simultaneously modulating translational strengths for the polypeptide components.

One technique for modulating translational strength is disclosed in Simmons et al., U.S. Pat. No. 5,840,523. It utilizes variants of the translational initiation region (TIR) within a cistron. For a given TIR, a series of amino acid or nucleic acid sequence variants can be created with a range of translational strengths, thereby providing a convenient means by which to adjust this factor for the desired expression level of the specific chain. TIR variants can be generated by conventional mutagenesis techniques that result in codon changes which can alter the amino acid sequence. In certain aspects, changes in the nucleotide sequence are silent. Alterations in the TIR can include, for example, alterations in the number or spacing of Shine-Dalgarno sequences, along with alterations in the signal sequence. One method for generating mutant signal sequences is the generation of a "codon bank" at the beginning of a coding sequence that does not change the amino acid sequence of the signal sequence (i.e., the changes are silent). This can be accomplished by changing the third nucleotide position of each codon; additionally, some amino acids, such as leucine, serine, and arginine, have multiple first and second positions that can add complexity in making the bank. This method of mutagenesis is described in detail in Yansura et al. (1992) METHODS: A Companion to Methods in Enzymol. 4:151-158.

In one aspect, a set of vectors is generated with a range of TIR strengths for
each cistron therein. This limited set provides a comparison of expression levels of each chain as well as the yield of the desired antibody products under various TIR strength combinations. TIR strengths can be determined by quantifying the expression level of a reporter gene as described in detail in Simmons et al. U.S. Pat. No. 5,840,523. Based on the translational strength comparison, the desired individual TIRs are selected to be combined in the expression vector constructs disclosed herein.

Prokaryotic host cells suitable for expressing antibodies disclosed herein include Archaebacteria and Eubacteria, such as Gram-negative or Gram-positive organisms. Examples of useful bacteria include Escherichia (e.g., E. coli), Bacilli (e.g., B. subtilis), Enterobacteria, Pseudomonas species (e.g., P. aeruginosa), Salmonella typhimurium, Serratia marcescans, Klebsiella, Proteus, Shigella, Rhizobia, Vitreoscilla, or Paracoccus. In one aspect, gram-negative cells are used. In one aspect, E. coli cells are used as hosts for the disclosure. Examples of E. coli strains include strain W3110 (Bachmann, Cellular and Molecular Biology, vol. 2 (Washington, D.C.: American Society for Microbiology, 1987), pp. 1190-1219; ATCC Deposit No. 27,325) and derivatives thereof, including strain 33D3 having genotype W3110 ΔfhuA (ΔtonA) ptr3 lac Iq lacL8 ΔompTΔ(nmpc-fepE) degP41 kanR (U.S. Pat. No. 5,639,635). Other strains and derivatives thereof, such as E. coli 294 (ATCC 31,446), E. coli B, E. coli λ1776 (ATCC 31,537) and E. coli RV308 (ATCC 31,608) are also suitable. These examples are illustrative rather than limiting. Methods for constructing derivatives of any of the above-mentioned bacteria having defined genotypes are known in the art and described in, for example, Bass et al., Proteins, 8:309-314 (1990). It is generally necessary to select the appropriate bacteria taking into consideration replicability of the replicon in the cells of a bacterium. For example, E. coli, Serratia, or Salmonella species can be suitably used as the host when well known plasmids such as pBR322, pBR325, pACYC177, or pKN410 are used to supply the replicon. Typically the host cell should secrete minimal amounts of proteolytic enzymes, and additional protease inhibitors may desirably be incorporated in the cell culture.

### Antibody Production

Host cells are transformed with the above-described expression vectors and cultured in conventional nutrient media modified as appropriate for inducing promoters, selecting transformants, or amplifying the genes encoding the desired sequences.

Transformation means introducing DNA into the prokaryotic host so that the DNA is replicable, either as an extrachromosomal element or by chromosomal integrant. Depending on the host cell used, transformation is done using standard techniques appropriate to such cells. The calcium treatment employing calcium chloride is generally used for bacterial cells that contain substantial cell-wall barriers. Another method for transformation employs polyethylene glycol/DMSO. Yet another technique used is electroporation.

Prokaryotic cells used to produce the polypeptides disclosed herein are grown in media known in the art and suitable for culture of the selected host cells. Examples of suitable media include luria broth (LB) plus necessary nutrient supplements. In some aspects, the media also contains a selection agent, chosen based on the construction of the expression vector, to selectively permit growth of prokaryotic cells containing the expression vector. For example, ampicillin is added to media for growth of cells expressing ampicillin resistant gene.

Any necessary supplements besides carbon, nitrogen, and inorganic phosphate sources may also be included at appropriate concentrations introduced alone or as a mixture with another supplement or medium such as a complex nitrogen source. Optionally the culture medium may contain one or more reducing agents selected from the group consisting of glutathione, cysteine, cystamine, thioglycollate, dithioerythritol and dithiothreitol.

The prokaryotic host cells are cultured at suitable temperatures. For E. coli growth, for example, growth occurs at a temperature range including, but not limited to, about 20° C. to about 39° C., about 25° C. to about 37° C., and at about 30° C. The pH of the medium may be any pH ranging from about 5 to about 9, depending mainly on the host organism. For E. coli, the pH can be from about 6.8 to about 7.4, or about 7.0.

If an inducible promoter is used in the expression vector disclosed herein, protein expression is induced under conditions suitable for the activation of the promoter. In one aspect of the disclosure, PhoA promoters are used for controlling transcription of the polypeptides. Accordingly, the transformed host cells are cultured in a phosphate-limiting medium for induction. In one aspect, the phosphate-limiting medium is the C.R.A.P
medium (see, e.g., Simmons et al., J. Immunol. Methods (2002), 263:133-147). A variety of other inducers may be used, according to the vector construct employed, as is known in the art.

In one aspect, the expressed polypeptides of the present disclosure are secreted into and recovered from the periplasm of the host cells. Protein recovery typically involves disrupting the microorganism, generally by such means as osmotic shock, sonication or lysis. Once cells are disrupted, cell debris or whole cells may be removed by centrifugation or
filtration. The proteins may be further purified, for example, by affinity resin chromatography. Alternatively, proteins can be transported into the culture media and isolated therein. Cells may be removed from the culture and the culture supernatant being filtered and concentrated for further purification of the proteins produced. The expressed polypeptides can be further isolated and identified using commonly known methods such as polyacrylamide gel electrophoresis (PAGE) and Western blot assay.

In one aspect of the disclosure, antibody production is conducted in large quantity by a fermentation process. Various large-scale fed-batch fermentation procedures are available for production of recombinant proteins. Large-scale fermentations have at least 1000 liters of capacity, for example about 1,000 to 100,000 liters of capacity. These fermentors use agitator impellers to distribute oxygen and nutrients, especially glucose (a common carbon/energy source). Small scale fermentation refers generally to fermentation in a fermentor that is no more than approximately 100 liters in volumetric capacity, and can range from about 1 liter to about 100 liters.

In a fermentation process, induction of protein expression is typically initiated after the cells have been grown under suitable conditions to a desired density, e.g., an OD550 of about 180-220, at which stage the cells are in the early stationary phase. A variety of inducers may be used, according to the vector construct employed, as is known in the art and described above. Cells may be grown for shorter periods prior to induction. Cells are usually induced for about 12-50 hours, although longer or shorter induction time may be used.

To improve the production yield and quality of the polypeptides disclosed herein, various fermentation conditions can be modified. For example, to improve the proper assembly and folding of the secreted antibody polypeptides, additional vectors overexpressing chaperone proteins, such as Dsb proteins (DsbA, DsbB, DsbC, DsbD and or DsbG) or FkpA
(a peptidylprolyl cis,trans-isomerase with chaperone activity) can be used to co-transform the host prokaryotic cells. The chaperone proteins have been demonstrated to facilitate the proper folding and solubility of heterologous proteins produced in bacterial host cells. Chen et al. (1999) J Bio Chem 274:19601-19605; Georgiou et al., U.S. Pat. No. 6,083,715; Georgiou et al., U.S. Pat. No. 6,027,888; Bothmann and Pluckthun (2000) J. Biol. Chem. 275:17100-17105; Ramm and Pluckthun (2000) J. Biol. Chem. 275:17106-17113; Arie et al. (2001) Mol. Microbiol. 39:199-210.

To minimize proteolysis of expressed heterologous proteins (especially those that are proteolytically sensitive), certain host strains deficient for proteolytic enzymes can be used for the present disclosure. For example, host cell strains may be modified to effect genetic mutation(s) in the genes encoding known bacterial proteases such as Protease III, OmpT, DegP, Tsp, Protease I, Protease Mi, Protease V, Protease VI and combinations thereof. Some E. coli protease-deficient strains are available and described in, for example, Joly et al. (1998), supra; Georgiou et al., U.S. Pat. No. 5,264,365; Georgiou et al., U.S. Pat. No. 5,508,192; Hara et al., Microbial Drug Resistance, 2:63-72 (1996).

In one aspect, E. coli strains deficient for proteolytic enzymes and transformed with plasmids overexpressing one or more chaperone proteins are used as host cells in the expression system of the disclosure.

### Antibody Purification

In one aspect, the antibody protein produced herein is further purified to obtain preparations that are substantially homogeneous for further assays and uses. Standard protein purification methods known in the art can be employed. The following procedures are exemplary of suitable purification procedures: fractionation on immunoaffinity or ion-exchange columns, ethanol precipitation, reverse phase HPLC, chromatography on silica or on a cation-exchange resin such as DEAE, chromatofocusing, SDS-PAGE, ammonium sulfate precipitation, and gel filtration using, for example, Sephadex G-75.

In one aspect, Protein A immobilized on a solid phase is used for immunoaffinity purification of the antibody products disclosed herein. Protein A is a 41 kD cell wall protein from Staphylococcus aureas which binds with a high affinity to the Fc region of antibodies. Lindmark et al (1983) J. Immunol. Meth. 62:1-13. The solid phase to which Protein A is immobilized can be a column comprising a glass or silica surface, or a controlled pore glass column or a silicic acid column. In some applications, the column is coated with a reagent, such as glycerol, to possibly prevent nonspecific adherence of contaminants.

As the first step of purification, the preparation derived from the cell culture as described above can be applied onto a Protein A immobilized solid phase to allow specific binding of the antibody of interest to Protein A. The solid phase would then be washed to remove contaminants non-specifically bound to the solid phase. Finally the antibody of interest is recovered from the solid phase by elution.

### Generating Antibodies Using Eukaryotic Host Cells

The vector components generally include one or more of the
following: a signal sequence, an origin of replication, one or more marker genes, an enhancer element, a promoter, and a transcription termination sequence.

### (i) Signal Sequence Component

A vector for use in a eukaryotic host cell may also contain a signal sequence or other polypeptide having a specific cleavage site at the N-terminus of the mature protein or polypeptide of interest. The heterologous signal sequence selected generally is one that is recognized and processed (i.e., cleaved by a signal peptidase) by the host cell. In mammalian cell expression, mammalian signal sequences as well as viral secretory leaders, for example, the herpes simplex gD signal, are available.

The DNA for such precursor region is ligated in reading frame to DNA encoding the antibody.

### (ii) Origin of Replication

Generally, an origin of replication component is not needed for mammalian expression vectors. For example, the SV40 origin may typically be used only because it contains the early promoter.

### (iii) Selection Gene Component

Expression and cloning vectors may contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, where relevant, or (c) supply critical nutrients not available from complex media.

One example of a selection scheme utilizes a drug to arrest growth of a host cell. Those cells that are successfully transformed with a heterologous gene produce a protein conferring drug resistance and thus survive the selection regimen. Examples of such dominant selection use the drugs neomycin, mycophenolic acid and hygromycin.

Another example of suitable selectable markers for mammalian cells are those that enable the identification of cells competent to take up the antibody nucleic acid, such as DHFR, thymidine kinase, metallothionein-I and -II (e.g., primate metallothionein genes), adenosine deaminase, ornithine decarboxylase, etc.

For example, cells transformed with the DHFR selection gene may first be identified by culturing all of the transformants in a culture medium that contains methotrexate (Mtx), a competitive antagonist of DHFR. Appropriate host cells when wild-type DHFR is employed include, for example, the Chinese hamster ovary (CHO) cell line deficient in DHFR activity (e.g., ATCC CRL-9096).

Alternatively, host cells (particularly wild-type hosts that contain endogenous DHFR) transformed or co-transformed with DNA sequences encoding an antibody, wild-type DHFR protein, and another selectable marker such as aminoglycoside 3'-phosphotransferase (APH) can be selected by cell growth in medium containing a selection agent for the selectable marker such as an aminoglycosidic antibiotic, e.g., kanamycin, neomycin, or G418. See U.S. Pat. No. 4,965,199.

### (iv) Promoter Component

Expression and cloning vectors usually contain a promoter that is recognized by the host organism and is operably linked to nucleic acid encoding a polypeptide of interest (e.g., an antibody). Promoter sequences are known for eukaryotes. Virtually all eukaryotic genes have an AT-rich region located approximately 25 to 30 bases upstream from the site where transcription is initiated. Another sequence found 70 to 80 bases upstream from the start of transcription of many genes is a CNCAAT region where N may be any nucleotide. At the 3' end of most eukaryotic genes is an AATAAA sequence that may be the signal for addition of the poly A tail to the 3' end of the coding sequence. All of these sequences are suitably inserted into eukaryotic expression vectors.

Antibody polypeptide transcription from vectors in mammalian host cells can be controlled, for example, by promoters obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, or from heat-shock promoters, provided such promoters are compatible with the host cell systems.

The early and late promoters of the SV40 virus are conveniently obtained as an SV40 restriction fragment that also contains the SV40 viral origin of replication. The immediate early promoter of the human cytomegalovirus is conveniently obtained as a HindIII E restriction fragment. A system for expressing DNA in mammalian hosts using the bovine papilloma virus as a vector is disclosed in U.S. Pat. No. 4,419,446. A modification of this system is described in U.S. Pat. No. 4,601,978. See also Reyes et al., Nature 297:598-601 (1982) on expression of human β-interferon cDNA in mouse cells under the control of a thymidine kinase promoter from herpes simplex virus. Alternatively, the Rous Sarcoma Virus long terminal repeat can be used as the promoter.

### (v) Enhancer Element Component

Transcription of DNA encoding an antibody polypeptide of the invention by higher eukaryotes can often be increased by inserting an enhancer sequence into the vector. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. See also Yaniv, Nature 297:17-18 (1982) on enhancing elements for activation of eukaryotic promoters. The enhancer may be spliced into the vector at a position 5' or 3' to the antibody polypeptide-encoding sequence, but is generally located at a site 5' from the promoter.

### (vi) Transcription Termination Component

Expression vectors used in eukaryotic host cells will typically also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding an antibody. One useful transcription termination component is the bovine growth hormone polyadenylation region. See WO94/11026 and the expression vector disclosed therein.

### (vii) Selection and Transformation of Host Cells

Suitable host cells for cloning or expressing the DNA in the vectors herein include higher eukaryote cells described herein, including vertebrate host cells. Propagation of vertebrate cells in culture (tissue culture) has become a routine procedure. Examples of useful mammalian host cell lines are monkey kidney CV1 line transformed by SV40 (COS-7, ATCC CRL 1651); human embryonic kidney line (293 or 293 cells subcloned for growth in suspension culture, Graham et al., J. Gen Virol. 36:59 (1977)); baby hamster kidney cells (BHK, ATCC CCL 10); Chinese hamster ovary cells/-DHFR (CHO, Urlaub et al., Proc. Natl. Acad. Sci. USA 77:4216 (1980)); mouse sertoli cells (TM4, Mather, Biol. Reprod. 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci. 383:44-68 (1982)); MRC 5 cells; FS4 cells; and a human hepatoma line (Hep G2).

Host cells are transformed with the above-described expression or cloning vectors for antibody production and cultured in conventional nutrient media modified as appropriate for inducing promoters, selecting transformants, or amplifying the genes encoding the desired sequences.

### (viii) Culturing the Host Cells

The host cells used to produce an antibody disclosed herein may be cultured in a variety of media. Commercially available media such as Ham's F10 (Sigma), Minimal Essential Medium ((MEM), (Sigma), RPMI-1640 (Sigma), and Dulbecco's Modified Eagle's Medium ((DMEM), Sigma) are suitable for culturing the host cells. In addition, any of the media described in Ham et al., Meth. Enz. 58:44 (1979), Barnes et al., Anal. Biochem. 102:255 (1980), U.S. Pat. Nos. 4,767,704; 4,657,866; 4,927,762; 4,560,655; or 5,122,469; WO 90/03430; WO 87/00195; or U.S. Pat. Re. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN™ drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

### (ix) Purification of Antibody

When using recombinant techniques, the antibody can be produced intracellularly, or directly secreted into the medium. If the antibody is produced intracellularly, as a first step, the particulate debris, either host cells or lysed fragments, are generally removed, for example, by centrifugation or ultrafiltration. Where the antibody is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

The antibody composition prepared from the cells can be purified using, for example, hydroxylapatite chromatography, gel electrophoresis, dialysis, and affinity chromatography, with affinity chromatography being a generally acceptable purification technique. The suitability of affinity reagents such as protein A as an affinity ligand depends on the species and isotype of any immunoglobulin Fc domain that is present in the antibody. Protein A can be used to purify antibodies that are based on human γ1, γ2, or γ4 heavy chains (Lindmark et al., J. Immunol. Meth. 62:1-13 (1983)). Protein G is recommended for all mouse isotypes and for human γ3 (Guss et al., EMBO J. 5:15671575 (1986)). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. Where the antibody comprises a CH3 domain, the Bakerbond ABX™ resin (J. T. Baker, Phillipsburg, N.J.) is useful for purification. Other techniques for protein purification such as fractionation on an ion-exchange column, ethanol precipitation, Reverse Phase HPLC, chromatography on silica, chromatography on heparin SEPHAROSE™ chromatography on an anion or cation exchange resin (such as a polyaspartic acid column), chromatofocusing, SDS-PAGE, and ammonium sulfate precipitation are also available depending on the antibody to be recovered.

Following any preliminary purification step(s), the mixture comprising the antibody of interest and contaminants may be subjected to further purification steps, as necessary, for example by low pH hydrophobic interaction chromatography using an elution buffer at a pH between about 2.5-4.5, generally performed at low salt concentrations (e.g., from about 0-0.25M salt).

It should be noted that, in general, techniques and methodologies for preparing antibodies for use in research, testing and clinical use are well-established in the art, consistent with the above and/or as deemed appropriate by one skilled in the art for the particular antibody of interest.

### Activity Assays

Antibodies disclosed herein can be characterized for their physical/chemical properties and biological functions by various assays known in the art.

Purified antibodies can be further characterized by a series of assays including N-terminal sequencing, amino acid analysis, non-denaturing size exclusion high pressure liquid chromatography (HPLC), mass spectrometry, ion exchange chromatography and papain digestion.

Where necessary, antibodies are analyzed for their biological activity. In some aspects, antibodies disclosed herein are tested for their antigen binding activity. The antigen binding assays that are known in the art and can be used herein include without limitation any direct or competitive binding assays using techniques such as western blots, radioimmunoassays, ELISA (enzyme linked immunosorbent assay), "sandwich" immunoassays, immunoprecipitation assays, fluorescent immunoassays, and protein A immunoassays.

In one aspect, the disclosure contemplates an altered antibody that possesses some but not all effector functions, which make it a desirable candidate for many applications in which the half life of the antibody in vivo is important yet certain effector functions (such as complement and ADCC) are unnecessary or deleterious. In certain aspects, the Fc activities of the antibody are measured to ensure that only the desired properties are maintained. In vitro and/or in vivo cytotoxicity assays can be conducted to confirm the reduction/depletion of CDC and/or ADCC activities. For example, Fc receptor (FcR) binding assays can be conducted to ensure that the antibody lacks FcγR binding (hence likely lacking ADCC activity), but retains FcRn binding ability. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol 9:457-92 (1991). An example of an in vitro assay to assess ADCC activity of a molecule of interest is described in U.S. Pat. No. 5,500,362 or U.S. Pat. No. 5,821,337. Useful effector cells for such assays include peripheral blood mononuclear cells (PBMC) and Natural Killer (NK) cells. Alternatively, or additionally, ADCC activity of the molecule of interest may be assessed in vivo, e.g., in a animal model such as that disclosed in Clynes et al. PNAS (USA) 95:652-656 (1998). C1q binding assays may also be carried out to confirm that the antibody is unable to bind C1q and hence lacks CDC activity. To assess complement activation, a CDC assay, e.g. as described in Gazzano-Santoro et al., J. Immunol. Methods 202:163 (1996), may be performed. FcRn binding and in vivo clearance/half life determinations can also be performed using methods known in the art.

### Antibody Fragments

The present disclosure encompasses antibody fragments. In certain circumstances there are advantages of using antibody fragments, rather than whole antibodies. The smaller size of the fragments allows for rapid clearance, and may lead to improved access to solid tumors.

Various techniques have been developed for the production of antibody fragments. Traditionally, these fragments were derived via proteolytic digestion of intact antibodies (see, e.g., Morimoto et al., Journal of Biochemical and Biophysical Methods 24:107-117 (1992); and Brennan et al., Science, 229:81 (1985)). However, these fragments can now be produced directly by recombinant host cells. Fab, Fv and ScFv antibody fragments can all be expressed in and secreted from E. coli, thus allowing the facile production of large amounts of these fragments. Antibody fragments can be isolated from the antibody phage libraries discussed above. Alternatively, Fab'-SH fragments can be directly recovered from E. coli and chemically coupled to form F(ab')2 fragments (Carter et al., Bio/Technology 10: 163-167 (1992)). According to another approach, F(ab')2 fragments can be isolated directly from recombinant host cell culture. Fab and F(ab')2 fragment with increased in vivo half-life comprising salvage receptor binding epitope residues are described in U.S. Pat. No. 5,869,046. Other techniques for the production of antibody fragments will be apparent to the skilled practitioner. In other aspects, the antibody of choice is a single chain Fv fragment (scFv). See WO 93/16185; U.S. Pat. Nos. 5,571,894; and 5,587,458. Fv and sFv are the only species with intact combining sites that are devoid of constant regions; thus, they are suitable for reduced nonspecific binding during in vivo use. sFv fusion proteins may be constructed to yield fusion of an effector protein at either the amino or the carboxy terminus of an sFv. See Antibody Engineering, ed. Borrebaeck, supra. The antibody fragment may also be a "linear antibody", e.g., as described in U.S. Pat. No. 5,641,870 for example. Such linear antibody fragments may be monospecific or bispecific.

### Humanized Antibodies

The disclosure encompasses humanized antibodies. Various methods for humanizing non-human antibodies are known in the art. For example, a humanized antibody can have one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al. (1986) Nature 321:522-525; Riechmann et al. (1988) Nature 332:323-327; Verhoeyen et al. (1988) Science 239:1534-1536), by substituting hypervariable region sequences for the corresponding sequences of a human antibody. Accordingly, such "humanized" antibodies are chimeric antibodies (U.S. Pat. No. 4,816,567) wherein substantially less than an intact human variable domain has been substituted by the corresponding sequence from a non-human species. In practice, humanized antibodies are typically human antibodies in which some hypervariable region residues and possibly some FR residues are substituted by residues from analogous sites in rodent antibodies.

The choice of human variable domains, both light and heavy, to be used in making the humanized antibodies can be important to reduce antigenicity. According to the so-called "best-fit" method, the sequence of the variable domain of a rodent antibody is screened against the entire library of known human variable-domain sequences. The human sequence which is closest to that of the rodent is then accepted as the human framework for the humanized antibody (Sims et al. (1993) J. Immunol. 151:2296; Chothia et al. (1987) J. Mol. Biol. 196:901. Another method uses a particular framework derived from the consensus sequence of all human antibodies of a particular subgroup of light or heavy chains. The same framework may be used for several different humanized antibodies (Carter et al. (1992) Proc. Natl. Acad. Sci. USA, 89:4285; Presta et al. (1993) J. Immunol., 151:2623.

It is further generally desirable that antibodies be humanized with retention of high affinity for the antigen and other favorable biological properties. To achieve this goal, according to one method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the hypervariable region residues are directly and most substantially involved in influencing antigen binding.

### Human Antibodies

Human anti-SSEA-3/SSEA-4/GLOBO H antibodies disclosed herein can be constructed by combining Fv clone variable domain sequence(s) selected from human-derived phage display libraries with known human constant domain sequences(s) as described above. Alternatively, human monoclonal anti-SSEA-3/SSEA-4/GLOBO H antibodies disclosed herein can be made by the hybridoma method. Human myeloma and mouse-human heteromyeloma cell lines for the production of human monoclonal antibodies have been described, for example, by Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).

It is now possible to produce transgenic animals (e.g. mice) that are capable, upon immunization, of producing a full repertoire of human antibodies in the absence of endogenous immunoglobulin production. For example, it has been described that the homozygous deletion of the antibody heavy-chain joining region (JH) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, e.g., Jakobovits et al., Proc. Natl. Acad. Sci. USA, 90: 2551 (1993); Jakobovits et al., Nature, 362: 255 (1993); Bruggermann et al., Year in Immunol., 7: 33 (1993).

Gene shuffling can also be used to derive human antibodies from non-human, e.g. rodent, antibodies, where the human antibody has similar affinities and specificities to the starting non-human antibody. According to this method, which is also called "epitope imprinting", either the heavy or light chain variable region of a non-human antibody fragment obtained by phage display techniques as described above is replaced with a repertoire of human V domain genes, creating a population of non-human chain/human chain scFv or Fab chimeras. Selection with antigen results in isolation of a non-human chain/human chain chimeric scFv or Fab wherein the human chain restores the antigen binding site destroyed upon removal of the corresponding non-human chain in the primary phage display clone, i.e. the epitope governs (imprints) the choice of the human chain partner. When the process is repeated in order to replace the remaining non-human chain, a human antibody is obtained (see PCT WO 93/06213 published Apr. 1, 1993). Unlike traditional humanization of non-human antibodies by CDR grafting, this technique provides completely human antibodies, which have no FR or CDR residues of non-human origin.

### Bispecific Antibodies

Bispecific antibodies are monoclonal antibodies that have binding specificities for at least two different antigens. In certain aspects, bispecific antibodies are human or humanized antibodies. In certain aspects, one of the binding specificities is for SSEA-3/SSEA-4/GLOBO H including a specific lysine linkage and the other is for any other antigen. In certain aspects, bispecific antibodies may bind to two different SSEA-3/SSEA-4/GLOBO Hs having two different lysine linkages. Bispecific antibodies can be prepared as full length antibodies or antibody fragments (e.g. F(ab')2 bispecific antibodies).

Methods for making bispecific antibodies are known in the art. Traditionally, the recombinant production of bispecific antibodies is based on the co-expression of two immunoglobulin heavy chain-light chain pairs, where the two heavy chains have different specificities (Milstein and Cuello, Nature, 305: 537 (1983)). Because of the random assortment of immunoglobulin heavy and light chains, these hybridomas (quadromas) produce a potential mixture of 10 different antibody molecules, of which only one has the correct bispecific structure. The purification of the correct molecule, which is usually done by affinity chromatography steps, is rather cumbersome, and the product yields are low. Similar procedures are disclosed in WO 93/08829 published May 13, 1993, and in Traunecker et al., EMBO J., 10: 3655 (1991).

According to a different aspect, antibody variable domains with the desired binding specificities (antibody-antigen combining sites) are fused to immunoglobulin constant domain sequences. The fusion, for example, is with an immunoglobulin heavy chain constant domain, comprising at least part of the hinge, CH2, and CH3 regions. In certain aspects, the first heavy-chain constant region (CHI), containing the site necessary for light chain binding, is present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In one aspect of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology, 121:210 (1986).

According to another approach, the interface between a pair of antibody molecules can be engineered to maximize the percentage of heterodimers which are recovered from recombinant cell culture. The interface comprises at least a part of the CH3 domain of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (U.S. Pat. No. 4,676,980), and for treatment of HIV infection (WO 91/00360, WO 92/00373, and EP 03089). Heteroconjugate antibodies may be made using any convenient cross-linking methods. Suitable cross-linking agents are well known in the art, and are disclosed in U.S. Pat. No. 4,676,980, along with a number of cross-linking techniques.

Techniques for generating bispecific antibodies from antibody fragments have also been described in the literature. For example, bispecific antibodies can be prepared using chemical linkage. Brennan et al., Science, 229: 81 (1985) describe a procedure wherein intact antibodies are proteolytically cleaved to generate F(ab')2 fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Recent progress has facilitated the direct recovery of Fab'-SH fragments from E. coli, which can be chemically coupled to form bispecific antibodies. Shalaby et al., J. Exp. Med., 175: 217-225 (1992) describe the production of a fully humanized bispecific antibody F(ab')2 molecule. Each Fab' fragment was separately secreted from E. coli and subjected to directed chemical coupling in vitro to form the bispecific antibody. The bispecific antibody thus formed was able to bind to cells overexpressing the HER2 receptor and normal human T cells, as well as trigger the lytic activity of human cytotoxic lymphocytes against human breast tumor targets.

Various techniques for making and isolating bispecific antibody fragments directly from recombinant cell culture have also been described. For example, bispecific antibodies have been produced using leucine zippers. Kostelny et al., J. Immunol., 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA, 90:6444-6448 (1993) has provided an alternative mechanism for making bispecific antibody fragments. The fragments comprise a heavy-chain variable domain (VH) connected to a light-chain variable domain (VL) by a linker which is too short to allow pairing between the two domains on the same chain. Accordingly, the VH and VL domains of one fragment are forced to pair with the complementary VL and VH domains of another fragment, thereby forming two antigen-binding sites. Another strategy for making bispecific antibody fragments by the use of single-chain Fv (sFv) dimers has also been reported. See Gruber et al., J. Immunol., 152:5368 (1994).

Antibodies with more than two valencies are contemplated. For example, trispecific antibodies can be prepared. Tutt et al. J. Immunol. 147: 60 (1991).

### Multivalent Antibodies

A multivalent antibody may be internalized (and/or catabolized) faster than a bivalent antibody by a cell expressing an antigen to which the antibodies bind. The antibodies disclosed herein can be multivalent antibodies (which are other than of the IgM class) with three or more antigen binding sites (e.g. tetravalent antibodies), which can be readily produced by recombinant expression of nucleic acid encoding the polypeptide chains of the antibody. The multivalent antibody can comprise a dimerization domain and three or more antigen binding sites. The dimerization domain comprises (or consists of), for example, an Fc region or a hinge region. In this scenario, the antibody will comprise an Fc region and three or more antigen binding sites amino-terminal to the Fc region. In one embodiment, a multivalent antibody comprises (or consists of), for example, three to about eight, or four antigen binding sites. The multivalent antibody comprises at least one polypeptide chain (for example, two polypeptide chains), wherein the polypeptide chain(s) comprise two or more variable domains. For instance, the polypeptide chain(s) may comprise VD1-(X1)n-VD2-(X2)n-Fc, wherein VD1 is a first variable domain, VD2 is a second variable domain, Fc is one polypeptide chain of an Fc region, X1 and X2 represent an amino acid or polypeptide, and n is 0 or 1. For instance, the polypeptide chain(s) may comprise: VH-CH1-flexible linker-VH-CH1-Fc region chain; or VH-CH1-VH-CH1-Fc region chain. The multivalent antibody herein may further comprise at least two (for example, four) light chain variable domain polypeptides. The multivalent antibody herein may, for instance, comprise from about two to about eight light chain variable domain polypeptides. The light chain variable domain polypeptides contemplated here comprise a light chain variable domain and, optionally, further comprise a CL domain. Antibody Variants

In some aspects, amino acid sequence modification(s) of the antibodies described herein are contemplated. For example, it may be desirable to improve the binding affinity and/or other biological properties of the antibody. Amino acid sequence variants of the antibody are prepared by introducing appropriate nucleotide changes into the antibody nucleic acid, or by peptide synthesis. Such modifications include, for example, deletions from, and/or insertions into and/or substitutions of, residues within the amino acid sequences of the antibody. Any combination of deletion, insertion, and substitution can be made to arrive at the final construct, provided that the final construct possesses the desired characteristics. The amino acid alterations may be introduced in the subject antibody amino acid sequence at the time that sequence is made.

A useful method for identification of certain residues or regions of the antibody that are preferred locations for mutagenesis is called "alanine scanning mutagenesis" as described by Cunningham and Wells (1989) Science, 244:1081-1085. Here, a residue or group of target residues are identified (e.g., charged residues such as arg, asp, his, lys, and glu) and replaced by a neutral or negatively charged amino acid (e.g., alanine or polyalanine) to affect the interaction of the amino acids with antigen. Those amino acid locations demonstrating functional sensitivity to the substitutions then are refined by introducing further or other variants at, or for, the sites of substitution. Thus, while the site for introducing an amino acid sequence variation is predetermined, the nature of the mutation per se need not be predetermined. For example, to analyze the performance of a mutation at a given site, ala scanning or random mutagenesis is conducted at the target codon or region and the expressed immunoglobulins are screened for the desired activity.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue or the antibody fused to a cytotoxic polypeptide. Other insertional variants of the antibody molecule include the fusion to the N- or C-terminus of the antibody to an enzyme (e.g. for ADEPT) or a polypeptide which increases the serum half-life of the antibody.

Another type of variant is an amino acid substitution variant. These variants have at least one amino acid residue in the antibody molecule replaced by a different residue. The sites of greatest interest for substitutional mutagenesis include the hypervariable regions, but FR alterations are also contemplated. Conservative substitutions are shown in Table A under the heading of "preferred substitutions". If such substitutions result in a change in biological activity, then more substantial changes, denominated "exemplary substitutions" in Table A, or as further described below in reference to amino acid classes, may be introduced and the products screened.

### TABLE A

Original Exemplary Preferred
Residue Substitutions Substitutions
Ala (A) Val; Leu; Ile Val
Arg (R) Lys; Gln; Asn Lys
Asn (N) Gln; His; Asp, Lys; Arg Gln
Asp (D) Glu; Asn Glu
Cys (C) Ser; Ala Ser
Gln (Q) Asn; Glu Asn
Glu (E) Asp; Gln Asp
Gly (G) Ala Ala
His (H) Asn; Gln; Lys; Arg Arg
Ile (I) Leu; Val; Met; Ala; Leu
Phe; Norleucine
Leu (L) Norleucine; Ile; Val; Ile
Met; Ala; Phe
Lys (K) Arg; Gln; Asn Arg
Met (M) Leu; Phe; Ile Leu
Phe (F) Trp; Leu; Val; Ile; Ala; Tyr Tyr
Pro (P) Ala Ala
Ser (S) Thr Thr
Thr (T) Val; Ser Ser
Trp (W) Tyr; Phe Tyr
Tyr (Y) Trp; Phe; Thr; Ser Phe
Val (V) Ile; Leu; Met; Phe; Leu
Ala; Norleucine

Substantial modifications in the biological properties of the antibody are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Amino acids may be grouped according to similarities in the properties of their side chains (in A. L. Lehninger, in Biochemistry, second ed., pp. 73-75, Worth Publishers, New York (1975)):
- (1) non-polar: Ala (A), Val (V), Leu (L), Ile (I), Pro (P), Phe (F), Trp (W), Met (M)
- (2) uncharged polar: Gly (G), Ser (S), Thr (T), Cys (C), Tyr (Y), Asn (N), Gln (O)
- (3) acidic: Asp (D), Glu (E)
- (4) basic: Lys (K), Arg (R), His (H)

Alternatively, naturally occurring residues may be divided into groups based on common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class. Such substituted residues also may be introduced into the conservative substitution sites or, into the remaining (non-conserved) sites.

One type of substitutional variant involves substituting one or more hypervariable region residues of a parent antibody (e.g. a humanized or human antibody). Generally, the resulting variant(s) selected for further development will have modified (e.g., improved) biological properties relative to the parent antibody from which they are generated. A convenient way for generating such substitutional variants involves affinity maturation using phage display. Briefly, several hypervariable region sites (e.g. 6-7 sites) are mutated to generate all possible amino acid substitutions at each site. The antibodies thus generated are displayed from filamentous phage particles as fusions to at least part of a phage coat protein (e.g., the gene III product of M13) packaged within each particle. The phage-displayed variants are then screened for their biological activity (e.g. binding affinity) as herein disclosed. In order to identify candidate hypervariable region sites for modification, scanning mutagenesis (e.g., alanine scanning) can be performed to identify hypervariable region residues contributing significantly to antigen binding. Alternatively, or additionally, it may be beneficial to analyze a crystal structure of the antigen-antibody complex to identify contact points between the antibody and antigen. Such contact residues and neighboring residues are candidates for substitution according to techniques known in the art, including those elaborated herein. Once such variants are generated, the panel of variants is subjected to screening using techniques known in the art, including those described herein, and antibodies with superior properties in one or more relevant assays may be selected for further development.

Nucleic acid molecules encoding amino acid sequence variants of the antibody are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the antibody.

It may be desirable to introduce one or more amino acid modifications in an Fc region of antibodies of the invention, thereby generating an Fc region variant. The Fc region variant may comprise a human Fc region sequence (e.g., a human IgG1, IgG2, IgG3 or IgG4 Fc region) comprising an amino acid modification (e.g. a substitution) at one or more amino acid positions including that of a hinge cysteine.

In accordance with this description and the teachings of the art, it is contemplated that in some aspects, an antibody disclosed herein may comprise one or more alterations as compared to the wild type counterpart antibody, e.g. in the Fc region. These antibodies would nonetheless retain substantially the same characteristics required for therapeutic utility as
compared to their wild type counterpart. For example, it is thought that certain alterations can be made in the Fc region that would result in altered (i.e., either improved or diminished) C1q binding and/or Complement Dependent Cytotoxicity (CDC), e.g., as described in WO99/51642. See also Duncan & Winter Nature 322:738-40 (1988); U.S. Pat. Nos. 5,648,260; 5,624,821; and WO94/29351 concerning other examples of Fc region variants.

In one aspect, the disclosure provides antibodies comprising modifications in the interface of Fc polypeptides comprising the Fc region, wherein the modifications facilitate and/or promote heterodimerization. These modifications comprise introduction of a protuberance into a first Fc polypeptide and a cavity into a second Fc polypeptide, wherein the protuberance is positionable in the cavity so as to promote complexing of the first and second Fc polypeptides. Methods of generating antibodies with these modifications are known in the art, e.g., as described in U.S. Pat. No. 5,731,168.

### Immunoconjugates

In another aspect, the disclosure provides immunoconjugates, or antibody-drug conjugates (ADC), comprising an antibody conjugated to a cytotoxic agent such as a chemotherapeutic agent, a drug, a growth inhibitory agent, a toxin (e.g., an enzymatically active toxin of bacterial, fungal, plant, or animal origin, or fragments thereof), or a radioactive isotope (i.e., a radioconjugate).

The use of antibody-drug conjugates for the local delivery of cytotoxic or cytostatic agents, i.e. drugs to kill or inhibit tumor cells in the treatment of cancer (Syrigos and Epenetos (1999) Anticancer Research 19:605-614; Niculescu-Duvaz and Springer (1997) Adv. Drg Del. Rev. 26:151-172; U.S. Pat. No. 4,975,278) allows targeted delivery of the drug moiety to tumors, and intracellular accumulation therein, where systemic administration of these unconjugated drug agents may result in unacceptable levels of toxicity to normal cells as well as the tumor cells sought to be eliminated (Baldwin et al., (1986) Lancet pp. (Mar. 15, 1986):603-05; Thorpe, (1985) "Antibody Carriers Of Cytotoxic Agents In Cancer Therapy: A Review," in Monoclonal Antibodies '84: Biological And Clinical Applications, A. Pinchera et al. (ed.s), pp. 475-506). Maximal efficacy with minimal toxicity is sought thereby. Both polyclonal antibodies and monoclonal antibodies have been reported as useful in these strategies (Rowland et al., (1986) Cancer Immunol. Immunother., 21:183-87). Drugs used in these methods include daunomycin, doxorubicin, methotrexate, and vindesine (Rowland et al., (1986) supra). Toxins used in antibody-toxin conjugates include bacterial toxins such as diphtheria toxin, plant toxins such as ricin, small molecule toxins such as geldanamycin (Mandler et al (2000) Jour. of the Nat. Cancer Inst. 92(19):1573-1581; Mandler et al (2000) Bioorganic & Med. Chem. Letters 10:1025-1028; Mandler et al (2002) Bioconjugate Chem. 13:786-791), maytansinoids (EP 1391213; Liu et al., (1996) Proc. Natl. Acad. Sci. USA 93:8618-8623), and calicheamicin (Lode et al (1998) Cancer Res. 58:2928; Hinman et al (1993) Cancer Res. 53:3336-3342). The toxins may effect their cytotoxic and cytostatic effects by mechanisms including tubutin binding, DNA binding, or topoisomerase inhibition. Some cytotoxic drugs tend to be inactive or less active when conjugated to large antibodies or protein receptor ligands.

ZEVALIN® (ibritumomab tiuxetan, Biogen/Idec) is an antibody-radioisotope conjugate composed of a murine IgG1 kappa monoclonal antibody directed against the CD20 antigen found on the surface of normal and malignant B lymphocytes and 111In or 90Y radioisotope bound by a thiourea linker-chelator (Wiseman et al (2000) Eur. Jour. Nucl. Med. 27(7):766-77; Wiseman et al (2002) Blood 99(12):4336-42; Witzig et al (2002) J. Clin. Oncol. 20(10):2453-63; Witzig et al (2002) J. Clin. Oncol. 20(15):3262-69). Although ZEVALIN has activity against B-cell non-Hodgkin's Lymphoma (NHL), administration results in severe and prolonged cytopenias in most patients. MYLOTARG™ (gemtuzumab ozogamicin, Wyeth Pharmaceuticals), an antibody drug conjugate composed of a hu CD33 antibody linked to calicheamicin, was approved in 2000 for the treatment of acute myeloid leukemia by injection (Drugs of the Future (2000) 25(7):686; U.S. Pat. Nos. 4,970,198; 5,079,233; 5,585,089; 5,606,040; 5,693,762; 5,739,116; 5,767,285; 5,773,001). Cantuzumab mertansine (Immunogen, Inc.), an antibody drug conjugate composed of the huC242 antibody linked via the disulfide linker SPP to the maytansinoid drug moiety, DM1, is tested for the treatment of cancers that express CanAg, such as colon, pancreatic, gastric, and others. MLN-2704 (Millennium Pharm., BZL Biologics, Immunogen Inc.), an antibody drug conjugate composed of the anti-prostate specific membrane antigen (PSMA) monoclonal antibody linked to the maytansinoid drug moiety, DM1, is tested for the potential treatment of prostate tumors. The auristatin peptides, auristatin E (AE) and monomethylauristatin (MMAE), synthetic analogs of dolastatin, were conjugated to chimeric monoclonal antibodies cBR96 (specific to Lewis Y on carcinomas) and cAC10 (specific to CD30 on hematological malignancies) (Doronina et al (2003) Nature Biotechnology 21(7):778-784) and are under therapeutic development.

Chemotherapeutic agents useful in the generation of immunoconjugates are described herein (above). Enzymatically active toxins and fragments thereof that can be used include diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. See, e.g., WO 93/21232 published Oct. 28, 1993. A variety of radionuclides are available for the production of radioconjugated antibodies. Examples include 212Bi, 1311, 131In, 90Y, and 186Re. Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis-(p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., Science, 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026.

Conjugates of an antibody and one or more small molecule toxins, such as a calicheamicin, maytansinoids, dolostatins, aurostatins, a trichothecene, and CC1065, and the derivatives of these toxins that have toxin activity, are also contemplated herein.

### Maytansine and Maytansinoids

In some aspects, the immunoconjugate comprises an antibody Disclosed herein conjugated to one or more maytansinoid molecules.

Maytansinoids are mitotic inhibitors which act by inhibiting tubulin polymerization. Maytansine was first isolated from the east African shrub Maytenus serrata (U.S. Pat. No. 3,896,111). Subsequently, it was discovered that certain microbes also produce maytansinoids, such as maytansinol and C-3 maytansinol esters (U.S. Pat. No. 4,151,042). Synthetic maytansinol and derivatives and analogues thereof are disclosed, for example, in U.S. Pat. Nos. 4,137,230; 4,248,870; 4,256,746; 4,260,608; 4,265,814; 4,294,757; 4,307,016; 4,308,268; 4,308,269; 4,309,428; 4,313,946; 4,315,929; 4,317,821; 4,322,348; 4,331,598; 4,361,650; 4,364,866; 4,424,219; 4,450,254; 4,362,663; and 4,371,533.

Maytansinoid drug moieties are attractive drug moieties in antibody drug conjugates because they are: (i) relatively accessible to prepare by fermentation or chemical modification, derivatization of fermentation products, (ii) amenable to derivatization with functional groups suitable for conjugation through the non-disulfide linkers to antibodies, (iii) stable in plasma, and (iv) effective against a variety of tumor cell lines.

Exemplary aspects of maytansinoid drug moieties include: DM1; DM3; and DM4. Immunoconjugates containing maytansinoids, methods of making same, and their therapeutic use are disclosed, for example, in U.S. Pat. Nos. 5,208,020, 5,416,064 and European Patent EP 0 425 235 B1,. Liu et al., Proc. Natl. Acad. Sci. USA 93:8618-8623 (1996) described immunoconjugates comprising a maytansinoid designated DM1 linked to the monoclonal antibody C242 directed against human colorectal cancer. The conjugate was found to be highly cytotoxic towards cultured colon cancer cells, and showed antitumor activity in an in vivo tumor growth assay. Chari et al., Cancer Research 52:127-131 (1992) describe immunoconjugates in which a maytansinoid was conjugated via a disulfide linker to the murine antibody A7 binding to an antigen on human colon cancer cell lines, or to another murine monoclonal antibody TA.1 that binds the HER-2/neu oncogene. The cytotoxicity of the TA.1-maytansonoid conjugate was tested in vitro on the human breast cancer cell line SK-BR-3, which expresses 3×105 HER-2 surface antigens per cell. The drug conjugate achieved a degree of cytotoxicity similar to the free maytansinoid drug, which could be increased by increasing the number of maytansinoid molecules per antibody molecule. The A7-maytansinoid conjugate showed low systemic cytotoxicity in mice.

Antibody-maytansinoid conjugates can be prepared by chemically linking an antibody to a maytansinoid molecule without significantly diminishing the biological activity of either the antibody or the maytansinoid molecule. See, e.g., U.S. Pat. No. 5,208,020. An average of 3-4 maytansinoid molecules conjugated per antibody molecule has shown efficacy in enhancing cytotoxicity of target cells without negatively affecting the function or solubility of the antibody, although even one molecule of toxin/antibody would be expected to enhance cytotoxicity over the use of naked antibody. Maytansinoids are well known in the art and can be synthesized by known techniques or isolated from natural sources. Suitable maytansinoids are disclosed, for example, in U.S. Pat. No. 5,208,020 and in the other patents and nonpatent publications referred to hereinabove. Maytansinoids include
maytansinol and maytansinol analogues modified in the aromatic ring or at other positions of the maytansinol molecule, such as various maytansinol esters.

There are many linking groups known in the art for making antibody-maytansinoid conjugates, including, for example, those disclosed in U.S. Pat. No. 5,208,020 or EP Patent 0 425 235 B1, Chari et al., Cancer Research 52:127-131 (1992), and U.S. patent application Ser. No. 10/960,602, filed Oct. 8, 2004
. Antibody-maytansinoid conjugates comprising the linker component SMCC
may be prepared as disclosed in U.S. patent application Ser. No. 10/960,602, filed Oct. 8, 2004. The linking groups include disulfide groups, thioether groups, acid labile groups, photolabile groups, peptidase labile groups, or esterase labile groups, as disclosed in the above-identified patents. Additional linking groups are described and exemplified herein.

Conjugates of the antibody and maytansinoid may be made using a variety of bifunctional protein coupling agents such as N-succinimidyl-3-(2-pyridyldithio) propionate (SPDP), succinimidyl-4-(N-maleimidomethyl)cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis-(p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). Coupling agents include, but are not limited to, N-succinimidyl-3-(2-pyridyldithio) propionate (SPDP) (Carlsson et al., Biochem. J. 173:723-737 (1978)) and N-succinimidyl-4-(2-pyridylthio)pentanoate (SPP) to provide for a disulfide linkage.

The linker may be attached to the maytansinoid molecule at various positions, depending on the type of the link. For example, an ester linkage may be formed by reaction with a hydroxyl group using conventional coupling techniques. The reaction may occur at the C-3 position having a hydroxyl group, the C-14 position modified with hydroxymethyl, the C-15 position modified with a hydroxyl group, and the C-20 position having a hydroxyl group. In one embodiment, the linkage is formed at the C-3 position of maytansinol or a maytansinol analogue.

### Auristatins and Dolostatins

In some aspects, the immunoconjugate comprises an antibody
Disclosed herein conjugated to dolastatins or dolostatin peptidic analogs and derivatives, the auristatins (U.S. Pat. Nos. 5,635,483; 5,780,588). Dolastatins and auristatins have been shown to interfere with microtubule dynamics, GTP hydrolysis, and nuclear and cellular division (Woyke et al (2001) Antimicrob. Agents and Chemother. 45(12):3580-3584) and have anticancer (U.S. 5663149) and antifungal activity (Pettit et al (1998) Antimicrob. Agents Chemother. 42:2961-2965). The dolastatin or auristatin drug moiety may be attached to the antibody through the N (amino) terminus or the C (carboxyl) terminus of the peptidic drug moiety (WO 02/088172).

Exemplary auristatin aspects include the N-terminus linked monomethylauristatin drug moieties DE and DF, disclosed in "Monomethylvaline Compounds Capable of Conjugation to Ligands", U.S. Ser. No. 10/983,340, filed Nov. 5, 2004.

Exemplary auristatin aspects include MMAE and MMAF. Additional exemplary aspects comprising MMAE or MMAF and various linker components (described further herein) include Ab-MC-vc-PAB-MMAF, Ab-MC-vc-PAB-MMAE, Ab-MC-MMAE and Ab-MC-MMAF.

Typically, peptide-based drug moieties can be prepared by forming a peptide bond between two or more amino acids and/or peptide fragments. Such peptide bonds can be prepared, for example, according to the liquid phase synthesis method (see E. Schroder and K. Lübke, "The Peptides", volume 1, pp 76-136, 1965, Academic Press) that is well known in the field of peptide chemistry. The auristatin/dolastatin drug moieties may be prepared according to the methods of: U.S. Pat. Nos. 5,635,483; 5,780,588; Pettit et al (1989) J. Am. Chem. Soc. 111:5463-5465; Pettit et al (1998) Anti-Cancer Drug Design 13:243-277; Pettit, G. R., et al. Synthesis, 1996, 719-725; and Pettit et al (1996) J. Chem. Soc. Perkin Trans. 1 5:859-863. See also Doronina (2003) Nat Biotechnol 21(7):778-784; "Monomethylvaline Compounds Capable of Conjugation to Ligands", U.S. Ser. No. 10/983,340, filed Nov. 5, 2004 (disclosing, e.g., linkers and methods of
preparing monomethylvaline compounds such as MMAE and MMAF conjugated to linkers).

### Calicheamicin

In other aspects, the immunoconjugate comprises an antibody
Disclosed herein conjugated to one or more calicheamicin molecules. The calicheamicin family of antibiotics is capable of producing double-stranded DNA breaks at sub-picomolar concentrations. For the preparation of conjugates of the calicheamicin family, see U.S. Pat. Nos. 5,712,374, 5,714,586, 5,739,116, 5,767,285, 5,770,701, 5,770,710, 5,773,001, and 5,877,296 (all to American Cyanamid Company). Structural analogues of calicheamicin which may be used include γ1 I, α2 I, α3 I, N-acetyl-γ1 I, PSAG and θI 1 (Hinman et al., Cancer Research 53:3336-3342 (1993), Lode et al., Cancer Research 58:2925-2928 (1998) and the aforementioned U.S. patents to American Cyanamid). Another anti-tumor drug to which the antibody can be conjugated is QFA, which is an antifolate. Both calicheamicin and QFA have intracellular sites of action and do not readily cross the plasma membrane.
Therefore, cellular uptake of these agents through antibody mediated internalization greatly enhances their cytotoxic effects.

### Other Cytotoxic Agents

Other antitumor agents that can be conjugated to the antibodies disclosed herein include BCNU, streptozoicin, vincristine and 5-fluorouracil, the family of agents known collectively LL-E33288 complex described in U.S. Pat. Nos. 5,053,394, 5,770,710, as well as esperamicins (U.S. Pat. No. 5,877,296).

Enzymatically active toxins and fragments thereof which can be used include diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes. See, for example, WO 93/21232 published Oct. 28, 1993.

The present disclosure further contemplates an immunoconjugate formed between an antibody and a compound with nucleolytic activity (e.g., a ribonuclease or a DNA
endonuclease such as a deoxyribonuclease; DNase).

For selective destruction of the tumor, the antibody may comprise a highly radioactive atom. A variety of radioactive isotopes are available for the production of radioconjugated antibodies. Examples include At211, 1131, 1125, Y90, Re186, Re188, Sm153, Bi212, P32, Pb212 and radioactive isotopes of Lu. When the conjugate is used for detection, it may comprise a radioactive atom for scintigraphic studies, for example Tc99m or 1123, or a spin label for nuclear magnetic resonance (NMR) imaging (also known as magnetic resonance imaging, MRI), such as iodine-123 again, iodine-131, indium-111, fluorine-19, carbon-13, nitrogen-15, oxygen-17, gadolinium, manganese or iron.

The radio- or other labels may be incorporated in the conjugate in known ways. For example, the peptide may be biosynthesized or may be synthesized by chemical amino acid synthesis using suitable amino acid precursors involving, for example, fluorine-19 in place of hydrogen. Labels such as Tc99m or 1123, Re186, Re188 and In111 can be attached via a cysteine residue in the peptide. Yttrium-90 can be attached via a lysine residue. The IODOGEN method (Fraker et al (1978) Biochem. Biophys. Res. Commun. 80: 49-57) can be used to incorporate iodine-123. "Monoclonal Antibodies in Immunoscintigraphy" (Chatal, CRC Press 1989) describes other methods in detail.

Conjugates of the antibody and cytotoxic agent may be made using a variety of bifunctional protein coupling agents such as N-succinimidyl-3-(2-pyridyldithio) propionate (SPDP), succinimidyl-4-(N-maleimidomethyl)cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis-(p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., Science 238:1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026. The linker may be a "cleavable linker" facilitating release of the cytotoxic drug in the cell. For example, an acid-labile linker, peptidase-sensitive linker, photolabile linker, dimethyl linker or disulfide-containing linker (Chari et al., Cancer Research 52:127-131 (1992); U.S. Pat. No. 5,208,020) may be used.

The compounds disclosed herein expressly contemplate, but are not limited to, ADC prepared with cross-linker reagents: BMPS, EMCS, GMBS, HBVS, LC-SMCC, MBS,
MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate) which are commercially available (e.g., from Pierce Biotechnology,
Inc., Rockford, Ill., U.S.A). See pages 467-498, 2003-2004 Applications Handbook and Catalog.

### Preparation of Antibody Drug Conjugates

In the antibody drug conjugates (ADC) disclosed herein, an antibody (Ab) is conjugated to one or more drug moieties (D), e.g. about 1 to about 20 drug moieties per antibody, through a linker (L). The ADC of Formula I may be prepared by several routes, employing organic chemistry reactions, conditions, and reagents known to those skilled in the art, including: (1) reaction of a nucleophilic group of an antibody with a bivalent linker reagent, to form Ab-L, via a covalent bond, followed by reaction with a drug moiety D; and (2) reaction of a nucleophilic group of a drug moiety with a bivalent linker reagent, to form D-L, via a covalent bond, followed by reaction with the nucleophilic group of an antibody. Additional methods for preparing ADC are described herein.

### Ab-(L-D)p I

The linker may be composed of one or more linker components. Exemplary linker components include 6-maleimidocaproyl ("MC"), maleimidopropanoyl ("MP"), valine-citrulline ("val-cit"), alanine-phenylalanine ("ala-phe"), p-aminobenzyloxycarbonyl ("PAB"), N-Succinimidyl 4-(2-pyridylthio) pentanoate ("SPP"), N-Succinimidyl 4-(N-maleimidomethyl)cyclohexane-1 carboxylate ("SMCC"), and N-Succinimidyl (4-iodo-acetyl) aminobenzoate ("SIAB"). Additional linker components are known in the art and some are described herein.

In some aspects, the linker may comprise amino acid residues. Exemplary amino acid linker components include a dipeptide, a tripeptide, a tetrapeptide or a pentapeptide. Exemplary dipeptides include: valine-citrulline (vc or val-cit), alanine-phenylalanine (af or ala-phe). Exemplary tripeptides include: glycine-valine-citrulline (gly-val-cit) and glycine-glycine-glycine (gly-gly-gly). Amino acid residues which comprise an amino acid linker component include those occurring naturally, as well as minor amino acids and non-naturally occurring amino acid analogs, such as citrulline. Amino acid linker components can be designed and optimized in their selectivity for enzymatic cleavage by a particular enzyme, for example, a tumor-associated protease, cathepsin B, C and D, or a plasmin protease.

Exemplary linker component structures are shown below (wherein the wavy line indicates sites of covalent attachment to other components of the ADC):
Figure US08133488-20120313-C00006
Additional exemplary linker components and abbreviations include (wherein the antibody (Ab) and linker are depicted, and p is 1 to about 8):
Figure US08133488-20120313-C00007
Nucleophilic groups on antibodies include: (i) N-terminal
amine groups, (ii) side chain amine groups, e.g. lysine, (iii) side chain thiol groups, e.g. cysteine, and (iv) sugar hydroxyl or amino groups where the antibody is glycosylated. Amine, thiol, and hydroxyl groups are nucleophilic and capable of reacting to form covalent bonds with electrophilic groups on linker moieties and linker reagents including: (i) active esters such as NHS esters, HOBt esters, haloformates, and acid halides; (ii) alkyl and benzyl halides such as haloacetamides; (iii) aldehydes, ketones, carboxyl, and maleimide groups. Certain antibodies have reducible interchain disulfides, i.e. cysteine bridges. Antibodies may be made reactive for conjugation with linker reagents by treatment with a reducing agent such as DTT (dithiothreitol). Each cysteine bridge will thus form, theoretically, two reactive thiol nucleophiles. Additional nucleophilic groups can be introduced into antibodies through the reaction of lysines with 2-iminothiolane (Traut's reagent) resulting in conversion of an amine into a thiol. Reactive thiol groups may be introduced into the antibody (or fragment thereof) by introducing one, two, three, four, or more cysteine residues (e.g., preparing mutant antibodies comprising one or more non-native cysteine amino acid residues).

Antibody drug conjugates disclosed herein may also be produced by modification of the antibody to introduce electrophilic moieties, which can react with nucleophilic substituents on the linker reagent or drug. The sugars of glycosylated antibodies may be oxidized, e.g. with periodate oxidizing reagents, to form aldehyde or ketone groups which may react with the amine group of linker reagents or drug moieties. The resulting imine Schiff base groups may form a stable linkage, or may be reduced, e.g. by borohydride reagents to form stable amine linkages. In one aspect, reaction of the carbohydrate portion of a glycosylated antibody with either galactose oxidase or sodium meta-periodate may yield carbonyl (aldehyde and ketone) groups in the protein that can react with appropriate groups on the drug (Hermanson, Bioconjugate Techniques). In another aspect, proteins containing N-terminal serine or threonine residues can react with sodium meta-periodate, resulting in production of an aldehyde in place of the first amino acid (Geoghegan & Stroh, (1992) Bioconjugate Chem. 3:138-146; U.S. Pat. No. 5,362,852). Such aldehyde can be reacted with a drug moiety or linker nucleophile.

Likewise, nucleophilic groups on a drug moiety include: amine,
thiol, hydroxyl, hydrazide, oxime, hydrazine, thiosemicarbazone, hydrazine carboxylate, and arylhydrazide groups capable of reacting to form covalent bonds with electrophilic groups on linker moieties and linker reagents including: (i) active esters such as NHS esters, HOBt esters, haloformates, and acid halides; (ii) alkyl and benzyl halides such as haloacetamides; (iii) aldehydes, ketones, carboxyl, and maleimide groups.

Alternatively, a fusion protein comprising the antibody and cytotoxic agent may be made, e.g., by recombinant techniques or peptide synthesis. The length of DNA may comprise respective regions encoding the two portions of the conjugate either adjacent to one another or separated by a region encoding a linker peptide which does not destroy the desired properties of the conjugate.

In yet another aspect, the antibody may be conjugated to a "receptor" (such streptavidin) for utilization in tumor pre-targeting wherein the antibody-receptor conjugate is administered to the patient, followed by removal of unbound conjugate from the circulation using a clearing agent and then administration of a "ligand" (e.g., avidin) which is conjugated to a cytotoxic agent (e.g., a radionucleotide).

Antibody (Ab)-MC-MMAE may be prepared by conjugation of any of the antibodies provided herein with MC-MMAE as follows. Antibody, dissolved in 500 mM sodium borate and 500 mM sodium chloride at pH 8.0 is treated with an excess of 100 mM dithiothreitol (DTT). After incubation at 37° C. for about 30 minutes, the buffer is exchanged by elution over Sephadex G25 resin and eluted with PBS with 1 mM DTPA. The thiol/Ab value is checked by determining the reduced antibody concentration from the absorbance at 280 nm of the solution and the thiol concentration by reaction with DTNB (Aldrich, Milwaukee, Wis.) and determination of the absorbance at 412 nm. The reduced antibody dissolved in PBS is chilled on ice. The drug linker reagent, maleimidocaproyl-monomethyl auristatin E (MMAE), i.e. MC-MMAE, dissolved in DMSO, is diluted in acetonitrile and water at known concentration, and added to the chilled reduced antibody 2H9 in PBS. After about one hour, an excess of maleimide is added to quench the reaction and cap any unreacted antibody thiol groups. The reaction mixture is concentrated by centrifugal ultrafiltration and 2H9-MC-MMAE is purified and desalted by elution through G25 resin in PBS, filtered through 0.2 µm filters under sterile conditions, and frozen for storage.

Antibody-MC-MMAF may be prepared by conjugation of any of the antibodies provided herein with MC-MMAF following the protocol provided for preparation of Ab-MC-MMAE.

Antibody-MC-val-cit-PAB-MMAE is prepared by conjugation of any of the antibodies provided herein with MC-val-cit-PAB-MMAE following the protocol provided for preparation of Ab-MC-MMAE.

Antibody-MC-val-cit-PAB-MMAF is prepared by conjugation of any of the antibodies provided herein with MC-val-cit-PAB-MMAF following the protocol provided for preparation of Ab-MC-MMAE.

Antibody-SMCC-DM1 is prepared by conjugation of any of the antibodies provided herein with SMCC-DM1 as follows. Purified antibody is derivatized with (Succinimidyl 4-(N-maleimidomethyl)cyclohexane-1-carboxylate (SMCC, Pierce Biotechnology, Inc) to introduce the SMCC linker. Specifically, antibody is treated at 20 mg/mL in 50 mM potassium phosphate/50 mM sodium chloride/2 mM EDTA, pH 6.5 with 7.5 molar equivalents of SMCC (20 mM in DMSO, 6.7 mg/mL). After stirring for 2 hours under argon at ambient temperature, the reaction mixture is filtered through a Sephadex G25 column equilibrated with 50 mM potassium phosphate/50 mM sodium chloride/2 mM EDTA, pH 6.5. Antibody-containing fractions are pooled and assayed.

Antibody-SMCC prepared thusly is diluted with 50 mM potassium phosphate/50 mM sodium chloride/2 mM EDTA, pH 6.5, to a final concentration of about 10 mg/ml, and reacted with a 10 mM solution of DM1 in dimethylacetamide. The reaction is stirred at ambient temperature under argon for 16.5 hours. The conjugation reaction mixture is filtered through a Sephadex G25 gel filtration column (1.5×4.9 cm) with 1×PBS at pH 6.5. The DM1 drug to antibody ratio (p) may be about 2 to 5, as measured by the absorbance at 252 nm and at 280 nm.

Ab-SPP-DM1 is prepared by conjugation of any of the antibodies provided herein with SPP-DM1 as follows. Purified antibody is derivatized with N-succinimidyl-4-(2-pyridylthio)pentanoate to introduce dithiopyridyl groups. Antibody (376.0 mg, 8 mg/mL) in 44.7 mL of 50 mM potassium phosphate buffer (pH 6.5) containing NaCl (50 mM) and EDTA (1 mM) is treated with SPP (5.3 molar equivalents in 2.3 mL ethanol). After incubation for 90 minutes under argon at ambient temperature, the reaction mixture is gel filtered through a Sephadex G25 column equilibrated with a 35 mM sodium citrate, 154 mM NaCl, 2 mM EDTA buffer. Antibody-containing fractions were pooled and assayed. The degree of modification of the antibody is determined as described above.

Antibody-SPP-Py (about 10 µmoles of releasable 2-thiopyridine groups) is diluted with the above 35 mM sodium citrate buffer, pH 6.5, to a final concentration of about 2.5 mg/mL. DM1 (1.7 equivalents, 17 µmoles) in 3.0 mM dimethylacetamide (DMA, 3% v/v in the final reaction mixture) is then added to the antibody solution. The reaction proceeds at ambient temperature under argon for about 20 hours. The reaction is loaded on a Sephacryl S300 gel filtration column (5.0 cm×90.0 cm, 1.77 L) equilibrated with 35 mM sodium citrate, 154 mM NaCl, pH 6.5. The flow rate may be about 5.0 mL/min and 65 fractions (20.0 mL each) are collected. The number of DM1 drug molecules linked per antibody molecule (p') is determined by measuring the absorbance at 252 nm and 280 nm, and may be about 2 to 4 DM1 drug moieties per 2H9 antibody.

Antibody-BMPEO-DM1 is prepared by conjugation of any of the antibodies provided herein with BMPEO-DM1 as follows. The antibody is modified by the bis-maleimido reagent BM(PEO)4 (Pierce Chemical), leaving an unreacted maleimido group on the surface of the antibody. This may be accomplished by dissolving BM(PEO)4 in a 50% ethanol/water mixture to a concentration of 10 mM and adding a tenfold molar excess to a solution containing antibody in phosphate buffered saline at a concentration of approximately 1.6 mg/ml (10 micromolar) and allowing it to react for 1 hour to form an antibody-linker intermediate, 2H9-BMPEO. Excess BM(PEO)4 is removed by gel filtration (HiTrap column, Pharmacia) in 30 mM citrate, pH 6 with 150 mM NaCl buffer. An approximate 10 fold molar excess DM1 is dissolved in dimethyl acetamide (DMA) and added to the 2H9-BMPEO intermediate. Dimethyl formamide (DMF) may also be employed to dissolve the drug moiety reagent. The reaction mixture is allowed to react overnight before gel filtration or dialysis into PBS to remove unreacted DM1. Gel filtration on S200 columns in PBS is used to remove high molecular weight aggregates and to furnish purified 2H9-BMPEO-DM1.

### Antibody Derivatives

Antibodies disclosed herein can be further modified to contain additional nonproteinaceous moieties that are known in the art and readily available. In one aspect, the moieties suitable for derivatization of the antibody are water soluble polymers. Nonlimiting examples of water soluble polymers include polyethylene glycol (PEG), copolymers of ethylene glycol/propylene glycol, carboxymethylcellulose, dextran, polyvinyl alcohol, polyvinyl pyrrolidone, poly-1,3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer is attached, the polymers can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another aspect, conjugates of an antibody and nonproteinaceous moiety that may be selectively heated by exposure to radiation are provided. In one aspect, the nonproteinaceous moiety is a carbon nanotube (Kam et al., Proc. Natl. Acad. Sci. 102: 11600-11605 (2005)). The radiation may be of any wavelength, and includes
wavelengths that do not harm ordinary cells, but which heat the nonproteinaceous moiety to a temperature at which cells proximal to the antibody-nonproteinaceous moiety are killed.

### Pharmaceutical Formulations

Therapeutic formulations comprising an antibody disclosed herein are prepared for storage by mixing the antibody having the desired degree of purity with optional
physiologically acceptable carriers, excipients or stabilizers (Remington's Pharmaceutical Sciences 16th edition, Osol, A. Ed. (1980)), in the form of aqueous solutions, lyophilized or other dried formulations. Acceptable carriers, excipients, or stabilizers are nontoxic to recipients at the dosages and concentrations employed, and include buffers such as phosphate, citrate, histidine and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN™, PLURONICS™ or polyethylene glycol (PEG).

The formulation herein may also contain more than one active compound as necessary for the particular indication being treated, including, those with
complementary activities that do not adversely affect each other. Such molecules are suitably present in combination in amounts that are effective for the purpose intended.

The active ingredients may also be entrapped in microcapsule prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The formulations to be used for in vivo administration must be sterile. This is readily accomplished by filtration through sterile filtration membranes.

Sustained-release preparations may be prepared. Suitable examples of sustained-release preparations include semipermeable matrices of solid hydrophobic polymers containing the immunoglobulin of the invention, which matrices are in the form of shaped articles, e.g., films, or microcapsule. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated immunoglobulins remain in the body for a long time, they may denature or aggregate as a result of exposure to moisture at 37° C., resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

### Uses

An antibody disclosed herein may be used in, for example, in vitro, ex vivo and in vivo therapeutic methods. Antibodies disclosed herein can be used as an antagonist to partially or fully block the specific antigen activity in vitro, ex vivo and/or in vivo. Moreover, at least some of the antibodies disclosed herein can neutralize antigen activity from other species.
Accordingly, antibodies disclosed herein can be used to inhibit a specific antigen activity, e.g., in a cell culture containing the antigen, in human subjects or in other mammalian subjects having the antigen with which an antibody disclosed herein cross-reacts (e.g. chimpanzee, baboon, marmoset, cynomolgus and rhesus, pig or mouse). In one aspect, an antibody disclosed herein can be used for inhibiting antigen activities by contacting the antibody with the antigen such that antigen activity is inhibited. In one aspect, the antigen is a human protein molecule.

In one aspect, an antibody disclosed herein can be used in a method for inhibiting an antigen in a subject suffering from a disorder in which the antigen activity is detrimental, comprising administering to the subject an antibody disclosed herein such that the antigen activity in the subject is inhibited. In one aspect, the antigen is a human protein molecule and the subject is a human subject. Alternatively, the subject can be a mammal expressing the antigen with which an antibody disclosed herein binds. Still further the subject can be a mammal into which the antigen has been introduced (e.g., by administration of the antigen or by expression of an antigen transgene). An antibody disclosed herein can be administered to a human subject for therapeutic purposes. Moreover, an antibody disclosed herein can be administered to a non-human mammal expressing an antigen with which the antibody cross-reacts (e.g., a primate, pig or mouse) for veterinary purposes or as an animal model of human disease. Regarding the latter, such animal models may be useful for evaluating the therapeutic efficacy of antibodies disclosed herein (e.g., testing of dosages and time courses of administration). Antibodies disclosed herein can be used to treat, inhibit, delay progression of, prevent/delay recurrence of, ameliorate, or prevent diseases, disorders or conditions associated with abnormal expression and/or activity of SSEA-3/SSEA-4/GLOBO Hs and SSEA-3/SSEA-4/GLOBO Hated proteins, including cancer, muscular disorders, ubiquitin-pathway-related genetic disorders, immune/inflammatory disorders, neurological disorders, and other ubiquitin pathway-related disorders.

In one aspect, a blocking antibody disclosed herein is specific for a SSEA-3/SSEA-4/GLOBO H.

In certain aspects, an immunoconjugate comprising an antibody
disclosed herein conjugated with a cytotoxic agent is administered to the patient. In some aspects, the immunoconjugate and/or antigen to which it is bound is/are internalized by cells expressing one or more proteins on their cell surface which are associated with SSEA-3/SSEA-4/GLOBO H, resulting in increased therapeutic efficacy of the immunoconjugate in killing the target cell with which it is associated. In one aspect, the cytotoxic agent targets or interferes with nucleic acid in the target cell. Examples of such cytotoxic agents include any of the chemotherapeutic agents noted herein (such as a maytansinoid or a calicheamicin), a radioactive isotope, or a ribonuclease or a DNA endonuclease.

Antibodies disclosed herein can be used either alone or in combination with other compositions in a therapy. For instance, an antibody disclosed herein may be co-administered with another antibody, and/or adjuvant/therapeutic agents (e.g., steroids). For instance, an antibody disclosed herein may be combined with an anti-inflammatory and/or antiseptic in a treatment scheme, e.g. in treating any of the diseases described herein, including cancer, muscular disorders, ubiquitin-pathway-related genetic disorders, immune/inflammatory
disorders, neurological disorders, and other ubiquitin pathway-related disorders. Such combined therapies noted above include combined administration (where the two or more agents are included in the same or separate formulations), and separate administration, in which case, administration of the antibody disclosed herein can occur prior to, and/or following, administration of the adjunct therapy or therapies.

An antibody disclosed herein (and adjunct therapeutic agent) can be administered by any suitable means, including parenteral, subcutaneous, intraperitoneal, intrapulmonary, and intranasal, and, if desired for local treatment, intralesional administration. Parenteral infusions include intramuscular, intravenous, intraarterial, intraperitoneal, or subcutaneous
administration. In addition, the antibody is suitably administered by pulse infusion,
particularly with declining doses of the antibody. Dosing can be by any suitable route, e.g. by injections, such as intravenous or subcutaneous injections, depending in part on whether the administration is brief or chronic.

The location of the binding target of an antibody disclosed herein may be taken into consideration in preparation and administration of the antibody. When the binding target is an intracellular molecule, certain aspects of the disclosure provide for the antibody or antigen-binding fragment thereof to be introduced into the cell where the binding target is located. In one aspect, an antibody disclosed herein can be expressed intracellularly as an intrabody. The term "intrabody," as used herein, refers to an antibody or antigen-binding portion thereof that is expressed intracellularly and that is capable of selectively binding to a
target molecule, as described in Marasco, Gene Therapy 4: 11-15 (1997); Kontermann, Methods 34: 163-170 (2004); U.S. Pat. Nos. 6,004,940 and 6,329,173; U.S. Patent Application Publication No. 2003/0104402, and PCT Publication No. WO2003/077945. Intracellular expression of an intrabody is effected by introducing a nucleic acid encoding the desired antibody or antigen-binding portion thereof (lacking the wild-type leader sequence and secretory signals normally associated with the gene encoding that antibody or antigen-binding fragment) into a target cell. Any standard method of introducing nucleic acids into a cell may be used, including, but not limited to, microinjection, ballistic injection, electroporation, calcium phosphate precipitation, liposomes, and transfection with retroviral, adenoviral, adeno-associated viral and vaccinia vectors carrying the nucleic acid of interest. One or more nucleic acids encoding all or a portion of an anti-SSEA-3/SSEA-4/GLOBO H antibody disclosed herein can be delivered to a target cell, such that one or more intrabodies are expressed which are capable of intracellular binding to a SSEA-3/SSEA-4/GLOBO H and modulation of one or more SSEA-3/SSEA-4/GLOBO H-mediated cellular pathways.

In another aspect, internalizing antibodies are provided. Antibodies can possess certain characteristics that enhance delivery of antibodies into cells, or can be modified to possess such characteristics. Techniques for achieving this are known in the art. For example, cationization of an antibody is known to facilitate its uptake into cells (see, e.g., U.S. Pat. No. 6,703,019). Lipofections or liposomes can also be used to deliver the antibody into cells. Where antibody fragments are used, the smallest inhibitory fragment that specifically binds to the binding domain of the target protein is generally advantageous. For example, based upon the variable-region sequences of an antibody, peptide molecules can be designed that retain the ability to bind the target protein sequence. Such peptides can be synthesized chemically and/or produced by recombinant DNA technology. See, e.g., Marasco et al., Proc. Natl. Acad. Sci. USA, 90: 7889-7893 (1993).

Entry of modulator polypeptides into target cells can be enhanced by methods known in the art. For example, certain sequences, such as those derived from HIV Tat or the Antennapedia homeodomain protein are able to direct efficient uptake of heterologous proteins across cell membranes. See, e.g., Chen et al., Proc. Natl. Acad. Sci. USA (1999), 96:4325-4329.

When the binding target is located in the brain, certain aspects of the Disclosure provide for the antibody or antigen-binding fragment thereof to traverse the blood-brain barrier. Certain neurodegenerative diseases are associated with an increase in permeability of the blood-brain barrier, such that the antibody or antigen-binding fragment can be readily introduced to the brain. When the blood-brain barrier remains intact, several art-known approaches exist for transporting molecules across it, including physical
methods, lipid-based methods, and receptor and channel-based methods.

Physical methods of transporting the antibody or antigen-binding fragment across the blood-brain barrier include circumventing the blood-brain barrier
entirely, or by creating openings in the blood-brain barrier. Circumvention methods include direct injection into the brain (see, e.g., Papanastassiou et al., Gene Therapy 9: 398-406 (2002)), interstitial infusion/convection-enhanced delivery (see, e.g., Bobo et al., Proc. Natl. Acad. Sci. USA 91: 2076-2080 (1994)), and implanting a delivery device in the brain (see, e.g., Gill et al., Nature Med. 9: 589-595 (2003); and Gliadel Wafers™, Guildford Pharmaceutical). Methods of creating openings in the barrier include ultrasound (see, e.g., U.S. Patent Publication No. 2002/0038086), osmotic pressure (e.g., by administration of hypertonic mannitol (Neuwelt, E. A., Implication of the Blood-Brain Barrier and its Manipulation, Vols 1 & 2, Plenum Press, N.Y. (1989))), permeabilization by, e.g., bradykinin or permeabilizer A-7 (see, e.g., U.S. Pat. Nos. 5,112,596, 5,268,164, 5,506,206, and 5,686,416), and transfection of neurons that straddle the blood-brain barrier with vectors containing genes encoding the antibody or antigen-binding fragment (see, e.g., U.S. Patent Publication No. 2003/0083299).

Lipid-based methods of transporting the antibody or antigen-binding fragment across the blood-brain barrier include encapsulating the antibody or antigen-binding fragment in liposomes that are coupled to antibody binding fragments that bind to receptors on the vascular endothelium of the blood-brain barrier (see, e.g., U.S. Patent Application Publication No. 20020025313), and coating the antibody or antigen-binding fragment in low-density lipoprotein particles (see, e.g., U.S. Patent Application Publication No. 20040204354) or apolipoprotein E (see, e.g., U.S. Patent Application Publication No. 20040131692).

Receptor and channel-based methods of transporting the antibody or antigen-binding fragment across the blood-brain barrier include using glucocorticoid blockers to increase permeability of the blood-brain barrier (see, e.g., U.S. Patent Application Publication Nos. 2002/0065259, 2003/0162695, and 2005/0124533); activating potassium channels (see, e.g., U.S. Patent Application Publication No. 2005/0089473), inhibiting ABC drug transporters (see, e.g., U.S. Patent Application Publication No. 2003/0073713); coating antibodies with a transferrin and modulating activity of the one or more transferrin receptors (see, e.g., U.S. Patent Application Publication No. 2003/0129186), and cationizing the antibodies (see, e.g., U.S. Pat. No. 5,004,697).

The antibody composition disclosed herein would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The antibody need not be, but is optionally formulated with one or more agents currently used to prevent or treat the disorder in question. The effective amount of such other agents depends on the amount of antibodies disclosed herein present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of an antibody disclosed herein (when used alone or in combination with other agents such as chemotherapeutic agents) will depend on the type of disease to be treated, the type of antibody, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments. Depending on the type and severity of the disease, about 1 µg/kg to 15 mg/kg (e.g. 0.1 mg/kg-10 mg/kg) of antibody can be an initial candidate dosage for administration to the patient, whether, for example, by one or more separate administrations, or by continuous infusion. One typical daily dosage might range from about 1 µg/kg to 100 mg/kg or more, depending on the factors mentioned above. For repeated administrations over several days or longer, depending on the condition, the treatment would generally be sustained until a desired suppression of disease symptoms occurs. One exemplary dosage of the antibody would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (e.g. such that the patient receives from about two to about twenty, or e.g. about six doses of the antibody). An initial higher loading dose, followed by one or more lower doses may be administered. An exemplary dosing regimen comprises administering an initial loading dose of about 4 mg/kg, followed by a weekly maintenance dose of about 2 mg/kg of the antibody. However, other dosage regimens may be useful. The progress of this therapy is easily monitored by conventional techniques and assays.

### Articles of Manufacture

In another aspect of the disclosure, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, etc. The containers may be formed from a variety of materials such as glass or plastic. The container holds a composition which is by itself or when combined with another composition effective for treating, preventing and/or diagnosing the condition and may have a sterile access port (for example the container may be an intravenous solution bag or a vial having a stopper pierceable by a hypodermic injection needle). At least one active agent in the composition is an antibody of the invention. The label or package insert indicates that the composition is used for treating the condition of choice. Moreover, the article of manufacture may comprise (a) a first container with a composition contained therein, wherein the composition comprises an antibody disclosed herein; and (b) a second container with a composition contained therein, wherein the composition comprises a further cytotoxic or otherwise therapeutic agent. The article of manufacture in this aspect of the
disclosure may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

The following are examples of the methods and compositions of the disclosure. It is understood that various other embodiments may be practiced, given the general description provided above.

In some aspects, the provided glycan conjugates, immunogenic compositions or vaccines are useful in treating, or diagnosing a cancer, including
acoustic neuroma, adenocarcinoma, adrenal gland cancer, anal cancer, angiosarcoma (*e.g*., lymphangiosarcoma, lymphangioendotheliosarcoma, hemangiosarcoma), appendix cancer, benign monoclonal gammopathy, biliary cancer (*e.g*., cholangiocarcinoma), bladder cancer, breast cancer (*e.g*., adenocarcinoma of the breast, papillary carcinoma of the breast, mammary cancer, medullary carcinoma of the breast), brain cancer (*e.g*., meningioma; glioma, *e.g*., astrocytoma, oligodendroglioma; medulloblastoma), bronchus cancer, carcinoid tumor, cervical cancer (*e.g.*, cervical adenocarcinoma), choriocarcinoma, chordoma, craniopharyngioma, colorectal cancer (*e.g.*, colon cancer, rectal cancer, colorectal adenocarcinoma), epithelial carcinoma, ependymoma, endotheliosarcoma (*e.g.*, Kaposi's sarcoma, multiple idiopathic hemorrhagic sarcoma), endometrial cancer (*e.g.*, uterine cancer, uterine sarcoma), esophageal cancer (*e.g.*, adenocarcinoma of the esophagus, Barrett's adenocarinoma), Ewing sarcoma, eye cancer (*e.g.*, intraocular melanoma, retinoblastoma), familiar hypereosinophilia, gall bladder cancer, gastric cancer (*e.g.*, stomach adenocarcinoma), gastrointestinal stromal tumor (GIST), head and neck cancer (*e.g.*, head and neck squamous cell carcinoma, oral cancer (*e.g.*, oral squamous cell carcinoma (OSCC), throat cancer (*e.g.*, laryngeal cancer, pharyngeal cancer, nasopharyngeal cancer, oropharyngeal cancer)), hematopoietic cancers (*e.g*., leukemia such as acute lymphocytic leukemia (ALL) (*e.g*., B-cell ALL, T-cell ALL), acute myelocytic leukemia (AML) (*e.g*., B-cell AML, T-cell AML), chronic myelocytic leukemia (CML) (*e.g*., B-cell CML, T-cell CML), and chronic lymphocytic leukemia (CLL) (*e.g*., B-cell CLL, T-cell CLL); lymphoma such as Hodgkin lymphoma (HL) (*e.g*., B-cell HL, T-cell HL) and non-Hodgkin lymphoma (NHL) (*e.g*., B-cell NHL such as diffuse large cell lymphoma (DLCL) (*e.g*., diffuse large B-cell lymphoma (DLBCL)), follicular lymphoma, chronic lymphocytic leukemia/small lymphocytic lymphoma (CLL/SLL), mantle cell lymphoma (MCL), marginal zone B-cell lymphomas (*e.g.*, mucosa-associated lymphoid tissue (MALT) lymphomas, nodal marginal zone B-cell lymphoma, splenic marginal zone B-cell lymphoma), primary mediastinal B-cell lymphoma, Burkitt lymphoma, lymphoplasmacytic lymphoma (*i.e*., "Waldenstrom's macroglobulinemia"), hairy cell leukemia (HCL), immunoblastic large cell lymphoma, precursor B-lymphoblastic lymphoma and primary central nervous system (CNS) lymphoma; and T-cell NHL such as precursor T-lymphoblastic lymphoma/leukemia, peripheral T-cell lymphoma (PTCL) (*e.g.*, cutaneous T-cell lymphoma (CTCL) (*e.g.*, mycosis fungiodes, Sezary syndrome), angioimmunoblastic T-cell lymphoma, extranodal natural killer T-cell lymphoma, enteropathy type T-cell lymphoma, subcutaneous panniculitis-like T-cell lymphoma, anaplastic large cell lymphoma); a mixture of one or more leukemia/lymphoma as described above; and multiple myeloma (MM)), heavy chain disease (*e.g.*, alpha chain disease, gamma chain disease, mu chain disease), hemangioblastoma, inflammatory myofibroblastic tumors, immunocytic amyloidosis, kidney cancer (*e.g.*, nephroblastoma *a.k.a.* Wilms' tumor, renal cell carcinoma), liver cancer (*e.g.*, hepatocellular cancer (HCC), malignant hepatoma), lung cancer (*e.g.*, bronchogenic carcinoma, small cell lung cancer (SCLC), non-small cell lung cancer (NSCLC), adenocarcinoma of the lung), leiomyosarcoma (LMS), mastocytosis (*e.g.*, systemic mastocytosis), myelodysplastic syndrome (MDS), mesothelioma, myeloproliferative disorder (MPD) (*e.g.*, polycythemia Vera (PV), essential thrombocytosis (ET), agnogenic myeloid metaplasia (AMM), a.k.a. myelofibrosis (MF), chronic idiopathic myelofibrosis, chronic myelocytic leukemia (CML), chronic neutrophilic leukemia (CNL), hypereosinophilic syndrome (HES)), neuroblastoma, neurofibroma (*e.g.*, neurofibromatosis (NF) type 1 or type 2, schwannomatosis), neuroendocrine cancer (*e.g.*, gastroenteropancreatic neuroendoctrine tumor (GEP-NET), carcinoid tumor), osteosarcoma, ovarian cancer (*e.g.*, cystadenocarcinoma, ovarian embryonal carcinoma, ovarian adenocarcinoma), papillary adenocarcinoma, pancreatic cancer (*e.g.*, pancreatic andenocarcinoma, intraductal papillary mucinous neoplasm (IPMN), islet cell tumors), penile cancer (*e.g.*, Paget's disease of the penis and scrotum), pinealoma, primitive neuroectodermal tumor (PNT), prostate cancer (*e.g.*, prostate adenocarcinoma), rectal cancer, rhabdomyosarcoma, salivary gland cancer, skin cancer (*e.g.*, squamous cell carcinoma (SCC), keratoacanthoma (KA), melanoma, basal cell carcinoma (BCC)), small bowel cancer (*e.g.*, appendix cancer), soft tissue sarcoma (*e.g.*, malignant fibrous histiocytoma (MFH), liposarcoma, malignant peripheral nerve sheath tumor (MPNST), chondrosarcoma, fibrosarcoma, myxosarcoma), sebaceous gland carcinoma, sweat gland carcinoma, synovioma, testicular cancer (*e.g.*, seminoma, testicular embryonal carcinoma), thyroid cancer (*e.g.*, papillary carcinoma of the thyroid, papillary thyroid carcinoma (PTC), medullary thyroid cancer), urethral cancer, vaginal cancer and vulvar cancer (*e.g.*, Paget's disease of the vulva). In certain embodiments, the provided glycan conjugates, immunogenic compositions or vaccines are useful for treating brain cancer, lung cancer, breast cancer, oral cancer, esophagus cancer, stomach cancer, liver cancer, bile duct cancer, pancreas cancer, colon cancer, kidney cancer, bone cancer, skin cancer, cervix cancer, ovary cancer, and prostate cancer.

To perform the treatment methods described herein, an effective amount of any of the glycan conjugates or immunogenic compositions or vaccines described herein may be administered to a subject in need of the treatment via a suitable route, as described above. The subject, such as a human subject, can be a patient having cancer, suspected of having cancer, or susceptible to cancer. The amount of the glycan conjugate or immunogenic composition administered to the subject may be effective in eliciting immune responses specific to the glycan moiety in the conjugate or composition. In some aspects, the amount of the glycan conjugate or immunogenic composition is sufficient to elicit immune responses leading to the inhibition of cancer growth and/or reduction of tumor mass. In other aspects, the amount of the glycan conjugate or immunogenic composition may be effective in delaying the onset of the target cancer or reducing the risk for developing the cancer. The exact amount of the provided glycan conjugates, immunogenic compositions or vaccines required to achieve an effective amount will vary from subject to subject, depending, for example, on species, age, and general condition of a subject, severity of the side effects or disorder, identity of the particular compound(s), mode of administration, and the like. The desired dosage can be delivered three times a day, two times a day, once a day, every other day, every third day, every week, every two weeks, every three weeks, or every four weeks. In certain aspects, the desired dosage can be delivered using multiple administrations (*e.g.*, two, three, four, five, six, seven, eight, nine, ten, eleven, twelve, thirteen, fourteen, or more administrations).

In certain aspects, an effective amount, of the provided glycan conjugates, immunogenic compositions or vaccines for administration one or more times a day to a 70 kg adult human may comprise about 0.0001 mg to about 3000 mg, about 0.0001 mg to about 2000 mg, about 0.0001 mg to about 1000 mg, about 0.001 mg to about 1000 mg, about 0.01 mg to about 1000 mg, about 0.1 mg to about 1000 mg, about 1 mg to about 1000 mg, about 1 mg to about 100 mg, about 10 mg to about 1000 mg, or about 100 mg to about 1000 mg, of a compound per unit dosage form.

In certain aspects, the provided glycan conjugates, immunogenic compositions or vaccines may be administered orally or parenterally at dosage levels sufficient to deliver from about 0.001 mg/kg to about 100 mg/kg, from about 0.01 mg/kg to about 50 mg/kg, preferably from about 0.1 mg/kg to about 40 mg/kg, preferably from about 0.5 mg/kg to about 30 mg/kg, from about 0.01 mg/kg to about 10 mg/kg, from about 0.1 mg/kg to about 10 mg/kg, and more preferably from about 1 mg/kg to about 25 mg/kg, of subject body weight per day, one or more times a day, to obtain the desired therapeutic effect.

It will be appreciated that dose ranges as described herein provide guidance for the administration of the provided glycan conjugates, immunogenic compositions or vaccines to an adult. The amount to be administered to, for example, a child or an adolescent can be determined by a medical practitioner or person skilled in the art and can be lower or the same as that administered to an adult.

It will be also appreciated that the provided glycan conjugates, immunogenic compositions or vaccines can be administered in combination with one or more additional therapeutically active agents. The provided glycan conjugates, immunogenic compositions or vaccines can be administered in combination with additional therapeutically active agents that improve their bioavailability, reduce and/or modify their metabolism, inhibit their excretion, and/or modify their distribution within the body. It will also be appreciated that the therapy employed may achieve a desired effect for the same disorder, and/or it may achieve different effects.

The provided glycan conjugates, immunogenic compositions or vaccines can be administered concurrently with, prior to, or subsequent to, one or more additional therapeutically active agents. In general, each agent will be administered at a dose and/or on a time schedule determined for that agent. In will further be appreciated that the additional therapeutically active agent utilized in this combination can be administered together in a single composition or administered separately in different compositions. The particular combination to employ in a regimen will take into account compatibility of the inventive compound with the additional therapeutically active agent and/or the desired therapeutic effect to be achieved. In general, it is expected that additional therapeutically active agents utilized in combination be utilized at levels that do not exceed the levels at which they are utilized individually. In some aspects, the levels utilized in combination will be lower than those utilized individually.

In certain aspects, the provided glycan conjugate, immunogenic composition or vaccine is administered in combination with one or more additional pharmaceutical agents described herein. In certain aspects, the additional pharmaceutical agent is an anti-cancer agent. Anti-cancer agents encompass biotherapeutic anti-cancer agents as well as chemotherapeutic agents.

Exemplary biotherapeutic anti-cancer agents include
interferons, cytokines (*e.g.*, tumor necrosis factor, interferon α, interferon γ), vaccines, hematopoietic growth factors, monoclonal serotherapy, immunostimulants and/or immunodulatory agents (*e.g.*, IL-1, 2, 4, 6, or 12), immune cell growth factors (*e.g.*, GM-CSF) and antibodies (*e.g.*, HERCEPTIN (trastuzumab), T-DM1, AVASTIN (bevacizumab), ERBITUX (cetuximab), VECTIBIX (panitumumab), RITUXAN (rituximab), BEXXAR (tositumomab)).

Exemplary chemotherapeutic agents include anti-estrogens (*e.g.*,
tamoxifen, raloxifene, and megestrol), LHRH agonists (*e.g.*, goscrclin and leuprolide), antiandrogens (*e.g.*, flutamide and bicalutamide), photodynamic therapies (*e.g.*, vertoporfin (BPD-MA), phthalocyanine, photosensitizer Pc4, and demethoxy-hypocrellin A (2BA-2-DMHA)), nitrogen mustards (*e.g.*, cyclophosphamide, ifosfamide, trofosfamide, chlorambucil, estramustine, and melphalan), nitrosoureas (*e.g.*, carmustine (BCNU) and lomustine (CCNU)), alkylsulphonates (*e.g.*, busulfan and treosulfan), triazenes (*e.g.*, dacarbazine, temozolomide), platinum containing compounds (*e.g.*, cisplatin, carboplatin, oxaliplatin), vinca alkaloids (*e.g.*, vincristine, vinblastine, vindesine, and vinorelbine), taxoids (*e.g.*, paclitaxel or a paclitaxel equivalent such as nanoparticle albumin-bound paclitaxel (Abraxane), docosahexaenoic acid bound-paclitaxel (DHA-paclitaxel, Taxoprexin), polyglutamate bound-paclitaxel (PG-paclitaxel, paclitaxel poliglumex, CT-2103, XYOTAX), the tumor-activated prodrug (TAP) ANG1005 (Angiopep-2 bound to three molecules of paclitaxel), paclitaxel-EC-1 (paclitaxel bound to the erbB2-recognizing peptide EC-1), and glucose-conjugated paclitaxel, *e.g*., 2'-paclitaxel methyl 2-glucopyranosyl succinate; docetaxel, taxol), epipodophyllins (*e.g*. etoposide, etoposide phosphate, teniposide, topotecan, 9-aminocamptothecin, camptoirinotecan, irinotecan, crisnatol, mytomycin C), anti-metabolites, DHFR inhibitors (e.g. methotrexate, dichloromethotrexate, trimetrexate, edatrexate), IMP dehydrogenase inhibitors (e.g. mycophenolic acid, tiazofurin, ribavirin, and EICAR), ribonuclotide reductase inhibitors (e.g. hydroxyurea and deferoxamine), uracil analogs (e.g. 5-fluorouracil (5-FU), floxuridine, doxifluridine, ratitrexed, tegafur-uracil, capecitabine), cytosine analogs (e.g. cytarabine (ara C), cytosine arabinoside, and fludarabine), purine analogs (e.g. mercaptopurine and Thioguanine), Vitamin D3 analogs (e.g. EB 1089, CB 1093, and KH 1060), isoprenylation inhibitors (*e.g.* lovastatin), dopaminergic neurotoxins (*e.g.* 1-methyl-4-phenylpyridinium ion), cell cycle inhibitors (*e.g.* staurosporine), actinomycin (*e.g.* actinomycin D, dactinomycin), bleomycin (*e.g*. bleomycin A2, bleomycin B2, peplomycin), anthracycline (*e.g*. daunorubicin, doxorubicin, pegylated liposomal doxorubicin, idarubicin, epirubicin, pirarubicin, zorubicin, mitoxantrone), MDR inhibitors (*e.g.* verapamil), Ca²⁺ ATPase inhibitors (*e.g*. thapsigargin), imatinib, thalidomide, lenalidomide, tyrosine kinase inhibitors (*e.g*., axitinib (AG013736), bosutinib (SKI-606), cediranib (RECENTIN™, AZD2171), dasatinib (SPRYCEL®, BMS-354825), erlotinib (TARCEVA®), gefitinib (IRESSA®), imatinib (Gleevec®, CGP57148B, STI-571), lapatinib (TYKERB®, TYVERB®), lestaurtinib (CEP-701), neratinib (HKI-272), nilotinib (TASIGNA®), semaxanib (semaxinib, SU5416), sunitinib (SUTENT®, SU11248), toceranib (PALLADIA®), vandetanib (ZACTIMA®, ZD6474), vatalanib (PTK787, PTK/ZK), trastuzumab (HERCEPTIN®), bevacizumab (AVASTIN®), rituximab (RITUXAN®), cetuximab (ERBITUX®), panitumumab (VECTIBIX®), ranibizumab (Lucentis®), nilotinib (TASIGNA®), sorafenib (NEXAVAR®), everolimus (AFINITOR®), alemtuzumab (CAMPATH®), gemtuzumab ozogamicin (MYLOTARG®), temsirolimus (TORISEL®), ENMD-2076, PCI-32765, AC220, dovitinib lactate (TKI258, CHIR-258), BIBW 2992 (TOVOK™), SGX523, PF-04217903, PF-02341066, PF-299804, BMS-777607, ABT-869, MP470, BIBF 1120 (VARGATEF®), AP24534, JNJ-26483327, MGCD265, DCC-2036, BMS-690154, CEP-11981, tivozanib (AV-951), OSI-930, MM-121, XL-184, XL-647, and/or XL228), proteasome inhibitors (*e.g*., bortezomib (VELCADE)), mTOR inhibitors (*e.g.*, rapamycin, temsirolimus (CCI-779), everolimus (RAD-001), ridaforolimus, AP23573 (Ariad), AZD8055 (AstraZeneca), BEZ235 (Novartis), BGT226 (Norvartis), XL765 (Sanofi Aventis), PF-4691502 (Pfizer), GDC0980 (Genetech), SF1126 (Semafoe) and OSI-027 (OSI)), oblimersen, gemcitabine, carminomycin, leucovorin, pemetrexed, cyclophosphamide, dacarbazine, procarbizine, prednisolone, dexamethasone, campathecin, plicamycin, asparaginase, aminopterin, methopterin, porfiromycin, melphalan, leurosidine, leurosine, chlorambucil, trabectedin, procarbazine, discodermolide, carminomycin,, aminopterin, and hexamethyl melamine.

In certain aspects, the subject being treated is a mammal. In certain aspects, the subject is a human. In certain aspects, the subject is a domesticated animal, such as a dog, cat, cow, pig, horse, sheep, or goat. In certain aspects, the subject is a companion animal such as a dog or cat. In certain aspects, the subject is a livestock animal such as a cow, pig, horse, sheep, or goat. In certain aspects, the subject is a zoo animal. In another aspects, the subject is a research animal such as a rodent, dog, or non-human primate. In certain aspects, the subject is a non-human transgenic animal such as a transgenic mouse or transgenic pig.

### Cancer Stem Cell Biomarkers

The discovery of cancer stem cells (CSCs), which are responsible for self-renewal and tumor-growth in heterogeneous cancer tissues, has stimulated interests in developing new cancer therapies and early diagnosis. The markers currently used for isolation of CSCs, however, are often not selective enough to enrich CSCs for the study of this special cell population. Here we show that the breast cancer stem cells (BCSCs) isolated with CD44⁺CD24^{-/lo}SSEA-3⁺ or ESA^{hi}PROCR^{hi}SSEA-3⁺ markers had higher tumorigenicity than those with conventional markers *in vitro* and *in vivo.* As few as 10 cells with CD44⁺CD24⁻/^{lo}SSEA-3⁺ formed tumor in mice, compared to more than 100 cells with CD44⁺CD24^{-/lo}. Suppression of SSEA-3 expression by knockdown of the gene encoding β-1,3-galactosyltransferase 5 (p3GalT5) in the globo-series pathway, led to apoptosis in cancer cells specifically but had no effect on normal cells. This finding is further supported by the analysis of SSEA-3 and the two related globo-series epitopes SSEA4 and globo-H in stem cells (ESCs and iPSCs) and various normal and cancer cells, and by the antibody approach to target the globo-series glycans and the late-stage clinical trials of a breast cancer vaccine.

Cancer stem cells are a special population of cancer cells with self-renewal and tumor-growth properties and are important targets for the development of anti-cancer therapy. We have found a glycolipid called stage-specific embryonic antigen 3 (SSEA-3) exclusively expressed on the surface of breast cancer stem cells, and when combined with the known protein markers (CD24 and CD44), breast cancer stem cells can be significantly enriched and as few as 10 such enriched cells can be used to grow tumor. In addition, the enzyme galactosyltransferase (β3GalT5) involved in the synthesis of SSEA3 is specifically expressed in breast cancer stem cells and cancer cells but not in normal cells, and both SSEA3 and β3GalT5 are found to be essential for cancer cell survival. These findings have led to the development of a new anti-cancer strategy.

Cancer stem cells (CSCs), which are rare cells with the ability of self-renewal and tumor initiation, are closely related to cancer progression and specific targets for effective therapy and early diagnosis (1-5). To date, many cancer stem cells have been identified and characterized by protein markers. Breast cancer stem cells (BCSCs) were first discovered in 2003 by Clarke *et al*.; it was demonstrated that breast cancer cells with CD44⁺CD24^{-/lo} expression have higher level of tumorigenicity than others and can form tumor in animals with -100 of such cells (6). In addition, other proteins such as ALDH-1, CD133, CD326 (ESA), CD201 (PROCR), and their combinations, are also reported as BCSCs biomarkers (7-9). However, the BCSCs obtained from the enrichment process based on these markers still contain a large number of non-cancer stem cells, and study of such cells would provide nonspecific characteristics of cancer stem cells. Therefore, new markers are required to enrich and obtain better-defined BCSCs for analysis and study.

Glycolipids are known to be altered during cancer development (10-15). In our previous study, the globo-series glycans SSEA-3 (Gb5), SSEA-4 (sialyl-Gb5) and globo-H (fucosyl-Gb5) are found exclusively on the cell surface of many cancers, including breast cancer and BCSCs (16-18). We also reported that BCSCs carrying either ESA^{hi}PROCR^{hi} or CD44⁺CD24^{-/lo} showed high expression of these globo-series epitopes (19). SSEA-3 is synthesized from Gb4 by β3GalT5 (20), and globo-H and SSEA-4 are synthesized from SSEA-3 by fucosyltransferases 1, 2 (FUT1, FUT2) (21, 22) and ST3 β-galactoside α-2,3-sialyltransferase 2 (ST3Gal2) (23), respectively.

We report herein regarding SSEA-3 and the related glycans and enzymes in the globo-series pathway are cancer specific and are BCSC markers.

### EXAMPLES

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### EXAMPLE 1: Exemplary Syntheses of SSEA3 analogues

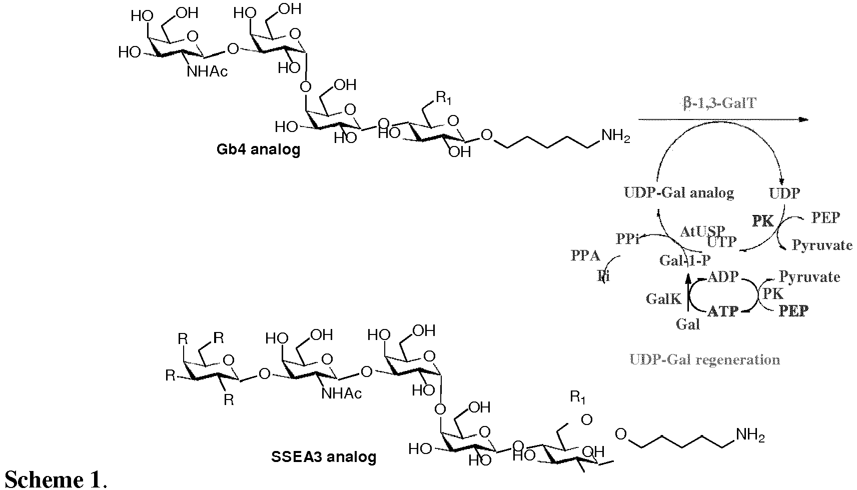

The combined compounds Gb4 analog, ATP, UTP, galactose analog, phosphoenolpyruvate, MgCl₂ with enzymes galactokinase (GalK), UDP-sugar pyrophosphorylase (AtUSP), β-1,3-galactosyltransferase (LgtD,), pyruvate kinase (PK), and inorganic pyrophosphatase (PPA) in the solution, and the reaction was initiated at room temperature with the pH controlled at 7.0, and the reaction was monitored by TLC until no more product could be observed. After completion of the reaction, the proteins in the reaction mixture were removed by heating for 30 min followed by centrifugation and filtration with 0.22 µM filter. The filtrate was then purified by C-18 gel chromatography. Fractions were collected and monitored by TLC.

### EXAMPLE 2: Exemplary Syntheses of SSEA4 analogues

### Method 1: Chemical Synthesis of SSEA4-Gc

Compound 1-6 were prepared by literature reported methods. To a solution of acceptor **3** (93 mg, 0.045 mmol) and imidates **6** (76 mg, 0.068 mmol) in 6 mL of dichloromethane (CH₂Cl₂) was added powdered molecular sieves (4A, 0.5 g). The mixture was stirred at room temperature for 2 h. After cooled to -10 °C, TMSOTf (5 µL, 0.03 mmol) was added, and the mixture was stirred at 5 °C (cold room) overnight. The reaction mixture was quenched by the addition of triethylamine (0.5 mL), diluted with CH₂Cl₂ and filtered through a pad of celite. The filtrate was washed with saturated sodium bicarbonate (NaHCO₃) aqueous solution, dried over sodium sulfate (Na₂SO₄), filtered, and concentrated. The residue was purified by flash silica get chromatography (50-100% EtOAc in Hexane) to afford hexasaccharide **7** contaminated with impurities from disaccharide imidates **6**. The yield is estimated by NMR (90 mg, 68%).

To a solution of hexasaccharide **7** (90 mg, 0.03 mmol) in glacial acetic acid (5.0 mL) was added Zinc dust (1 g), and the mixture was stirred for 1-2 h, until compound **7** was consumed by TLC analysis. The reaction mixture was diluted with CH₂Cl₂, filtered through a pad of celite, and concentrated under reduced pressure. The residue was dissolved in pyridine/Ac₂O (1:1, 2.0 mL), stirred for 1 h, and concentrated. The residue was purified by flash silica get chromatography. The acylated material was dissolved in anhydrous CH₂Cl₂ and MeOH (2:8, 10 mL) and treated with NaOMe (45 mg). After stirring at room temperature for 4 h, water (0.2 mL) was added, and the resulting mixture was stirred for 16 h. The reaction mixture was neutralized with amberlyst IR-120, filtered, and concentrated. The residue was purified by reverse phase chromatography (RP-18).

To the adduct in a mixture of methanol/water/ Acetic acid (10:10:0.5, 6 mL) was added palladium hydroxide (20% in Charcoal, 50 mg), and the reaction mixture was stirred at room temperature under a positive pressure of hydrogen for 16 h. The reaction mixture was filtered through a pad of celite and concentrated. The residue was purified by reverse phase chromatography to afford 8 (17 mg, 43%).

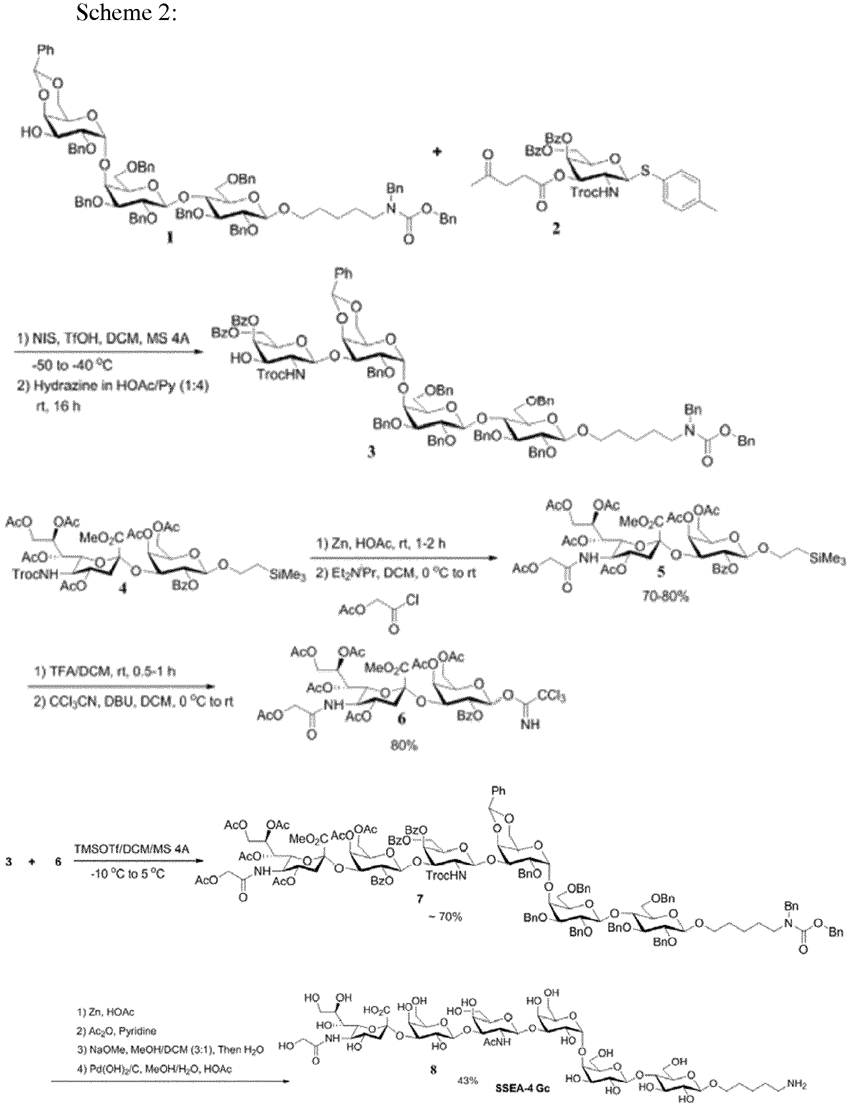

### Method 2: General Strategy of Chemoenzymatic Synthesis of SSEA4 analog

The CMP-sialic acid analog was synthesized by 3 enzymatic (ManNAc-6-kinase, NeuAc-9-P-synthase, and NeuAc-9-P-phosphase) reaction by using ManNAc as a starting material. The CMP-sialic acid analog reacted with Gb5 analog udner 2,3-sialyltransferase reaction combined with CMP-Neu5AC regeneration can obtained the SSEA4 analog.ref2

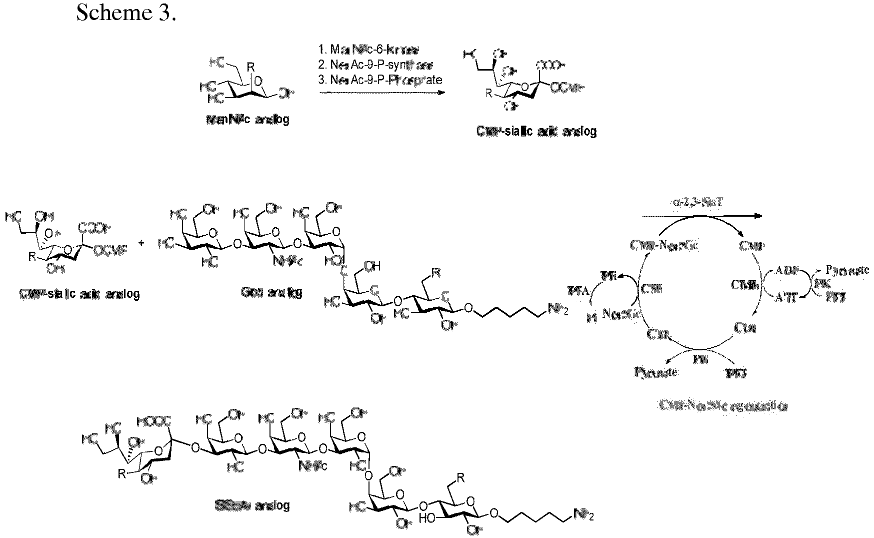

### EXAMPLE 3: Synthesis of SSEA-3 / SSEA-4 derivatives DT-conjugates

### General Methods:

### Step A. To modify SSEA3 analog-NH2 or SSEA4 analog-NH2 into SSEA3 analog-SH or SSEA4-analog-SH

To synthesize SSEA3/4 analog DT-conjugates, the amine-terminated SSEA3/4 analog were reacted with the DTSSP linker in PBS buffer (pH 7.4) at room temperature. To monitor the pH value of solution by pH paper, and add some NaOH solution to the solution when the solution became neuter or acid. After the reaction was stirred at room temperature for 12 hours, DTT was added to the solution at room temperature. The solution was kept stirring at 40 , and then the solvent was removed under reduced pressure. The residue was purified by LH-20 column chromatography to give SSEA3/4 analog-SH.

### Step B: To modify CRM197 into CRM197-maleimide.

After the salt of commercial CRM197 (1.0 mg) was removed via alternate dissolving in water and dialyzing (Amicon Ultra-0.5, 10 kDa,), the residue was dissolved in PBS buffer (pH 6.5, 1.0 mL) and transferred into a sample vial. Sulfo-EMCS (1.0 mg, 8.22×10-6 mol) was added to the solution, and then the reaction was kept stirring at room temperature for 2 hours. The mixture was purified by Amicon Ultra-0.5 (10 kDa). After using MALDI-TOF to check the molecular weight and BCA assay to calculate the amount of protein, the CRM197-maleimid was stored in PBS buffer (pH 7.2, 1.0 mg/mL) for next step. According to the data of MALDI-TOF, the amount of maleimid function groups could be calculated. For example, when the molecular weight of CRM197-maleimid was 61841, the numbers of maleimide function groups on CRM197-maleimid were (61841-58326)/193 = 18.2.

### Step C: The Synthesis of SSEA3/4 analog-CRM197 Conjugates

The CRM197-maleimids were dissolved in PBS buffer (pH 7.2, the concentration was 1.0 mg/mL) and then different amount of SSEA3/4 analog-SH (5.0 mg/mL in PBS buffer, pH 7.2) were added into the solution. The mixtures were stirred at room temperature for 2 hours. The SSEA3/4 analog-CRM197 conjugates were purified by using Amicon Ultra-0.5 (10 kDa) to remove the nonreactive SSEA3/4 analog-SH and sodium phosphate salt via dialysis. The obtained SSEA3/4 analog-CRM197 conjugates could be characterized by MALDI-TOF analysis to determine the carbohydrate incorporation rate. The nonreactive SSEA3/4 analog-SH could be recovered after reacting with DTT and purifying by LH-20 column chromatography.

### EXAMPLE 4: Syntheses of SSEA4-Gc CRM197 Conjugates

### Step A: To modify SSEA4-Gc-NH2 into SSEA4-Gc-SH

DTSSP (5.0 mg, 8.22×10-6 mol) was added to a flask of SSEA4-Gc-NH2 (5.0 mg, 4.01×10-6 mol) in PBS buffer (pH 7.4, 1.0 mL) at room temperature. To monitor the pH value of solution by pH paper, and add some NaOH (1 M / water) to the solution when the solution became neuter or acid. After the reaction was stirred at room temperature for 12 hours, DTT (5.0 mg, 32.41×10-6 mol) was added to the solution at room temperature. The solution was kept stirring at 40 for 1 hour, and then the solvent was removed under reduced pressure. The residue was purified by LH-20 column chromatography to give SSEA4-Gc-SH (5.0 mg, 93%)

### Step B: To modify CRM197 into CRM197-maleimide.

After the salt of commercial CRM197 (1.0 mg) was removed via alternate dissolving in water and dialyzing (Amicon Ultra-0.5, 10 kDa,), the residue was dissolved in PBS buffer (pH 6.5, 1.0 mL) and transferred into a sample vial. Sulfo-EMCS (1.0 mg, 8.22×10-6 mol) was added to the solution, and then the reaction was kept stirring at room temperature for 2 hours. The mixture was purified by Amicon Ultra-0.5 (10 kDa). After using MALDI-TOF to check the molecular weight and BCA assay to calculate the amount of protein, the CRM197-maleimid was stored in PBS buffer (pH 7.2, 1.0 mg/mL) for next step. According to the data of MALDI-TOF, the amount of maleimid function groups could be calculated. For example, when the molecular weight of CRM197-maleimid was 61841, the numbers of maleimide function groups on CRM197-maleimid were (61841-58326)/193 = 18.2.

As following the Table 1, the CRM197-maleimids were dissolved in PBS buffer (pH 7.2, the concentration was 1.0 mg/mL) and then different amount of SSE4Gc-SH (5.0 mg/mL in PBS buffer, pH 7.2) were added into the solution. The mixtures were stirred at room temperature for 2 hours. The SSEA4-Gc-CRM197 conjugates were purified by using Amicon Ultra-0.5 (10 kDa) to remove the nonreactive SSEA4-Gc-SH and sodium phosphate salt via dialysis. The obtained SSEA4-Gc-CRM197 conjugates could be characterized by MALDI-TOF analysis to determine the carbohydrate incorporation rate as showing in Table 1. The nonreactive SSEA4-Gc-SH could be recovered after reacting with DTT and purifying by LH-20 column chromatography.

### Step C: To trap the nonreactive maleimides of CRM197-maleimide

The SSEA4-Gc-CRM197 conjugates were dissolved in PBS buffer (pH 7.2, the concentration was 1.0 mg/mL) and 10.0 equivalent of 2-mercaptoethanol (5 mg/mL, PBS buffer, pH 7.2) were added to the solution. The mixtures were stirred at room temperature for 2 hours. The SSEA4-Gc-CRM197 conjugates were purified by using Amicon Ultra-0.5 (10 kDa) to remove the nonreactive 2-mercaptoethanol and sodium phosphate salt via dialysis and then lyphophilized to a white powder.

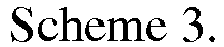

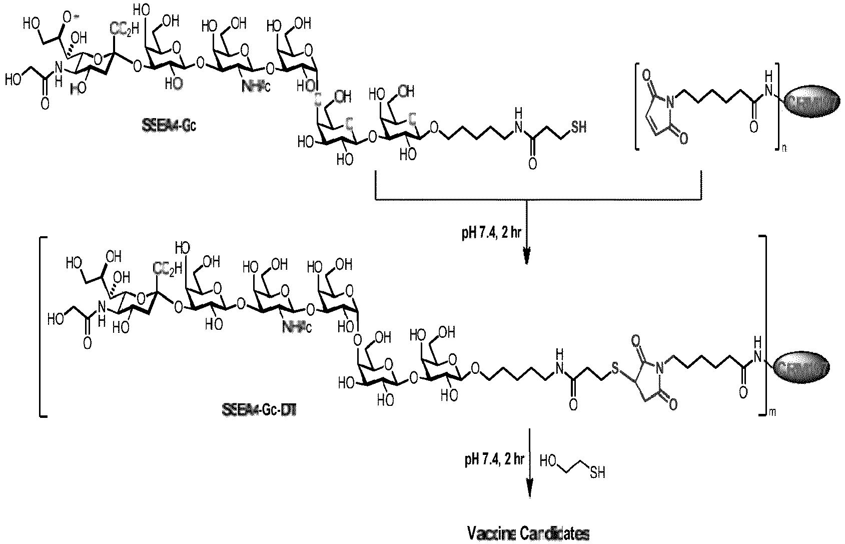

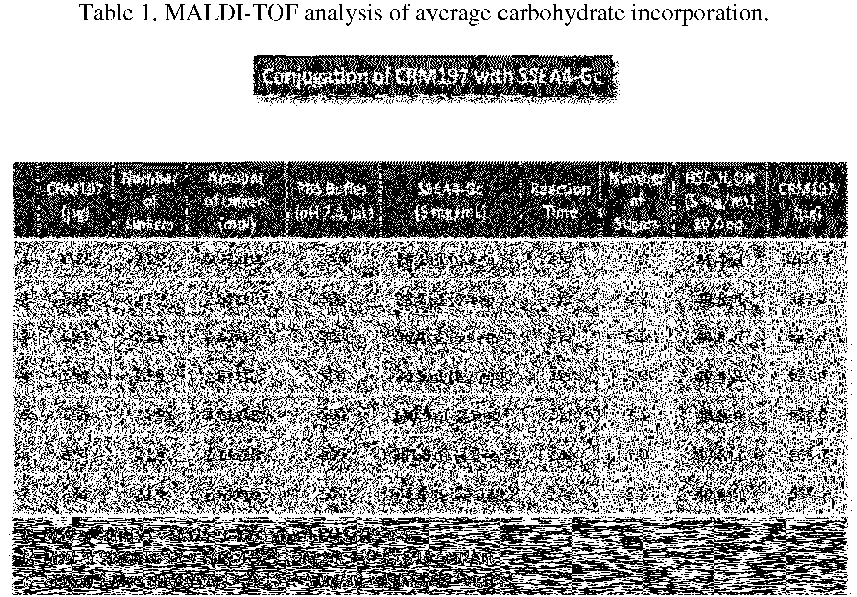

### EXAMPLE 5: Immunogenicity Study of the SSEA-4 derivatives DT-conjugates

To investigate the immunogenicity of the SSEA4 analog DT-conjugates (1-DT to 10-DT), five female BALB/c mice were immunized intramuscularly with 2 µg of SSEA4 analog DT-conjugates and 2 µg of the glycolipid adjuvant C34 three times at biweekly intervals. In the previous study, the anti-GH antibodies titer was low with SSEA4 analog-protein conjugates alone without any adjuvants. The antisera from each immunogen were obtained ten days after the third immunization and were tested on the glycan microarray containing 94 chemically synthesized glycans, including globo series glycans and other tumor-associated carbohydrate antigens. Because some chemical modifications were carried out on the glycan, some functional linkers were also included in the glycan array to check the cross reactivity.

Antibodies induced by the SSEA4-Gc CRM197-conjugates were specifically recognized by SSEA4-Gc, native SSEA4 or SSEA4 tetrasaccharide fragments but not by other TACAs and functional linkers. The sera obtained from the glycoconjugates induced high IgG antibody titers, indicating a T-cell-dependent immune response. Interestingly, no significant IgM production was observed for SSEA4-Gc or native SSEA4. Regarding the IgG level against GH, the titers of antibodies induced by SSEA4-Gc CRM197 was much higher than the nature form native SSEA-CRM197 conjugate. Among them the 6.9 molecule of SSEA4-Gc conjugated with one molecule of CRM197 can induce the highest antibody titers (also see Fig 12).

### Mice Dosage and Immunization Schedule

For comparing the immunogenicity of SSEA4 analog CRM197, ten groups of five mice (8-week-old female Balb/c mice, BioLASCO, Taiwan) were immunized intramuscularly with glycolipid C34. Three immunizations were given at 2-week intervals. Each vaccination contained 2 µg SSEA4 analog and 2 µg C34. Control mice were injected with phosphate buffer saline (PBS). Mice were bled before the first immunization (preimmune) and 10 d after the third immunization. All of the sera were obtained by centrifugation at 4,000 × g for 10 min. The serologic responses were analyzed by glycan microarray.

### Serologic assay with glycan array

Mouse sera were diluted with 1% BSA/PBST buffer (PBST buffer: PBS and 0.05% Tween-20, pH 7.4). The glycan microarray was blocked with Superblock blocking buffer (Pierce) for 1 h at 4 oC and washed three times with PBST buffer before use. The serum dilutions were then introduced to the glycan microarray and incubated at 4 oC for 1 h. Excess serum antibodies were washed out and the microarrays were incubated individually with Alexa Fluor 647-conjugated goat anti-mouse IgG antibody or DyLight 649-conjugated goat anti-mouse IgM antibody as the 2nd antibody at 4 oC in dark for 1 h. The slides were then washed three times with PBST and scanned at 635 nm wavelength with a microarray fluorescence chip reader (GenePix 4300A; Molecular Devices Corporation) and scanned images were analyzed with GenePix Pro-6.0 analysis software (Axon Instruments, Union City, CA, USA).

### EXAMPLE 6

### Stage-Specific Embryonic Antigen-3 (SSEA-3) and β3GalT5 are Cancer Specific and Significant Markers for Breast Cancer Stem Cells

### Example: Cell Culture

Breast cancer cell lines MDA-MB-231, MCF-7 and human breast cancer associated fibroblast (CAF) were obtained from American Type Culture Collection (ATCC). The culture of MDA-MB-231 was in DMEM supplemented with 10% of heat-inactivated FBS and antibiotic-antimycotic whereas that of MCF-7 culture was in RPMI supplemented with 10% of heat-inactivated FBS, non-essential amino acids and antibiotic-antimycotic. For the culture of CAF, it was in DMEM/F12 supplemented with 10% of heat-inactivated FBS, non-essential amino acids, sodium pyruvate, glutamine, penicillin and streptomycin. They were incubated at 37oC incubator with 5% of CO2 and humidified atmosphere control. All the cell culture media and supplements were purchased from Life Technologies. Human ESC H9 and induced pluripotent stem cell 5 (iPSC5) were maintained and cultured on mitomycin C treated-mouse embryonic fibroblasts (MEFs) in human ES medium (**Knockout** DMEM with **Knockout Serum Replacement**, GlutaMAX, non-essential amino acids, 2-Mercaptoethanol, Penicillin/Streptomycin and bFGF) and were passaged weekly using collagenase IV.

### Example: Derivation of iPSCs from dermal fibroblasts

Fibroblasts derived from dermal biopsies were reprogrammed into pluripotent stem cells using the CytoTune-iPS Sendai Reprogramming Kit (Life Technologies). Briefly, 5 × 104 fibroblasts were seeded per well in a 6-well dish at passage 3 for recovery overnight. The next day, Sendai viruses expressing human transcription factors OCT4, SOX2, Klf4, and c-Myc were mixed in fibroblast medium to infect fibroblast cells according to the manufacturer's instructions. After 2 days, the medium was exchanged with human ES medium supplemented with the ALK5 inhibitor SB431542 (2 µM; Stemgent), the MEK inhibitor PD0325901 (0.5 µM; Stemgent), and thiazovivin (0.5 µM; Stemgent). Day 7-10 after infection, cells were detached using TrypLE (Life Technologies) and passaged onto feeder cells. Individual colonies of iPSCs were picked between days 21 and 28 after infection, and each iPSC line was expanded from a single colony. All iPSCs lines were cultured on mouse embryonic fibroblast cells in human ES medium.

Karyotyping was performed by Cell Line Genetics Inc. In teratoma analysis, 1-2 × 107 from each iPSC line were detached and collected after TrypLE treatment. They were suspended in 0.5 mL human ES media. Followed by mixing with 0.5 mL Matrigel (BD Biosciences), cells were injected subcutaneously into dorsal flanks of an immunodeficient mouse (NOD.Cg-Prkdcscid Il2rgtm1Wjl/SzJ, stock no. 005557, The Jackson Laboratory). Eight weeks after injection, teratomas were harvested, fixed overnight with 4% paraformaldehyde, and processed according to standard procedures for paraffin embedding. The samples were then sectioned and H&E stained.

### Example: Overexpression and Knockdown of 3GalT5

To establish human β3GalT5 overexpression stable lines, full-length cDNA that encodes human β3GalT5 was PCR amplified (forward primer-GCAGATCTATGGCTTTCCCGAAGATG; reverse primer-GTCTCGAGTCAGACA GGCGGACAAT), and subcloned into BglII/XhoI cut pMSCVpuro vector (Clontech). Murine stem cell virus (MSCV)-control and MSCV- β3GalT5 vesicular stomatitis virus G glycoprotein (VSV-G) pseudotyped retrovirus were then generated in GP2-293 cells (Clontech) and used to infect MCF-7 and MDA-MB-231 cells. Two days after viral infection, control and β3GalT5 stable pools were selected with puromycin (2 µg/mL). To establish β3GalT5 knockdown cells, the lentivirus-shRNA systems for human β3ΓαλT5 were purchased from National RNAi Core Facility Platform, Academia Sinica, and the 3GalT5-short hairpin sequence is
5'CCGGGCAAGTGGTTTGTCAGTAAATCTCGAGATTTACTGACAAACCACTTGCTT TTTG-3'. Briefly, shβ3GalT5 and shControl lentiviruses were incubated with MCF7 and MDA-MB-231 cells according to the manufacturer's instructions. Infected cells were harvested 48 h post-infection or selected with puromycin (2 µg/mL) and the knockdown efficiency was determined by quantitative RT-PCR (qPCR).

### Example: Cell Proliferation Assay

Cell proliferation was analysed using a cell permeable tetrazolium salt, WST-1 (4-[3-(4-iodophenyl)-2-(4-nitrophenyl)-2H-5-tetrazolio]- 1,3-benzene disulfonate), according to the manufacturer's instruction (Roche). 2 × 103 cells/well were cultured in 96-well plates. At different time points as indicated, WST-1 (20 µL per well for 200 µL culture medium) was added and incubated for 3 h at 37°C incubator. Signaling detection of absorbance at 450 nm and 690 nm (as reference) were read by a SpectraMax M5 microplate spectrum reader (Molecular Devices).

### Example: Apoptosis Assay

Cells were treated with or without β3GalT5 shRNA lentivirus (MOI: 5), Z-DEVD-FMK (caspase-3 inhibitor) (50 µM and 100 µM; R&D Systems), Z-IETD-FMK (caspase-8 inhibitor), Z-LEHD-FMK (caspase-9 inhibitor), or Z-ATAD-FMK (caspase-12 inhibitor) (100 µM; R&D Systems) at 105 cells/mL as previously described. Three days later, cells were washed with PBS and incubated with allophycocyanin (APC)-conjugated annexin V (1:40 dilution; BD Biosciences) in binding buffer (0.01 M HEPES, 0.14 M NaCl, 2.5 mM CaCl2) for 15 min on ice and then subjected to flow cytometric analysis.

### Example: Western Blot Analysis

Protein lysates of MCF-7 and MDA-MB-231 cells were prepared using lysis buffer (150 mM NaCl2, 100 mM phosphate buffer at pH 7.4, 1 % NP40, 10% glycerol) supplied with protease inhibitors (Roche). The proteins from cell lysate were denatured in sample buffer at 95 °C for 5 min before being applied to 4-12% gradient SDS/PAGE and were transferred onto methanol-rinsed PVDF membranes using transfer device (Bio-Rad). Membrane was blocked with 5% nonfat milk-supplied TBST for 30 min before probing with the anti-caspase-3 antibody that recognizes either procaspase-3 or cleaved/active form of caspase-3 (1:1,000 dilution; Abcam), followed by incubation with HRP-conjugated anti-rabbit antibody (1:5,000 dilution; Jackson ImmunoResearch) for 90 min. The signals were developed using the ECL Substrate Kit (Millipore) and detected by Fujifilm LAS-4000 imaging system.

### Example: qPCR.

Total mRNA from cell lines was extracted using GeneJET RNA Purification Kit (Thermo Scientific) and 2ug of it was reverse transcribed to cDNA by High Capacity cDNA Reverse Transcription Kits (Life Technologies). qPCR reactions were prepared in a total volume of 20 µl containing 2 µl of cDNA of the test sample or control sample with 2X SYBR Green master mix (Thermo Scientific) optimized by the manufacturer's protocol. cDNA was examined the expression of B3GalT5 (Forward primer: 5'AGCGGA AACGAA AGAGGTGGAC 3' ; Reversed primer: 5' CCTGAGGACAAA AGCGATGGAC 3') by Applied Biosystems 7300 Real-Time PCR system (Life Technologies). The relative gene expression was normalized as the ratio of the B3GalT5 gene to the internal GAPDH gene expression according to the Ct values using 7300 software.

### Example: Extraction of Glycosphingolipids

Cells were harvested, washed with PBS, and homogenized in water. Methanol and chloroform were added to the homogenate at a ratio of 8:4:3 (vol/vol/vol), and the sample was incubated in a bath sonicator for 30 min. After centrifugation at 3,000 x g for 15 min, the pellet was repeatedly extracted with 4:8:3 (vol/vol/vol) chloroform/ methanol/water, and the combined supernatant was dried under a stream of nitrogen.

### Example: Release of Glycans from Glycosphingolipids (GSLs) (26)

Cell were collected and quantified for the amount of total protein for normalization, and 1-3 × 106 cells were homogenized. In a typical procedure for the release of free glycans from GSLs, the GSLs were treated with ozone in chloroform/methanol (2:1; 1.0 mg/mL) in a glass tube until blue color occurs (10 min). The resulting solution was dried in a SpeedVac and treated by base for release of glycans from GSLs; briefly, aqueous sodium hydroxide solution (20-50 mM) was added, and the mixture was incubated for 16 h at room temperature. The resulting aqueous solution is lyophilized for labeling with NAIM tag.

### Example: Labeling Glycans with NAIM Tag and LC-MS Analysis

After release from GSLs, the glycan mixture was lyophilized and labeled by following literature procedures (27, 28). Briefly, the glycan mixture was added 2,3-naphthalenediamine (NAIM, 1.0 mg) and iodine (1.0 mg) in AcOH (1.0 mL) at room temperature and stirred for 4 h. The completion of reaction was checked by TLC analysis. The reaction mixture was then triturated with EtOAc (10.0 mL × 2) to give precipitates (globo-H-NAIM, SSEA-4-NAIM and SSEA-3-NAIM), which were collected by filtration using nylon membrane filter. The NAIM-labeled glycans, which showed enhanced ionization ability in MS (29), were analysed by high resolution and high mass accuracy nanoflow LC-MS/MS. Samples were injected at 10 µL/min into a precolumn (150 µm I.D. × 30 mm, 5 µm, 200 Å) and then separated in a reversed phase C18 nano-column (75 µm I.D. × 200 mm, 2.5 µm, 100 Å) for analysis in an LTQ FT Ultra mass spectrometer (Thermo Fisher Scientific) was equipped with a nanoelectrospry ion source (New Objective). Separation was performed at 300 nL/min using 0.1% formic acid in water as mobile phase A and 0.1% formic acid in 80% acetonitrile as mobile phase B. Survey full scan MS spectra (from m/z 320 to 2,000) were acquired in the FT with a mass resolution of 100,000 at m/z 400.

### Example 9

### Demonstration that Stage-Specific Embryonic Antigen-3 (SSEA-3) and β3GalT5 are Cancer Specific and Significant Markers for Breast Cancer Stem Cells

To demonstrate the tumorigenic ability of cells, cancer cells were stained with corresponding antibodies against the glycolipid molecules SSEA-3, SSEA-4 and globo-H and the known marker sets CD44/CD24 and ESA/PROCR, in breast cancer cell lines MCF-7 and MDA-MB-231, respectively, for the cell sorting (Fig. S1, sorting 1). The isolated cell populations were next analyzed by both in vitro (24) and in vivo (8) assays (Fig. 1). In MCF-7, cancer cells expressing CD44+CD24-/loSSEA-3+ formed a higher percentage of mammospheres than those expressing CD44+CD24-/loSSEA-3- or CD44+CD24-/lo (Fig. 1A, left panel). Similarly, in MDA-MB-231, the ESAhiPROCRhiSSEA-3+ subpopulation formed a higher percentage of cell colonies than ESAhiPROCRhiSSEA-3- or ESAhiPROCRhi cells in the soft agar assay (Fig. 1C, left panel). However, there were no significant differences in the formation of cell colony and mammosphere using the cells isolated by the known marker sets along with the glycolipid epitopes SSEA-4 or globo-H (Fig. S2). In order to show the tumorigenicity in cells carrying known BCSC markers and SSEA-3, different subpopulations were inoculated into the mammary glands of NOD-SCID mice for tumor-growth. The result showed that both CD44+CD24-/loSSEA-3+ and ESAhiPROCRhiSSEA-3- effectively generated tumor in vivo with a low cell number, compared with other corresponding subpopulations (Fig. 1B, D). Particularly for cells expressing CD44+CD24-/loSSEA-3+, as few as 10 cells were able to form tumor in mice (Fig. 1B). In terms of tumor-growth, the tumor volume of CD44+CD24-/loSSEA-3+ cells was twice larger than that of CD44+CD24-/loSSEA-3- cells (Fig. IE, left panel). In addition, ESAhiPROCRhiSSEA-3+ cells developed tumor earlier, and formed tumor in a greater average volume than ESAhiPROCRhiSSEA-3-cells. These results indicate that SSEA-3 is a specific marker for the enrichment of BCSCs in different breast cancer cell models. Among these glycan molecules, cells carrying SSEA-3 and known BCSC markers had a higher tumorigenicity than other subpopulations.

We next compared the stem-like properties of cancer cells with highly expressed SSEA-3 and those without SSEA-3 (Fig S1, sorting 2). In SSEA-3+ MCF-7 cells, the top 1% of cells expressing a high level of SSEA-3 within the total population, formed a higher percentage of mammosphere than the bulk population and those without SSEA-3 and CD44+CD24-/lo (Fig. 1A, right panel). In addition, the top 1% of MDA-MB 231 cells with the highest SSEA-3 expression within the bulk population also formed more cell colonies than the bulk population and other subpopulations (Fig. 1c, right panel). In the animal study, results showed that the cells with top 1% SSEA-3 expression had a higher potential to form tumor than SSEA-3- cells (Fig. 1B and D), and the average tumor volume of SSEA-3+ cells was greater than that of SSEA-3- cells (Fig. IE, F, right panel). Thus, cancer cells expressing a high level of SSEA-3 had a higher tumorigencity than those without SSEA-3 on cell surface, indicating that SSEA-3 is also an independent CSCs marker for breast cancer.

To understand the function of SSEA-3, the gene of β3GalT5 responsible for SSEA-3 biosynthesis (Fig. S3) was overexpressed or knocked down for further study. Overexpression of β3GalT5 increased the expression level of surface SSEA-3 in both MCF-7 and MDA-MB-231 cells (Fig. 2). Notably, in MCF-7 cells, the percentage of CD44+CD24-/lo cell population showed five-fold increase comparing to control (Fig. 2A); in MDA-MB-231 cells, there was no change in the percentage of ESAhiPROCRhi when β3GalT5 was overexpressed (Fig. 2B). In MCF-7 cells with β3GalT5 knockdown, comparing with control cells, the expression level of surface CD44 was reduced, and therefore the CD44-CD24+ cell population increased 10 folds (Fig. 2A). In MDA-MB-231 cells with β3GalT5 knockdown, the level of surface PROCR decreased and the ESAhiPROCRhi BCSC subpopulation reduced (Fig. 2B). These findings demonstrate that SSEA-3 is a critical glycan molecule associated with BCSCs.

To demonstrate the role of SSEA-3 in breast cancer and normal cells, the cellular phenotypes were examined in the β3GalT5 knockdown cells. In both MDA-MB-231 and MCF-7 cells, knockdown of β3GalT5 suppressed cell growth (Fig. 4A and B), along with the appearance of cell apoptosis, especially in MDA-MB-231 cells that >60% of cells underwent apoptosis on day 4 (Fig. 3B and C). In contrast, in normal breast cells MCF-10A and human telomerase reverse transcriptase (hTERT)-immortalized human mammary epithelial cells, hTERT-HME1, the same growth rate and no apoptosis was observed with knockdown of β3GalT5 (Fig. 3B, 3C, 4C and 4D). However, knockdown of FUT1 and FUT2 for the synthesis of globo-H from SSEA-3 or ST3Gal2 for the synthesis of SSEA-4 from SSEA-3 did not induce cell apoptosis in MDA-MB-231 cells (Fig. 3A, D).

To further investigate if the apoptosis induced by beta3GalT5 knockdown is associated with the activation of caspase-3, the most effector caspase for the downstream execution of apoptosis. Results showed that caspase-3 was activated in MDA-MB-231 cells with knockdown of beta 3GalT5 (Fig. 4E). When the inhibitor for caspase 3, Z-DEVD was added, the percentage of apoptosis induced by beta3GalT5 knockdown reduced (Fig. 4F). The involvement of caspase-3 in the apoptosis induced by beta3GalT5 knockdown was also confirmed in MCF-7, a caspase-3-deficient cell line. Although the growth rate of MCF-7 was significantly reduced by knockdown of beta3GalT5, only a low level of apoptosis was shown when the expression of SSEA-3 was suppressed (Fig. 3B and C). Further investigation of the upstream caspases (caspase-8, -9, and -12) was then studied by testing with specific inhibitors, and the result illustrated that caspase-8 also reduced the percentage of cell apoptosis in MDA-MB-231 cells with beta3GalT5 knockdown (Fig. 3G). These results suggest that SSEA-3, the immediate enzymatic product of beta3GalT5, is an important glycolipid for growth and survival in cancer.

To confirm if SSEA-3 or any of the three globo-series glycans was only found in cancer cells, the glycolipids from embryonic stem cells (ESCs), induced pluripotent stem cells (iPSCs, Fig. S4), MCF-7 and MDA-MB-231 cells, and normal cell lines, including MCF-10A and hTERT-HME1, were extracted and the glycans were released, tagged and examined by LC-MS analysis (Fig. 5A). The data was compared with the results of flow cytometric analysis, in which same antibodies used for cell sorting were used to detect the expression levels of SSEA-3, SSEA-4, and globo-H (Fig. 5). It was found that ESCs, iPSCs and cancer cell lines but not normal cell lines expressed SSEA-3, SSEA-4 and globo-H. The result from this study was also supported by qPCR of beta3GalT5 gene expression in normal and cancer cell lines (Fig. S5). Further analysis of the glycol-series glycans on different normal and cancer cell lines will be carried out.

The expression level of SSEA-3 in MCF-7 cells detected by flow cytometry was relatively higher than that by the LC-MS analysis, while the level of SSEA-3 in MDA-MB-231 detected by LC-MS was much higher than that by flow cytometry. The variation between the LC-MS and flow cytometry data could be due to the specificity of antibody and the distribution of the glycans on the cell surface (25). Due to the cross-reaction of anti-SSEA-3 antibody (MC-631) toward SSEA-4 and to a lesser extent, Gb4 (14), it is possible to overestimate the level of SSEA-3 detected by flow cytometry when there is a high expression level of SSEA-4. On the other hand, the level of SSEA-3 could be underestimated because of hindrance caused by other biomolecules on cell surface and thus SSEA-3 on the cells may not be reached in antibody staining (26, 27). Therefore, we believe that the LC-MS result, which is supported by the qPCR detection of β3GalT5 gene expression (Fig S5), more accurately reflects the expression of these glycolipids.

In the process of BCSC isolation, it is possible that some cells with a high level of SSEA-4 expression but carry no SSEA-3 are enriched when sorted based on MC-631 staining. Since we proved that both SSEA-3 and its synthetic enzyme β3GalT5 are BCSCs markers, SSEA-3 negative cells are low tumorigenic. The cell population is not purified enough and thus the tumorigenicity of the cells sorted based on anti-SSEA-3 staining may be underestimated. We consider an antibody or molecule, which is highly specific to SSEA-3, can be generated for the enrichment of BCSC. On the other hand, if SSEA-3 on the cell surface can be specifically detected and sorted by flow cytometry, the results of both antibody staining and LC-MS analysis should be consistent.

SSEA-3 is a BCSC marker that plays a major role in cancer progression. From the experiments, we showed that manipulating the expression of β3GalT5 in cancer cells controlled the cell surface level of SSEA-3, SSEA-4 and globo-H, as well as the cellular survival and tumorigenicity. Interestingly, knockdown of β3GalT5 in cancer cells could trigger both apoptosis and inhibition of cell proliferation through different mechanisms, as MCF-7, a caspase-3 null cell line, underwent a limited level of apoptosis and profound suppression of cell growth after knockdown of beta3GalT5. In contrast, in normal mammary epithelial cells, which lack SSEA-3 expression, knockdown of beta3GalT5 did not affect these phenotypes.

In summary, this report demonstrated that SSEA-3 is a novel glycan marker useful for the enrichment of BCSCs, and both SSEA-3 and beta3GalT5 are potential new targets for the development of breast cancer therapeutics. In addition to their specific expression on most cancer stem cells and cancer cells, the globo-series glycolipids SSEA-3, SSEA-4 and globo-H are also highly expressed on the surface of ESCs and iPSCs, but they disappear after differentiation of ESCs. It would be interesting to understand the fate of the globo-series glycolipids after differentiation of iPSCs for use in regenerative medicine.

### Example Materials and Methods

### Cell Culture

Breast cancer cell lines MDA-MB-231, MCF-7 and human breast cancer associated fibroblast (CAF) were obtained from American Type Culture Collection (ATCC). The culture of MDA-MB-231 was in DMEM supplemented with 10% of heat-inactivated FBS and antibiotic-antimycotic whereas that of MCF-7 culture was in RPMI supplemented with 10% of heat-inactivated FBS, non-essential amino acids and antibiotic-antimycotic. For the culture of CAF, it was in DMEM/F12 supplemented with 10% of heat-inactivated FBS, non-essential amino acids, sodium pyruvate, glutamine, penicillin and streptomycin. They were incubated at 37oC incubator with 5% of CO2 and humidified atmosphere control. All the cell culture media and supplements were purchased from Life Technologies. Human ESC H9 and induced pluripotent stem cell 5 (iPSC5) were maintained and cultured on mitomycin C treated-mouse embryonic fibroblasts (MEFs) in human ES medium (**Knockout** DMEM with **Knockout Serum Replacement,** GlutaMAX, non-essential amino acids, 2-Mercaptoethanol, Penicillin/Streptomycin and bFGF) and were passaged weekly using collagenase IV.

### Example

### Derivation of iPSCs from dermal fibroblasts

Fibroblasts derived from dermal biopsies were reprogrammed into pluripotent stem cells using the CytoTune-iPS Sendai Reprogramming Kit (Life Technologies). Briefly, 5 × 104 fibroblasts were seeded per well in a 6-well dish at passage 3 for recovery overnight. The next day, Sendai viruses expressing human transcription factors OCT4, SOX2, Klf4, and c-Myc were mixed in fibroblast medium to infect fibroblast cells according to the manufacturer's instructions. After 2 days, the medium was exchanged with human ES medium supplemented with the ALK5 inhibitor SB431542 (2 µM; Stemgent), the MEK inhibitor PD0325901 (0.5 µM; Stemgent), and thiazovivin (0.5 µM; Stemgent). Day 7-10 after infection, cells were detached using TrypLE (Life Technologies) and passaged onto feeder cells. Individual colonies of iPSCs were picked between days 21 and 28 after infection, and each iPSC line was expanded from a single colony. All iPSCs lines were cultured on mouse embryonic fibroblast cells in human ES medium.

Karyotyping was performed by Cell Line Genetics Inc. In teratoma analysis, 1-2 × 107 from each iPSC line were detached and collected after TrypLE treatment. They were suspended in 0.5 mL human ES media. Followed by mixing with 0.5 mL Matrigel (BD Biosciences), cells were injected subcutaneously into dorsal flanks of an immunodeficient mouse (NOD.Cg-Prkdcscid Il2rgtm1Wjl/SzJ, stock no. 005557, The Jackson Laboratory). Eight weeks after injection, teratomas were harvested, fixed overnight with 4% paraformaldehyde, and processed according to standard procedures for paraffin embedding. The samples were then sectioned and H&E stained.

### Example

### Flow Cytometry and Cell Sorting

Cell labeling was done by staining with antibodies in buffer composed of PBS supplemented with 1% FBS. Accutase (eBioscience, San Diego, CA) detached cells were incubated with antibodies (using antibody titration suggested by the supplier) for 30 min on ice in the dark. Antibodies used in this study were PE-conjugated anti-PROCR (RCR-252; BD Biosciences, San Jose, CA), APC-conjugated anti-ESA (1B7; eBioscience), PE-conjugated anti-CD24 (SN3 A5-2H10, eBioscience), APC-conjugated anti-CD44 (IM-7, eBioscience) along with biotinylated anti-SSEA-3 (MC-631; eBioscience) at 4oC for 30 min in the dark. After washing twice, the cells were stained with Alexa Fluor 488-conjugated streptavidin at 4oC for 30 min in the dark. Proper isotype controls were used for each cell labeling experiment. The same antibodies were used in all staining and sorting experiments in this study. Live cell sorting was done using a BD FACSAriaU with a 100 m nozzle following the manufacturer's instructions. For MDA-MB-231 cells, the sorted cells were incubated with DMEM/10%FBS/antibiotics/antimycotics to recover in ultra-low attachment surface plates overnight in a humidified 37oC incubator before further analyses. For MCF-7 cells, they were subject to further experiments readily after sorting. The percentage of cells in different marker populations was evaluated using the software FlowJo.

### Example

### Soft Agar Assay

Soft agar colony formation assay was performed by seeding cells in a layer of 0.35% SeaPlaque agarose (Lonza, Switzland) with DMEM/FBS over a basal layer of 0.5% SeaPlaque agarose/DMEM/FBS. Cultures were maintained in a humidified 37oC incubator. Additional media was added every 2-3 days to continuously supply growth supplements to the cells. On day 21 after seeding, cells were fixed with pure ethanol containing 0.05% crystal violet and colony forming efficiency quantified by light microscopy.

### Example

### Mammosphere Formation

In the mammosphere formation assay, cells were incubated in DMEM/F12 with supplement B27 (Life Technologies) and 10 ng/ml EGF on 96-well low-attachment plates in the density of 100 cells/well. Culture was maintained in a humidified 37oC incubator. After 14 days, the number of mammospheres was counted under a light microscope.

### Example

### Mouse Tumorigenicity Assay

NOD-SCID (NS) mice were used to evaluate the stem cell properties of sorted cells expressing potential stem cell markers from the human breast cancer cell lines. Animal care and experiments were approved by the Institutional Animal Care and Utilization Committee of Academia Sinica (IACUC#130-09-575). Four-week old NS mice were injected with sorted cancer cells mixed with CAF (1:1) and Matrigel (BD bioscience) (1:1) in fat pads. For MCF-7, mice were additionally injected with estrogen pellets (0.18 mg/pellet, 90 days release, Innovative Research of America) before the day of experiment. Tumor volumes were evaluated every five days after initial detection. The tumor formation efficiency was determined on day 50 after cell injection.

### Example

Overexpression and Knockdown of β3GalT5

To establish human β3GalT5 overexpression stable lines, full-length cDNA that encodes human β3GalT5 was PCR amplified (forward primer-GCAGATCTATGGCTTTCCCGAAGATG; reverse primer-GTCTCGAGTCAGACA GGCGGACAAT), and subcloned into BglII/XhoI cut pMSCVpuro vector (Clontech). Murine stem cell virus (MSCV)-control and MSCV- β3GalT5 vesicular stomatitis virus G glycoprotein (VSV-G) pseudotyped retrovirus were then generated in GP2-293 cells (Clontech) and used to infect MCF-7 and MDA-MB-231 cells. Two days after viral infection, control and β3GalT5 stable pools were selected with puromycin (2 µg/mL). To establish β3GalT5 knockdown cells, the lentivirus-shRNA systems for human β3GalT5 were purchased from National RNAi Core Facility Platform, Academia Sinica, and the β3GalT5-short hairpin sequence is
5'CCGGGCAAGTGGTTTGTCAGTAAATCTCGAGATTTACTGACAAACCACTTGCTT TTTG-3'. Briefly, shβ3GalT5 and shControl lentiviruses were incubated with MCF7 and MDA-MB-231 cells according to the manufacturer's instructions. Infected cells were harvested 48 h post-infection or selected with puromycin (2 µg/mL) and the knockdown efficiency was determined by quantitative RT-PCR (qPCR).

### OTHER EMBODIMENTS

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

From the above description, one skilled in the art can easily ascertain the essential characteristics of the described embodiments, and without departing from the spirit and scope thereof, can make various changes and modifications of the embodiments to adapt it to various usages and conditions. Thus, other embodiments are also within the claims.

## Claims

1. A method of detecting a ESA^{hi}PROCR^{hi}SSEA-3⁺ or CD44⁺CD24^{-/lo}SSEA-3⁺ breast cancer stem cell in a patient, the method comprising
detecting whether the breast cancer stem cell is present in an isolated biological sample by contacting the biological sample with an anti-SSEA3 antibody and antibodies that bind to tumor cell antigens ESA/PROCR and tumor cell antigens CD44/CD24 on the breast cancer stem cell, and detecting the specific binding by the anti-SSEA3 antibody to the breast cancer stem cell and the specific binding by the antibodies that bind to PROCR, ESA, CD44, and CD24 on the breast cancer stem cell; thereby detecting the ESA^{hi}PROCR^{hi}SSEA-3⁺ or CD44⁺CD24^{-/lo}SSEA-3⁺ breast cancer stem cell.

2. A method for diagnosing, staging and/or prognosing breast cancer and/or monitoring the susceptibility to treatment, comprising the step of analyzing the expression of tumor-associated carbohydrate antigens on cells in a biological sample isolated from a patient, wherein said biological sample is stained with binding agents that specifically bind to the tumor-associated carbohydrate antigens, said binding agents being antibodies,
wherein the tumor-associated carbohydrate antigen is selected from the group consisting of SSEA3, CD24, CD44, PROCR, and ESA, wherein the presence of cells expressing ESA^{hi}PROCR^{hi}SSEA-3⁺ or CD44⁺CD24^{-/lo}SSEA-3⁺ indicates the presence of breast cancer stem cells in said biological sample.

3. The method of claim 1 or 2, wherein the biological sample is a bodily fluid sample.

4. The method of claim 3, wherein the bodily fluid sample is a blood sample.

5. The method of claim 1, wherein the detection is done using flow cytometry.

## Patentansprüche

1. Verfahren zum Nachweisen einer ESA^{hi}PROCR^{hi}SSEA-3⁺- oder CD44⁺CD24^{-/lo}SSEA-3⁺-Brustkrebsstammzelle in einem Patienten, wobei das Verfahren umfasst
Nachweisen, ob die Brustkrebsstammzelle in einer isolierten biologischen Probe vorhanden ist, indem die biologischen Probe mit einem Anti-SSEA3-Antikörper und Antikörpern in Kontakt gebracht wird, die an Tumorzellen-Antigene ESA/PROCR und Tumorzellen-Antigene CD44/CD24 auf der Brustkrebsstammzelle binden, und Nachweisen der spezifischen Bindung durch den Anti-SSEA3-Antikröper an die Brustkrebsstammzelle und der spezifischen Bindung durch die Antikörper, die an PROCR, ESA, CD44, und CD24 auf der Brustkrebsstammzelle binden, um dadurch die ESA^{hi}PROCR^{hi}SSEA-3⁺- oder CD44⁺CD24^{-/lo}SSEA-3⁺-Brustkrebsstammzelle nachzuweisen.

2. Verfahren zur Diagnose, Einstufung und/oder Prognose von Brustkrebs und/oder Überwachung der Ansprechbarkeit auf eine Behandlung, umfassend den Schritt des Analysierens der Expression von tumorassoziierten Kohlenhydrat-Antigenen auf Zellen in einer biologischen Probe, die aus einem Patienten isoliert wird,
wobei die biologische Probe mit bindenden Agenzien gefärbt wird, die spezifisch an tumorassoziierte Kohlenhydrat-Antigene binden, wobei die bindenden Agenzien Antikörper sind,
wobei das tumorassoziierte Kohlenhydrat-Antigen aus der Gruppe bestehend aus SSEA3, CD24, CD44, PROCR und ESA ausgewählt wird, wobei das Vorhandensein von Zellen, die ESA^{hi}PROCRₕᵢSSEA-3⁺ oder CD44⁺CD24^{-/lo}SSEA-3⁺ exprimieren, auf das Vorhandensein von Brustkrebsstammzellen in der biologischen Probe hinweist.

3. Verfahren nach Anspruch 1 oder 2, wobei die biologische Probe eine Körperflüssigkeitsprobe ist.

4. Verfahren nach Anspruch 3, wobei die Körperflüssigkeitsprobe eine Blutprobe ist.

5. Verfahren nach Anspruch 1, wobei der Nachweis unter Verwendung von Durchflusszytometrie erfolgt.

## Revendications

1. Procédé de détection d'une cellule souche du cancer du sein ESA^{hi}PROCR^{hi}SSEA-3⁺ ou CD44⁺CD24^{-/lo}SSEA-3⁺ chez un patient, le procédé comprenant
la détection du fait que la cellule souche du cancer du sein soit présente ou non dans un échantillon biologique isolé par une mise en contact de l'échantillon biologique avec un anticorps anti-SSEA3 et des anticorps qui se lient à des antigènes de cellules tumorales ESA/PROCR et des antigènes de cellules tumorales CD44/CD24 sur la cellule souche du cancer du sein, et la détection de la liaison spécifique par l'anticorps anti-SSEA3 à la cellule souche du cancer du sein et de la liaison spécifique par les anticorps qui se lient à PROCR, ESA, CD44, et CD24 sur la cellule souche du cancer du sein ; en détectant ainsi la cellule souche du cancer du sein ESA^{hi}PROCR^{hi}SSEA-3⁺ ou CD44⁺CD24^{-/lo}SSEA-3⁺.

2. Procédé de diagnostic, stadification et/ou pronostic du cancer du sein et/ou de surveillance de la sensibilité à un traitement, comprenant l'étape consistant à analyser l'expression d'antigènes glucidiques associés aux tumeurs sur des cellules dans un échantillon biologique isolé chez un patient, où ledit échantillon biologique est coloré avec des agents de liaison qui se lient spécifiquement aux antigènes glucidiques associés aux tumeurs, lesdits agents de liaison étant des anticorps,
où l'antigène glucidique associé aux tumeurs est sélectionné dans le groupe consistant en SSEA3, CD24, CD44, PROCR, et ESA, où la présence de cellules exprimant ESA^{hi}PROCR^{hi}SSEA-3⁺ ou CD44⁺CD24^{-/lo}SSEA-3⁺ indique la présence de cellules souches du cancer du sein dans ledit échantillon biologique.

3. Procédé selon la revendication 1 ou 2, dans lequel l'échantillon biologique est un échantillon de liquide organique.

4. Procédé selon la revendication 3, dans lequel l'échantillon de liquide organique est un échantillon de sang.

5. Procédé selon la revendication 1, dans lequel la détection est réalisée en utilisant la cytométrie en flux.
